# EUROPÄISCHE PATENTSCHRIFT

(11) **EP 2 102 197 B1**
(45) Veröffentlichungstag und Bekanntmachung des Hinweises auf die Patenterteilung: **13.07.2011**
(21) Anmeldenummer: 07847052.3
(22) Anmeldetag: 11.12.2007
(51) Int. Cl.: C07D 413/04, C07D 417/04, A61K 31/433, A61P 3/10, A61P 3/04, A61P 3/06

(54) **5-([1,3,4]OXADIAZOL-2-YL)-1H-INDAZOL UND 5-([1,3,4]THIADIAZOL-2-YL)-1H-INDAZOL DERIVATE ALS SGK-INHIBITOREN ZUR BEHANDLUNG VON DIABETES**
5-([1,3,4]OXADIAZOL-2-YL)-1H-INDAZOL AND 5-([1,3,4]THIADIAZOL-2-YL)-1H-INDAZOL DERIVATIVES AS SGK INHIBITORS FOR THE TREATMENT OF DIABETES
DERIVES DE 5-([1,3,4]OXADIAZOLE-2-YL)-1H-INDAZOLE ET 5-([1,3,4]THIADIAZOLE-2-YL)-1H-INDAZOLE COMME INHIBITEURS DE LA SGK POUR LE TRAITEMENT DE DIABETES

(30) Priorität: 18.01.2007 DE 102007002717
(43) Veröffentlichungstag der Anmeldung: 23.09.2009
(73) Patentinhaber: Merck Patent GmbH, 64293 Darmstadt (DE)
(72) Erfinder: KLEIN, Markus, 64291 Darmstadt (DE); BEIER, Norbert, 64354 Reinheim (DE)
(86) Internationale Anmeldenummer: PCT/EP2007/010771
(87) Internationale Veröffentlichungsnummer: WO 2008/086854

(56) Entgegenhaltungen:
- WO-A-02/10137
- WO-A-2004/094388
- WO-A-2006/058007
- WO-A-2008/016123
- US-A1- 2005 009 876

## Beschreibung

Der Erfindung lag die Aufgabe zugrunde, neue Verbindungen mit wertvollen Eigenschaften aufzufinden, insbesondere solche, die zur Herstellung von Arzneimitteln verwendet werden können.

Die vorliegende Erfindung betrifft Verbindungen, bei denen die Hemmung, Regulierung und/oder Modulation der Signaltransduktion von Kinasen, insbesondere der zellvolumenregulierten humanen Kinase h-sgk (human serum and glucocorticoid dependent kinase oder SGK) eine Rolle spielt, ferner pharmazeutische Zusammensetzungen, die diese Verbindungen enthalten, sowie die Verwendung der Verbindungen zur Behandlung SGKbedingter Krankheiten.

Die SGK mit den Isoformen SGK-1, SGK-2 und SGK-3 sind eine Serin/Threonin-Proteinkinase Familie (WO 02/17893).

Die erfindungsgemäßen Verbindungen sind vorzugsweise selektive Inhibitoren der SGK-1. Ferner können sie Inhibitoren der SGK-2 und/oder SGK-3 sein.

Im einzelnen betrifft die vorliegende Erfindung Verbindungen, die die Signaltransduktion der SGK hemmen, regulieren und/oder modulieren, Zusammensetzungen, die diese Verbindungen enthalten, sowie Verfahren zu ihrer Verwendung zur Behandlung von SGK-bedingten Krankheiten und Leiden wie Diabetes (z.B. Diabetes mellitus, diabetische Nephropathie, diabetische Neuropathie, diabetische Angiopathie und Mikroangiopathie), Fettsucht, metabolisches Syndrom (Dyslipidämie), systemische und pulmonale Hypertonie, Herzkreislauferkrankungen (z.B. kardiale Fibrosen nach Myokardinfarkt, Herzhypertrophie und Herzinsuffizienz, Arteriosklerose) und Nierenerkrankungen (z.B. Glomerulosklerose, Nephrosklerose, Nephritis, Nephropathie, Störung der Elektrolytausscheidung), allgemein bei jeglicher Art von Fibrosen und entzündlichen Prozessen (z.B. Leberzirrhose, Lungenfibrose, fibrosierende Pankreatitis, Rheumatismus und Arthrosen, Morbus Crohn, chronische Bronchitis, Strahlenfibrose, Sklerodermitis, zystische Fibrose, Narbenbildung, Morbus Alzheimer).

Die erfindungsgemäßen Verbindungen können auch das Wachstum von Tumorzellen und Tumormetastasen hemmen und sind deshalb für die Tumortherapie geeignet.

Die erfindungsgemäßen Verbindungen finden auch Verwendung bei der Behandlung von peptischen Ulcera, insbesondere bei Formen, die durch Stress ausgelöst werden.

Die erfindungsgemäßen Verbindungen finden weiterhin Verwendung zur Behandlung von Koagulopathien, wie z.B. Dysfibrinogenämie, Hypoprokonvertinämie, Hämophilie B, Stuart-Prower-Defekt, ProthrombinKomplex-Mangel, Verbrauchskoagulopathie, Hyperfibrinolyse, Immunokoagulopathie oder komplexer Koagulopathien, wie auch bei neuronaler Erregbarkeit, z.B. Epilepsie. Die erfindungsgemäßen Verbindungen können auch bei der Behandlung eines Glaukoms oder Katarakt therapeutisch eingesetzt werden.

Die erfindungsgemäßen Verbindungen finden ferner Verwendung bei der Behandlung bakterieller Infektionen sowie in einer antiinfektiösen Therapie. Die erfindungsgemäßen Verbindungen können auch zur Steigerung der Lernfähigkeit und Aufmerksamkeit therapeutisch eingesetzt werden. Darüberhinaus wirken die erfindungsgemäßen Verbindungen der Zellalterung und Stress entgegen und steigern somit die Lebenserwartung und die Fitness im Alter.

Die erfindungsgemäßen Verbindungen finden ferner Verwendung bei der Behandlung von Tinitus.

Die Identifikation von kleinen Verbindungen, die die Signaltransduktion der SGK spezifisch hemmen, regulieren und/oder modulieren, ist daher wünschenswert und ein Ziel der vorliegenden Erfindung.

Es wurde gefunden, daß die erfindungsgemäßen Verbindungen und ihre Salze bei guter Verträglichkeit sehr wertvolle pharmakologische Eigenschaften besitzen.

Insbesondere zeigen sie inhibierende Eigenschaften der SGK.

Die erfindungsgemäßen Verbindungen zeigen weiterhin Aktivität auf andere Kinasen wie Aurora-B, MAPK2, MSK1, PRK2, DYRK1, CHK2, GSK3-beta, PKB (AKT), ROCKII oder S6K1.

Gegenstand der vorliegenden Erfindung sind deshalb erfindungsgemäße Verbindungen als Arzneimittel und/oder Arzneimittelwirkstoffe bei der Behandlung und/oder Prophylaxe der genannten Erkrankungen und die Verwendung von erfindungsgemäßen Verbindungen zur Herstellung eines Pharmazeutikums für die Behandlung und/oder Prophylaxe der genannten Erkrankungen wie auch ein Verfahren zur Behandlung der genannten Erkrankungen umfassend die Verabreichung eines oder mehrerer erfindungsgemäßer Verbindungen an einen Patienten mit Bedarf an einer derartigen Verabreichung.

Der Wirt oder Patient kann jeglicher Säugerspezies angehören, z. B. einer Primatenspezies, besonders Menschen; Nagetieren, einschließlich Mäusen, Ratten und Hamstern; Kaninchen; Pferden, Rindern, Hunden, Katzen usw. Tiermodelle sind für experimentelle Untersuchungen von Interesse, wobei sie ein Modell zur Behandlung einer Krankheit des Menschen zur Verfügung stellen.

Zur Identifizierung eines Signalübertragungswegs und zum Nachweis von Wechselwirkungen zwischen verschiedenen Signalübertragungswegen wurden von verschiedenen Wissenschaftlern geeignete Modelle oder Modellsysteme entwickelt, z.B. Zellkulturmodelle (z.B. Khwaja et al., EMBO, 1997, 16, 2783-93) und Modelle transgener Tiere (z.B. White et al., Oncogene, 2001, 20, 7064-7072). Zur Bestimmung bestimmter Stufen in der Signalübertragungskaskade können wechselwirkende Verbindungen genutzt werden, um das Signal zu modulieren (z.B. Stephens et al., Biochemical J., 2000, 351, 95-105). Die erfindungsgemäßen Verbindungen können auch als Reagenzien zur Testung kinaseabhängiger Signalübertragungswege in Tieren und/oder Zellkulturmodellen oder in den in dieser Anmeldung genannten klinischen Erkrankungen verwendet werden.

Die Messung der Kinaseaktivität ist eine dem Fachmann wohlbekannte Technik. Generische Testsysteme zur Bestimmung der Kinaseaktivität mit Substraten, z.B. Histon (z.B. Alessi et al., FEBS Lett. 1996, 399, 3, Seiten 333-338) oder dem basischen Myelinprotein sind in der Literatur beschrieben (z.B. Campos-González, R. und Glenney, Jr., J.R. 1992, J. Biol. Chem. 267, Seite 14535).

Zur Identifikation von Kinase-Inhibitoren stehen verschiedene Assay-Systeme zur Verfügung. Beim Scintillation-Proximity-Assay (Sorg et al., J. of. Biomolecular Screening, 2002, 7, 11-19) und dem FlashPlate-Assay wird die radioaktive Phosphorylierung eines Proteins oder Peptids als Substrat mit γATP gemessen. Bei Vorliegen einer inhibitorischen Verbindung ist kein oder ein vermindertes radioaktives Signal nachweisbar. Ferner sind die Homogeneous Time-resolved Fluorescence Resonance Energy Transfer- (HTR-FRET-) und Fluoreszenzpolarisations- (FP-) Technologien als Assay-Verfahren nützlich (Sills et al., J. of Biomolecular Screening, 2002, 191-214).

Andere nicht radioaktive ELISA-Assay-Verfahren verwenden spezifische Phospho-Antikörper (Phospho-AK). Der Phospho-AK bindet nur das phosphorylierte Substrat. Diese Bindung ist mit einem zweiten Peroxidasekonjugierten Anti-Schaf-Antikörper durch Chemolumineszenz nachweisbar (Ross et al., Biochem. J., 2002, 366, 977-981).

### STAND DER TECHNIK

Andere Indazolderivate zur Behandlung von Krankheiten des Zentralnervensystem sind in WO 2006/058007 A2 beschrieben.

In US 2005/0009876 A1 werden andere Indazolderivate zur Behandlung von Diabetes offenbart.

Wiederum andere Indazolderivate zur Behandlung von Diabetes kennt man aus WO 2004/094388 A2 und aus WO 02/10137 A2.

In der WO 00/62781 ist die Verwendung von Arzneimitteln enthaltend Hemmstoffe der zellvolumenregulierten humanen Kinase H-SGK beschrieben.

Andere heterocyclische Indazolderivate zur Behandlung von Diabetes und/oder Krebserkrankungen kennt man aus WO 2006/044860 und WO 2005056550.

In US 2005090529 sind andere Indazolderivate zur Behandlung von diabetischer Retinopathie offenbart.

Andere Indazolderivate sind als Cytokinininhibitoren in WO2005023761 beschrieben.

Andere Indazolderivate zur Behandlung von Tumoren sind in WO 2005000813 offenbart, solche zur Behandlung von cardiovasculärer Erkrankungen in WO 2004060318.

Andere heterocyclische Verbindungen zur Behandlung von Tumoren kennt man aus WO2004052280.

Ferner sind andere Heterocyclen zur Behandlung psychotischer Erkrankungen in EP 328200 offenbart.

Andere Indazolderivate sind als Proteinkinase-Inhibitoren in der WO 03/064397 beschrieben.

In Bioorganic & Medicinal Chemistry Letters 13 (2003) 3059-3062 beschreibt J. Witherington et al. die Herstellung anderer Indazolderivate. Andere Indazolderivate sind als Kinaseinhibitoren in WO 2003097610 beschrieben.

Andere Indazolderivate sind als GSK-3-Inhibitoren in WO 2003051847 offenbart.

Die Herstellung von Indazolverbindungen, die als Rho-Kinase-Inhibitoren wirken, kennt man aus WO 2005035506.

Die Herstellung von Aminoindazolen, die als Protein Tau Phosphorylierungs-Inhibitoren wirken, ist in WO 2004062662, FR 2848554, WO 2004022544 und FR 2844267 offenbart.

Die Verwendung von Kinase-Inhibitoren in der antiinfektiösen Therapie ist von C.Doerig in Cell. Mol. Biol. Lett. Vol.8, No. 2A, 2003, 524-525 beschrieben.

Die Verwendung von Kinase-Inhibitoren bei Fettsucht ist von N.Perrotti in J. Biol. Chem. 2001, März 23; 276(12):9406-9412 beschrieben.

In nachstehenden Literaturstellen wird die Verwendung von SGK-Hemmern bei der Behandlung von Krankheiten nahegelegt und/oder beschrieben:
1: Chung EJ, Sung YK, Farooq M, Kim Y, Im S, Tak WY, Hwang YJ, Kim YI, Han HS, Kim JC, Kim MK. Gene expression profile analysis in human hepatocellular carcinoma by cDNA microarray. Mol Cells. 2002;14:382-7.
2: Brickley DR, Mikosz CA, Hagan CR, Conzen SD. Ubiquitin modification of serum and glucocorticoid-induced protein kinase-1(SGK-1). J Biol Chem. 2002;277:43064-70.
3: Fillon S, Klingel K, Warntges S, Sauter M, Gabrysch S, Pestel S, Tanneur V, Waldegger S, Zipfel A, Viebahn R, Haussinger D, Broer S, Kandolf R, Lang F. Expression of the serine/threonine kinase hSGK1 in chronic viral hepatitis. Cell Physiol Biochem. 2002;12:47-54.
4: Brunet A, Park J, Tran H, Hu LS, Hemmings BA, Greenberg ME. Protein kinase SGK mediates survival signals by phosphorylating the forkhead transcription factor FKHRL1 (FOXO3a). Mol Cell Biol 2001;21:952-65
5: Mikosz CA, Brickley DR, Sharkey MS, Moran TW, Conzen SD. Glucocorticoid receptor-mediated protection from apoptosis is associated with induction of the serine/threonine survival kinase gene, sgk-1. J Biol Chem. 2001;276:16649-54.
6: Zuo Z, Urban G, Scammell JG, Dean NM, McLean TK, Aragon I, Honkanen RE. Ser/Thr protein phosphatase type 5 (PP5) is a negative regulator of glucocorticoid receptor-mediated growth arrest. Biochemistry. 1999;38:8849-57.
7: Buse P, Tran SH, Luther E, Phu PT, Aponte GW, Firestone GL. Cell cycle and hormonal control of nuclear-cytoplasmic localization of the serum- and glucocorticoid-inducible protein kinase, Sgk, in mammary tumor cells. A novel convergence point of anti-proliferative and proliferative cell signalling pathways. J Biol Chem. 1999;274:7253-63.
8: M. Hertweck, C. Göbel, R. Baumeister: C.elegans SGK-1 is the critical component in the Akt/PKB Kinase complex to control stress response and life span. Developmental Cell, Vol. 6, 577-588, April, 2004.

### ZUSAMMENFASSUNG DER ERFINDUNG

Die Erfindung betrifft Verbindungen der Formel I 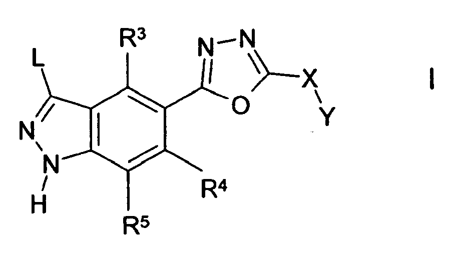 worin
- L: H, Hal oder NR⁷R^{B},
- X: CH₂, CH₂CH₂, CH(CH₃)CH₂, CH₂CH(CH₃), NH, O, NHCH₂, NHCH(CH₃), CH(CH₃)NH, CH₂NH, OCH₂, OCH₂CH₂, CH(CH₃)O, CH₂O, CH₂CH₂O, NHCH₂CH₂, NHCH(CH₃)CH₂, CH₂SO₂ , NHCONHCH₂ oder NHCH₂CONH,
- Y: H, A, Ar oder Het,
- R³: H, Hal oder A,
- R⁴ R⁵: jeweils unabhängig voneinander H, Hal oder A,
- R⁷, R⁸: jeweils unabhängig voneinander H oder CH₃,
- A: Alkyl mit 1-10 C-Atomen, worin 1-7 H-Atome durch F und/oder Cl ersetzt sein können,
- Ar: unsubstituiertes oder ein-, zwei-, drei- oder vierfach durch A, Hal, OH und/oder OA substituiertes Phenyl,
- Het: unsubstituiertes oder ein-, zwei- oder dreifach durch A, Hal, OH und/oder OA substituiertes 2- oder 3-Furyl, 2- oder 3-Thienyl, 1- , 2- oder 3-Pyrrolyl, 1-, 2, 4- oder 5-Imidazolyl, 1-, 3-, 4- oder 5- Pyrazolyl, 2-, 4- oder 5-Oxazolyl, 3-, 4- oder 5-lsoxazolyl, 2-, 4- oder 5-Thiazolyl, 3-, 4- oder 5-lsothiazolyl, 2-, 3- oder 4-Pyridyl, 2-, 4-, 5- oder 6-Pyrimidinyl, 1,2,3-Triazol-1-, -4- oder -5-yl, 1,2,4-Triazol-1-, -3- oder 5-yl, 1- oder 5-Tetrazolyl, 1,2,3- Oxadiazol-4- oder -5-yl, 1,2,4-Oxadiazol-3- oder -5-yl, 1,3,4- Thiadiazol-2- oder -5-yl, 1,2,4-Thiadiazol-3- oder -5-yl, 1,2,3- Thiadiazol-4- oder -5-yl, 3- oder 4-Pyridazinyl, Pyrazinyl, 1-, 2-, 3-, 4-, 5-, 6- oder 7-Indolyl, 4- oder 5-Isoindolyl, 1-, 2-, 4- oder 5- Benzimidazolyl, 1-, 2-, 3-, 4-, 5-, 6- oder 7-Indazolyl, Pyrrolidinyl, Piperidinyl oder Morpholinyl,
- Hal: F, Cl, Br oder I,
bedeuten,
sowie ihre pharmazeutisch verwendbaren Salze, Solvate und Stereoisomere, einschließlich deren Mischungen in allen Verhältnissen.

Gegenstand der Erfindung sind die Verbindungen der Formel I und ihre Salze sowie ein Verfahren zur Herstellung von Verbindungen der Formel I nach den Ansprüchen 1-2 sowie ihrer pharmazeutisch verwendbaren Solvate, Salze und Stereoisomere, dadurch gekennzeichnet, daß man
a) sie aus einem ihrer funktionellen Derivate durch Behandeln mit einem solvolysierenden oder hydrogenolysierenden Mittel in Freiheit setzt, indem man
   eine konventionelle Aminoschutzgruppe durch Behandeln mit einem solvolysierenden oder hydrogenolysierenden Mittel durch Wasserstoff ersetzt oder
   eine durch eine konventionelle Schutzgruppe geschützte Aminogruppe in Freiheit setzt,
   oder
b) zur Herstellung von Verbindungen der Formel I, worin
   - L: NH₂ bedeutet,
   eine Verbindung der Formel II 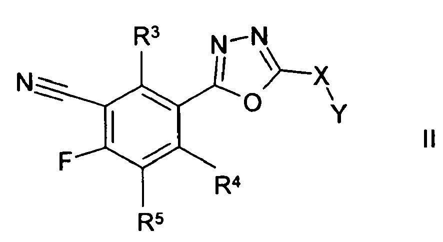 worin
   R³, R⁴, R⁵, X und Y die in Anspruch 1 angegebenen Bedeutungen haben,
   mit Hydrazin umsetzt,
   oder
c) eine Verbindung der Formel III 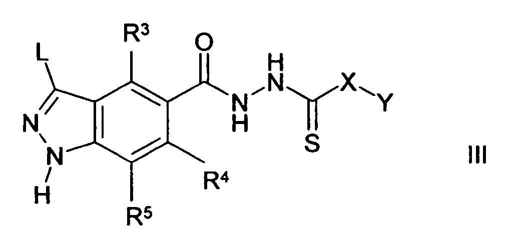 worin
   L, R³, R⁴, R⁵, X und Y die in Anspruch 1 angegebenen Bedeutungen haben,
   in Gegenwart eines Hg(II) - salzes cyclisiert,
   oder
d) eine Verbindung der Formel IV 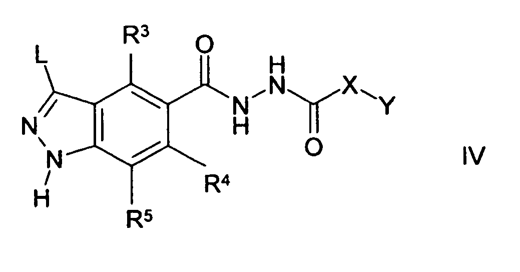 worin
   L, R³, R⁴, R⁵, X und Y die in Anspruch 1 angegebenen Bedeutungen haben,
   in Gegenwart von POCl₃ cyclisiert,
und/oder eine Base oder Säure der Formel I in eines ihrer Salze umwandelt.

Gegenstand der Erfindung sind auch die Stereoisomeren (E, Z-Isomeren) sowie die Hydrate und Solvate dieser Verbindungen. Unter Solvate der Verbindungen werden Anlagerungen von inerten Lösungsmittelmolekülen an die Verbindungen verstanden, die sich aufgrund ihrer gegenseitigen Anziehungskraft ausbilden. Solvate sind z.B. Mono- oder Dihydrate oder Alkoholate.

Der Ausdruck "wirksame Menge" bedeutet die Menge eines Arzneimittels oder eines pharmazeutischen Wirkstoffes, die eine biologische oder medizinische Antwort in einem Gewebe, System, Tier oder Menschen hervorruft, die z.B. von einem Forscher oder Mediziner gesucht oder erstrebt wird.

Darüberhinaus bedeutet der Ausdruck "therapeutisch wirksame Menge" eine Menge, die, verglichen zu einem entsprechenden Subjekt, das diese Menge nicht erhalten hat, folgendes zur Folge hat:
verbesserte Heilbehandlung, Heilung, Prävention oder Beseitigung einer Krankheit, eines Krankheitsbildes, eines Krankheitszustandes, eines Leidens, einer Störung oder von Nebenwirkungen oder auch die Verminderung des Fortschreitens einer Krankheit, eines Leidens oder einer Störung.

Die Bezeichnung "therapeutisch wirksame Menge" umfaßt auch die Mengen, die wirkungsvoll sind, die normale physiologische Funktion zu erhöhen.

Gegenstand der Erfindung sind auch Mischungen der erfindungsgemäßen Verbindungen der Formel I, z.B. Gemische zweier Diastereomerer oder Enantiomerer z.B. im Verhältnis 1:1, 1:2, 1:3, 1:4, 1:5, 1:10, 1:100 oder 1:1000.

Besonders bevorzugt handelt es sich dabei um Mischungen stereoisomerer Verbindungen, insbesondere liegen die erfindungsgemäßen Verbindungen als Racemat vor.

Für alle Reste, die mehrfach auftreten, gilt, daß deren Bedeutungen unabhängig voneinander sind.

Vor- und nachstehend haben die Reste bzw. Parameter L, X, Y, R³, R⁴ und R⁵ die bei der Formel I angegebenen Bedeutungen, falls nicht ausdrücklich etwas anderes angegeben ist.

A bedeutet Alkyl, ist unverzweigt (linear) oder verzweigt, und hat 1, 2, 3, 4, 5, 6, 7, 8, 8, 9 oder 10 C-Atome. A bedeutet vorzugsweise Methyl, weiterhin Ethyl, Propyl, Isopropyl, Butyl, Isobutyl, sek.-Butyl oder tert.-Butyl, ferner auch Pentyl, 1-, 2- oder 3-Methylbutyl, 1,1- , 1,2- oder 2,2-Dimethylpropyl, 1-Ethylpropyl, Hexyl, 1- , 2- , 3- oder 4-Methylpentyl, 1,1- , 1,2- , 1,3- , 2,2- , 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl, weiter bevorzugt z.B. Trifluormethyl.

A bedeutet ganz besonders bevorzugt Alkyl mit 1, 2, 3, 4, 5 oder 6 C-Atomen, vorzugsweise Methyl, Ethyl, Propyl, Isopropyl, Butyl, Isobutyl, sek.-Butyl, tert.-Butyl, Pentyl, Hexyl, Trifluormethyl, Pentafluorethyl oder 1,1,1-Trifluorethyl.

Cycloalkyl bedeutet vorzugsweise Cyclopropyl, Cyclobutyl, Cylopentyl, Cyclohexyl oder Cycloheptyl.

Ar bedeutet z.B. Phenyl, o-, m- oder p-Tolyl, o-, m- oder p-Ethylphenyl, o-, m- oder p-Propylphenyl, o-, m- oder p-Isopropylphenyl, o-, m- oder p-tert.-Butylphenyl, o-, m- oder p-Hydroxyphenyl, o-, m- oder p-Methoxyphenyl, o-m- oder p-Ethoxyphenyl, o-, m- oder p-Fluorphenyl, o-, m- oder p-Bromphenyl, o-, m- oder p- Chlorphenyl, weiter bevorzugt 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibromphenyl, 2,5- oder 3,4-Dimethoxyphenyl, 2,3,4-, 2,3,5-, 2,3,6-, 2,4,6- oder 3,4,5-Trichlorphenyl, 2,4,6-Trimethoxyphenyl, 2-Hydroxy-3,5-dichlorphenyl, p-lodphenyl, 4-Fluor-3-chlorphenyl, 2-Fluor-4-bromphenyl, 2,5-Difluor-4-bromphenyl, 3-Brom-6-methoxyphenyl, 3-Chlor-6-methoxyphenyl, 3-Fluor-4-methoxyphenyl oder 2,5-Dimethyl-4-chlorphenyl.

Ar bedeutet vorzugsweise unsubstituiertes oder ein-, zwei-, drei- oder vierfach durch A, Hal, OH und/oder OA substituiertes Phenyl, wie z.B. o-, m- oder p-Methoxyphenyl, o-, m- oder p-Hydroxyphenyl, o-, m- oder p-Fluorphenyl, o-, m- oder p-Chlorphenyl, 2,3-, 2,4-, 2,5-, 2,6-, 3,4- oder 3,5-Dichlorphenyl, 2,3-, 2,4-, 2,5-, 2,6-, 3,4-, 3,5-Difluorphenyl oder 3-Chlor-4-fluor-phenyl.

Het bedeutet ganz besonders bevorzugt unsubstituiertes oder ein-, zwei- oder dreifach durch A, Hal, OH und/oder OA substituiertes 2- oder 3-Furyl, 2- oder 3-Thienyl, 1-, 2- oder 3-Pyrrolyl, 1-, 2, 4- oder 5-Imidazolyl, 1-, 3-, 4- oder 5-Pyrazolyl, 2-, 4- oder 5-Oxazolyl, 3-, 4- oder 5-lsoxazolyl, 2-, 4-oder 5-Thiazolyl, 3-, 4- oder 5-Isothiazolyl, 2-, 3- oder 4-Pyridyl, 2-, 4-, 5-oder 6-Pyrimidinyl, 1,2,3-Triazol-1-, -4- oder -5-yl, 1,2,4-Triazol-1-, -3- oder . 5-yl, 1- oder 5-Tetrazolyl, 1,2,3-Oxadiazol-4- oder -5-yl, 1,2,4-Oxadiazol-3-oder -5-yl, 1,3,4-Thiadiazol-2- oder -5-yl, 1,2,4-Thiadiazol-3- oder -5-yl, 1,2,3-Thiadiazol-4- oder -5-yl, 3- oder 4-Pyridazinyl, Pyrazinyl, 1-, 2-, 3-, 4-, 5-, 6- oder 7-Indolyl, 4- oder 5-lsoindolyl, 1-, 2-, 4- oder 5-Benzimidazolyl, 1-, 2-, 3-, 4-, 5-, 6- oder 7-Indazolyl, Pyrrolidinyl, Piperidinyl oder Morpholinyl.

In einer anderen besonders bevorzugten Ausführungsform bedeutet Het unsubstituiertes oder einfach durch Hal substituiertes 2- oder 3-Furyl, 2-oder 3-Thienyl oder Morpholinyl.

L bedeutet vorzugsweise H, Hal oder NR⁷R⁸, ganz besonders bevorzugt H, Cl, F oder NH₂.

X bedeutet besonders bevorzugt CH₂, CH₂CH₂, CH(CH₃)CH₂, CH₂CH(CH₃), NH, O NHCH₂, NHCH(CH₃), CH(CH₃)NH, CH₂NH, OCH₂, OCH₂CH₂, CH(CH₃)O, CH₂O, CH₂CH₂O NHCH₂CH₂, NHCH(CH₃)CH₂, CH₂SO₂, NHCONHCH₂ oder NHCH₂CONH..

R³ bedeutet vorzugsweise H, Hal oder A.

R⁴, R⁵ bedeuten vorzugsweise, jeweils unabhängig voneinander H, Hal oder A.

R⁷, R⁸ bedeuten besonders bevorzugt, jeweils unabhängig voneinander H oder CH₃.

Die Verbindungen der Formel I können ein oder mehrere chirale Zentren besitzen und daher in verschiedenen stereoisomeren Formen vorkommen. Die Formel I umschließt alle diese Formen.

Die erfindungsgemäßen Verbindungen und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den erfindungsgemäßen Verbindungen umsetzt.

Die Ausgangsverbindungen sind in der Regel bekannt. Sind sie neu, so können sie aber nach an sich bekannten Methoden hergestellt werden.

Verbindungen der Formel I können vorzugsweise erhalten werden, indem man Verbindungen der Formel I aus einem ihrer funktionellen Derivate durch Behandeln mit einem solvolysierenden oder hydrogenolysierenden Mittel in Freiheit setzt.

Bevorzugte Ausgangsstoffe für die Solvolyse bzw. Hydrogenolyse sind solche, die sonst der Formel I entsprechen, aber anstelle einer oder mehrerer freier Amino- und/oder Hydroxygruppen entsprechende geschützte Amino- und/oder Hydroxygruppen enthalten, vorzugsweise solche, die anstelle eines H-Atoms, das mit einem N-Atom verbunden ist, eine Aminoschutzgruppe tragen, insbesondere solche, die anstelle einer HN-Gruppe eine R'-N-Gruppe tragen, worin R' eine Aminoschutzgruppe bedeutet, und/oder solche, die anstelle des H-Atoms einer Hydroxygruppe eine Hydroxyschutzgruppe tragen, z.B. solche, die der Formel I entsprechen, jedoch anstelle einer Gruppe -COOH eine Gruppe -COOR" tragen, worin R" eine Hydroxyschutzgruppe bedeutet.

Es können auch mehrere - gleiche oder verschiedene - geschützte Amino- und/oder Hydroxygruppen im Molekül des Ausgangsstoffes vorhanden sein. Falls die vorhandenen Schutzgruppen voneinander verschieden sind, können sie in vielen Fällen selektiv abgespalten werden.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind, nachdem die gewünschte chemische Reaktion an anderen Stellen des Moleküls durchgeführt worden ist. Typisch für solche Gruppen sind insbesondere unsubstituierte oder substituierte Acyl-, Aryl-, Aralkoxymethyl- oder Aralkylgruppen. Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen. Der Ausdruck "Acylgruppe" ist im Zusammenhang mit dem vorliegenden Verfahren in weitestem Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl- und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie POA; Alkoxycarbonyl wie Methoxycarbonyl, Ethoxycarbonyl, 2,2,2-Trichlorethoxycarbonyl, BOC (tert.-Butyloxycarbonyl), 2-lodethoxycarbonyl; Aralkyloxycarbonyl wie CBZ ("Carbobenzoxy"), 4-Methoxybenzyloxycarbonyl, FMOC; Arylsulfonyl wie Mtr. Bevorzugte Aminoschutzgruppen sind BOC und Mtr, ferner CBZ, Fmoc, Benzyl und Acetyl.

Der Ausdruck "Hydroxyschutzgruppe" ist ebenfalls allgemein bekannt und bezieht sich auf Gruppen, die geeignet sind, eine Hydroxygruppe vor chemischen Umsetzungen zu schützen, die aber leicht entfernbar sind, nachdem die gewünschte chemische Reaktion an anderen Stellen des Moleküls durchgeführt worden ist. Typisch für solche Gruppen sind die oben genannten unsubstituierten oder substituierten Aryl-, Aralkyl- oder Acylgruppen, ferner auch Alkylgruppen. Die Natur und Größe der Hydroxy schutzgruppen ist nicht kritisch, da sie nach der gewünschten chemischen Reaktion oder Reaktionsfolge wieder entfernt werden; bevorzugt sind Gruppen mit 1-20, insbesondere 1-10 C-Atomen. Beispiele für Hydroxyschutzgruppen sind u.a. Benzyl, p-Nitrobenzoyl, p-Toluolsulfonyl, tert.-Butyl und Acetyl, wobei Benzyl und tert.-Butyl besonders bevorzugt sind.

Das In-Freiheit-Setzen der Verbindungen der Formel I aus ihren funktionellen Derivaten gelingt - je nach der benutzten Schutzgruppe - z. B. mit starken Säuren, zweckmäßig mit TFA oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure. Die Anwesenheit eines zusätzlichen inerten Lösungsmittels ist möglich, aber nicht immer erforderlich. Als inerte Lösungsmittel eignen sich vorzugsweise organische, beispielsweise Carbonsäuren wie Essigsäure, Ether wie Tetrahydrofuran oder Dioxan, Amide wie DMF, halogenierte Kohlenwasserstoffe wie Dichlormethan, ferner auch Alkohole wie Methanol, Ethanol oder Isopropanol, sowie Wasser. Ferner kommen Gemische der vorgenannten Lösungsmittel in Frage. TFA wird vorzugsweise im Überschuß ohne Zusatz eines weiteren Lösungsmittels verwendet, Perchlorsäure in Form eines Gemisches aus Essigsäure und 70 %iger Perchlorsäure im Verhältnis 9:1. Die Reaktionstemperaturen für die Spaltung liegen zweckmäßig zwischen etwa 0 und etwa 50°, vorzugsweise arbeitet man zwischen 15 und 30° (Raumtemperatur).

Die Gruppen BOC, OBut und Mtr können z. B. bevorzugt mit TFA in Dichlormethan oder mit etwa 3 bis 5n HCl in Dioxan bei 15-30° abgespalten werden, die FMOC-Gruppe mit einer etwa 5- bis 50 %igen Lösung von Dimethylamin, Diethylamin oder Piperidin in DMF bei 15-30°.

Hydrogenolytisch entfernbare Schutzgruppen (z. B. CBZ, Benzyl oder die Freisetzung der Amidinogruppe aus ihrem Oxadiazolderivat)) können z. B. durch Behandeln mit Wasserstoff in Gegenwart eines Katalysators (z. B. eines Edelmetallkatalysators wie Palladium, zweckmäßig auf einem Träger wie Kohle) abgespalten werden. Als Lösungsmittel eignen sich dabei die oben angegebenen, insbesondere z. B. Alkohole wie Methanol oder Ethanol oder Amide wie DMF. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt. Eine Hydrogenolyse der CBZ-Gruppe gelingt z. B. gut an 5 bis 10 %igem Pd/C in Methanol oder mit Ammomiumformiat (anstelle von Wasserstoff) an Pd/C in Methanol/DMF bei 20-30°.

Verbindungen der Formel I können weiterhin vorzugsweise erhalten werden, indem man eine Verbindung der Formel II mit Hydrazin umsetzt.

Die Ausgangsverbindungen der Formel II sind in der Regel bekannt. Sind sie neu, so können aber nach an sich bekannten Methoden hergestellt werden.

Die Umsetzung der Verbindung der Formel II mit Hydrazin erfolgt nach Methoden, die dem Fachmann bekannt sind.

Die Umsetzung erfolgt in der Regel in einem inerten Lösungsmittel.

Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Chlorform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel.

Als Lösungsmittel besonders bevorzugt ist 1-Butanol.

Die Reaktionszeit liegt je nach den angewendeten Bedingungen zwischen einigen Minuten und 14 Tagen, die Reaktionstemperatur zwischen etwa -30° und 140°, normalerweise zwischen 20° und 120°, insbesondere zwischen etwa 70° und etwa 100°.

Verbindungen der Formel I können weiterhin vorzugsweise erhalten werden, indem man eine Verbindung der Formel III in Gegenwart in Gegenwart eines Hg(II) - salzes cyclisiert. Bevorzugt ist Hg(II)acetat, ferner Hg(II)oxid.

Die Ausgangsverbindungen der Formel III sind in der Regel bekannt. Sind sie neu, so können aber nach an sich bekannten Methoden hergestellt werden.

Die Cyclisierung der Verbindung der Formel III erfolgt nach Methoden, die dem Fachmann bekannt sind.

Die Umsetzung erfolgt in der Regel in einem inerten Lösungsmittel und unter Bedingungen wie oben beschrieben.

Als Lösungsmittel besonders bevorzugt ist Methanol. Die Reaktionstemperatur liegt insbesondere zwischen etwa 50° und etwa 90°.

Verbindungen der Formel I können weiterhin vorzugsweise erhalten werden, indem man eine Verbindung der Formel IV in Gegenwart von POCl₃ cyclisiert.

Die Ausgangsverbindungen der Formel IV sind in der Regel bekannt. Sind sie neu, so können aber nach an sich bekannten Methoden hergestellt werden.

Die Cyclisierung der Verbindung der Formel IV erfolgt nach Methoden, die dem Fachmann bekannt sind.

Die Reaktionstemperatur liegt insbesondere zwischen etwa 50° und etwa 100°.

Verbindungen der Formel I können ferner erhalten werden, indem man einen Rest L, Y, R³, R⁴ und/oder R⁵ in einen oder mehrere Rest(e) L, Y, R³, R⁴ und/oder R⁵ umwandelt, z.B. indem man mit lod oder N-Chlorsuccinimid umsetzt oder indem man Nitrogruppen (beispielsweise durch Hydrierung an Raney-Nickel oder Pd-Kohle in einem inerten Lösungsmittel wie Methanol oder Ethanol) zu Aminogruppen reduziert.

Ferner kann man freie Aminogruppen in üblicher Weise mit einem Säurechlorid oder -anhydrid acylieren oder mit einem unsubstituierten oder substituierten Alkylhalogenid alkylieren, zweckmäßig in einem inerten Lösungsmittel wie Dichlormethan oder THF und /oder in Gegenwart einer Base wie Triethylamin oder Pyridin bei Temperaturen zwischen -60 und +30°C.

Die Spaltung eines Ethers erfolgt unter Methoden, wie sie dem Fachmann bekannt sind.

Eine Standardmethode zur Etherspaltung, z.B. eines Methylethers, ist die Verwendung von Bortribromid.

Hydrogenolytisch entfernbare Gruppen, z.B. die Spaltung eines Benzylethers, können z. B. durch Behandeln mit Wasserstoff in Gegenwart eines Katalysators (z. B. eines Edelmetallkatalysators wie Palladium, zweckmäßig auf einem Träger wie Kohle) abgespalten werden. Als Lösungsmittel eignen sich dabei die oben angegebenen, insbesondere z. B. Alkohole wie Methanol oder Ethanol oder Amide wie DMF. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt.

Ester können z.B. mit Essigsäure oder mit NaOH oder KOH in Wasser, Wasser-THF oder Wasser-Dioxan bei Temperaturen zwischen 0 und 100° verseift werden.

### Pharmazeutische Salze und andere Formen

Die genannten erfindungsgemäßen Verbindungen lassen sich in ihrer endgültigen Nichtsalzform verwenden. Andererseits umfaßt die vorliegende Erfindung auch die Verwendung dieser Verbindungen in Form ihrer pharmazeutisch unbedenklichen Salze, die von verschiedenen organischen und anorganischen Säuren und Basen nach fachbekannten Vorgehensweisen abgeleitet werden können. Pharmazeutisch unbedenkliche Salzformen der Verbindungen der Formel I werden größtenteils konventionell hergestellt. Sofern die Verbindung der Formel I eine Carbonsäuregruppe enthält, läßt sich eines ihrer geeigneten Salze dadurch bilden, daß man die Verbindung mit einer geeigneten Base zum entsprechenden Basenadditionssalz umsetzt. Solche Basen sind zum Beispiel Alkalimetallhydroxide, darunter Kaliumhydroxid, Natriumhydroxid und Lithiumhydroxid; Erdalkalimetallhydroxide wie Bariumhydroxid und Calciumhydroxid; Alkalimetallalkoholate, z.B. Kaliumethanolat und Natriumpropanolat; sowie verschiedene organische Basen wie Piperidin, Diethanolamin und N-Methylglutamin. Die Aluminiumsalze der Verbindungen der Formel I zählen ebenfalls dazu. Bei bestimmten Verbindungen der Formel I lassen sich Säureadditionssalze dadurch bilden, daß man diese Verbindungen mit pharmazeutisch unbedenklichen organischen und anorganischen Säuren, z.B. Halogenwasserstoffen wie Chlorwasserstoff, Bromwasserstoff oder Jodwasserstoff, anderen Mineralsäuren und ihren entsprechenden Salzen wie Sulfat, Nitrat oder Phosphat und dergleichen sowie Alkyl- und Monoarylsulfonaten wie Ethansulfonat, Toluolsulfonat und Benzolsulfonat, sowie anderen organischen Säuren und ihren entsprechenden Salzen wie Acetat, Trifluoracetat, Tartrat, Maleat, Succinat, Citrat, Benzoat, Salicylat, Ascorbat und dergleichen behandelt. Dementsprechend zählen zu pharmazeutisch unbedenklichen Säureadditionssalzen der Verbindungen der Formel I die folgenden: Acetat, Adipat, Alginat, Arginat, Aspartat, Benzoat, Benzolsulfonat (Besylat), Bisulfat, Bisulfit, Bromid, Butyrat, Kampferat, Kampfersulfonat, Caprylat, Chlorid, Chlorbenzoat, Citrat, Cyclopentanpropionat, Digluconat, Dihydrogenphosphat, Dinitrobenzoat, Dodecylsulfat, Ethansulfonat, Fumarat, Galacterat (aus Schleimsäure), Galacturonat, Glucoheptanoat, Gluconat, Glutamat, Glycerophosphat, Hemisuccinat, Hemisulfat, Heptanoat, Hexanoat, Hippurat, Hydrochlorid, Hydrobromid, Hydroiodid, 2-Hydroxyethansulfonat, lodid, Isethionat, Isobutyrat, Lactat, Lactobionat, Malat, Maleat, Malonat, Mandelat, Metaphosphat, Methansulfonat, Methylbenzoat, Monohydrogenphosphat, 2-Naphthalinsulfonat, Nicotinat, Nitrat, Oxalat, Oleat, Pamoat, Pectinat, Persulfat, Phenylacetat, 3-Phenylpropionat, Phosphat, Phosphonat, Phthalat, was jedoch keine Einschränkung darstellt.

Weiterhin zählen zu den Basensalzen der erfindungsgemäßen Verbindungen Aluminium-, Ammonium-, Calcium-, Kupfer-, Eisen(III)-, Eisen(II)-, Lithium-, Magnesium-, Mangan(III)-, Mangan(II), Kalium-, Natrium- und Zinksalze, was jedoch keine Einschränkung darstellen soll. Bevorzugt unter den oben genannten Salzen sind Ammonium; die Alkalimetallsalze Natrium und Kalium,sowie die Erdalkalimetalsalze Calcium und Magnesium. Zu Salzen der Verbindungen der Formel I, die sich von pharmazeutisch unbedenklichen organischen nicht-toxischen Basen ableiten, zählen Salze primärer, sekundärer und tertiärer Amine, substituierter Amine, darunter auch natürlich vorkommender substituierter Amine, cyclischer Amine sowie basischer lonenaustauscherharze, z.B. Arginin, Betain, Koffein, Chlorprocain, Cholin, N,N'-Dibenzylethylendiamin (Benzathin), Dicyclohexylamin, Diethanolamin, Diethylamin, 2-Diethylaminoethanol, 2-Dimethylaminoethanol, Ethanolamin, Ethylendiamin, N-Ethylmorpholin, N-Ethylpiperidin, Glucamin, Glucosamin, Histidin, Hydrabamin, Iso-propylamin, Lidocain, Lysin, Meglumin, N-Methyl-D-glucamin, Morpholin, Piperazin, Piperidin, Polyaminharze, Procain, Purine, Theobromin, Triethanolamin, Triethylamin, Trimethylamin, Tripropylamin sowie Tris-(hydroxymethyl)-methylamin (Tromethamin), was jedoch keine Einschränkung darstellen soll.

Verbindungen der vorliegenden Erfindung, die basische stickstoffhaltige Gruppen enthalten, lassen sich mit Mitteln wie (C₁-C₄) Alkylhalogeniden, z.B. Methyl-, Ethyl-, Isopropyl- und tert.-Butylchlorid, -bromid und -iodid; Di(C₁-C₄)Alkylsulfaten, z.B. Dimethyl-, Diethyl- und Diamylsulfat; (C₁₀-C₁₈)Alkylhalogeniden, z.B. Decyl-, Dodecyl-, Lauryl-, Myristyl- und Stearylchlorid, -bromid und -iodid; sowie Aryl-(C₁-C₄)Alkylhalogeniden, z.B. Benzylchlorid und Phenethylbromid, quarternisieren. Mit solchen Salzen können sowohl wasser- als auch öllösliche erfindungsgemäße Verbindungen hergestellt werden.

Zu den oben genannten pharmazeutischen Salzen, die bevorzugt sind, zählen Acetat, Trifluoracetat, Besylat, Citrat, Fumarat, Gluconat, Hemisuccinat, Hippurat, Hydrochlorid, Hydrobromid, lsethionat, Mandelat, Meglumin, Nitrat, Oleat, Phosphonat, Pivalat, Natriumphosphat, Stearat, Sulfat, Sulfosalicylat, Tartrat, Thiomalat, Tosylat und Tromethamin, was jedoch keine Einschränkung darstellen soll.

Die Säureadditionssalze basischer Verbindungen der Formel I werden dadurch hergestellt, daß man die freie Basenform mit einer ausreichenden Menge der gewünschten Säure in Kontakt bringt, wodurch man auf übliche Weise das Salz darstellt. Die freie Base läßt sich durch In-Kontakt-Bringen der Salzform mit einer Base und Isolieren der freien Base auf übliche Weise regenerieren. Die freien Basenformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Basenformen.

Wie erwähnt werden die pharmazeutisch unbedenklichen Basenadditionssalze der Verbindungen der Formel I mit Metallen oder Aminen wie Alkalimetallen und Erdalkalimetallen oder organischen Aminen gebildet. Bevorzugte Metalle sind Natrium, Kalium, Magnesium und Calcium. Bevorzugte organische Amine sind N,N'-Dibenzylethylendiamin, Chlorprocain, Cholin, Diethanolamin, Ethylendiamin, N-Methyl-D-glucamin und Procain.

Die Basenadditionssalze von erfindungsgemäßen sauren Verbindungen werden dadurch hergestellt, daß man die freie Säureform mit einer ausreichenden Menge der gewünschten Base in Kontakt bringt, wodurch man das Salz auf übliche Weise darstellt. Die freie Säure läßt sich durch In-Kontakt-Bringen der Salzform mit einer Säure und Isolieren der freien Säure auf übliche Weise regenerieren. Die freien Säureformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Säureformen.

Enthält eine erfindungsgemäße Verbindung mehr als eine Gruppe, die solche pharmazeutisch unbedenklichen Salze bilden kann, so umfaßt die Erfindung auch mehrfache Salze. Zu typischen mehrfachen Salzformen zählen zum Beispiel Bitartrat, Diacetat, Difumarat, Dimeglumin, Diphosphat, Dinatrium und Trihydrochlorid, was jedoch keine Einschränkung darstellen soll.

Im Hinblick auf das oben Gesagte sieht man, daß unter dem Ausdruck "pharmazeutisch unbedenkliches Salz" im vorliegenden Zusammenhang ein Wirkstoff zu verstehen ist, der eine Verbindung der Formel 1 in der Form eines ihrer Salze enthält, insbesondere dann, wenn diese Salzform dem Wirkstoff im Vergleich zu der freien Form des Wirkstoffs oder irgendeiner anderen Salzform des Wirkstoffs, die früher verwendet wurde, verbesserte pharmakokinetische Eigenschaften verleiht. Die pharmazeutisch unbedenkliche Salzform des Wirkstoffs kann auch diesem Wirkstoff erst eine gewünschte pharmakokinetische Eigenschaft verleihen, über die er früher nicht verfügt hat, und kann sogar die Pharmakodynamik dieses Wirkstoffs in bezug auf seine therapeutische Wirksamkeit im Körper positiv beeinflussen.

Erfindungsgemäße Verbindungen der Formel I können aufgrund ihrer Molekülstruktur chiral sein und können dementsprechend in verschiedenen enantiomeren Formen auftreten. Sie können daher in racemischer oder in optisch aktiver Form vorliegen.

Da sich die pharmazeutische Wirksamkeit der Racemate bzw. der Stereoisomeren der erfindungsgemäßen Verbindungen unterscheiden kann, kann es wünschenswert sein, die Enantiomere zu verwenden. In diesen Fällen kann das Endprodukt oder aber bereits die Zwischenprodukte in enantiomere Verbindungen, durch dem Fachmann bekannte chemische oder physikalische Maßnahmen, aufgetrennt oder bereits als solche bei der Synthese eingesetzt werden.

Im Falle racemischer Amine werden aus dem Gemisch durch Umsetzung mit einem optisch aktiven Trennmittel Diastereomere gebildet. Als Trennmittel eignen sich z.B. optisch aktiven Säuren, wie die R- und S-Formen von Weinsäure, Diacetylweinsäure, Dibenzoylweinsäure, Mandelsäure, Äpfelsäure, Milchsäure, geeignet N-geschützte Aminosäuren (z.B. N-Benzoylprolin oder N-Benzolsulfonylprolin) oder die verschiedenen optisch aktiven Camphersulfonsäuren. Vorteilhaft ist auch eine chromatographische Enantiomerentrennung mit Hilfe eines optisch aktiven Trennmittels (z.B. Dinitrobenzoylphenylglycin, Cellulosetriacetat oder andere Derivate von Kohlenhydraten oder auf Kieselgel fixierte chiral derivatisierte Methacrylatpolymere). Als Laufmittel eignen sich hierfür wäßrige oder alkoholische Lösungsmittelgemische wie z.B. Hexan/Isopropanol/ Acetonitril z.B. im Verhältnis 82:15:3.

Gegenstand der Erfindung ist ferner die Verwendung der Verbindungen und/oder ihrer physiologisch unbedenklichen Salze zur Herstellung eines Arzneimittels (pharmazeutische Zubereitung), insbesondere auf nichtchemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoffen in eine geeignete Dosierungsform gebracht werden.

Gegenstand der Erfindung sind ferner Arzneimittel, enthaltend mindestens eine erfindungsgemäße Verbindung und/oder ihre pharmazeutisch verwendbaren Salze, Solvate und Stereoisomere, einschließlich deren Mischungen in allen Verhältnissen, sowie gegebenenfalls Träger- und/oder Hilfsstoffe.

Pharmazeutische Formulierungen können in Form von Dosiseinheiten, die eine vorbestimmte Menge an Wirkstoff pro Dosiseinheit enthalten, dargereicht werden. Eine solche Einheit kann beispielsweise 0,5 mg bis 1 g, vorzugsweise 1 mg bis 700 mg, besonders bevorzugt 5 mg bis 100 mg einer erfindungsgemäßen Verbindung enthalten, je nach dem behandelten Krankheitszustand, dem Verabreichungsweg und dem Alter, Gewicht und Zustand des Patienten, oder pharmazeutische Formulierungen können in Form von Dosiseinheiten, die eine vorbestimmte Menge an Wirkstoff pro Dosiseinheit enthalten, dargereicht werden. Bevorzugte Dosierungseinheitsformulierungen sind solche, die eine Tagesdosis oder Teildosis, wie oben angegeben, oder einen entsprechenden Bruchteil davon eines Wirkstoffs enthalten. Weiterhin lassen sich solche pharmazeutischen Formulierungen mit einem der im pharmazeutischen Fachgebiet allgemein bekannten Verfahren herstellen.

Pharmazeutische Formulierungen lassen sich zur Verabreichung über einen beliebigen geeigneten Weg, beispielsweise auf oralem (einschließlich buccalem bzw. sublingualem), rektalem, nasalem, topischem (einschließlich buccalem, sublingualem oder transdermalem), vaginalem oder parenteralem (einschließlich subkutanem, intramuskulärem, intravenösem oder intradermalem) Wege, anpassen. Solche Formulierungen können mit allen im pharmazeutischen Fachgebiet bekannten Verfahren hergestellt werden, indem beispielsweise der Wirkstoff mit dem bzw. den Trägerstoff(en) oder Hilfsstoff(en) zusammengebracht wird.

An die orale Verabreichung angepaßte pharmazeutische Formulierungen können als separate Einheiten, wie z.B. Kapseln oder Tabletten; Pulver oder Granulate; Lösungen oder Suspensionen in wäßrigen oder nichtwäßrigen Flüssigkeiten; eßbare Schäume oder Schaumspeisen; oder Öl-in-Wasser-Flüssigemulsionen oder Wasser-in-Öl-Flüssigemulsionen dargereicht werden.

So läßt sich beispielsweise bei der oralen Verabreichung in Form einer Tablette oder Kapsel die Wirkstoffkomponente mit einem oralen, nicht-toxischen und pharmazeutisch unbedenklichen inerten Trägerstoff, wie z.B. Ethanol, Glycerin, Wasser u.ä. kombinieren. Pulver werden hergestellt, indem die Verbindung auf eine geeignete feine Größe zerkleinert und mit einem in ähnlicher Weise zerkleinerten pharmazeutischen Trägerstoff, wie z.B. einem eßbaren Kohlenhydrat wie beispielsweise Stärke oder Mannit vermischt wird. Ein Geschmacksstoff, Konservierungsmittel, Dispersionsmittel und Farbstoff können ebenfalls vorhanden sein.

Kapseln werden hergestellt, indem ein Pulvergemisch wie oben beschrieben hergestellt und geformte Gelatinehüllen damit gefüllt werden. Gleit- und Schmiermittel wie z.B. hochdisperse Kieselsäure, Talkum, Magnesiumstearat, Kalziumstearat oder Polyethylenglykol in Festform können dem Pulvergemisch vor dem Füllvorgang zugesetzt werden. Ein Sprengmittel oder Lösungsvermittler, wie z.B. Agar-Agar, Kalziumcarbonat oder Natriumcarbonat, kann ebenfalls zugesetzt werden, um die Verfügbarkeit des Medikaments nach Einnahme der Kapsel zu verbessern.

Außerdem können, falls gewünscht oder notwendig, geeignete Bindungs-, Schmier- und Sprengmittel sowie Farbstoffe ebenfalls in das Gemisch eingearbeitet werden. Zu den geeigneten Bindemitteln gehören Stärke, Gelatine, natürliche Zucker, wie z.B. Glukose oder Beta-Lactose, Süßstoffe aus Mais, natürliche und synthetische Gummi, wie z.B. Akazia, Traganth oder Natriumalginat, Carboxymethylzellulose, Polyethylenglykol, Wachse, u.ä. Zu den in diesen Dosierungsformen verwendeten Schmiermitteln gehören Natriumoleat, Natriumstearat, Magnesiumstearat, Natriumbenzoat, Natriumacetat, Natriumchlorid u.ä. Zu den Sprengmitteln gehören, ohne darauf beschränkt zu sein, Stärke, Methylzellulose, Agar, Bentonit, Xanthangummi u.ä. Die Tabletten werden formuliert, indem beispielsweise ein Pulvergemisch hergestellt, granuliert oder trockenverpreßt wird, ein Schmiermittel und ein Sprengmittel zugegeben werden und das Ganze zu Tabletten verpreßt wird. Ein Pulvergemisch wird hergestellt, indem die in geeigneter Weise zerkleinerte Verbindung mit einem Verdünnungsmittel oder einer Base, wie oben beschrieben, und gegebenenfalls mit einem Bindemittel, wie z.B. Carboxymethylzellulose, einem Alginat, Gelatine oder Polyvinylpyrrolidon, einem Lösungsverlangsamer, wie z.B. Paraffin, einem Resorptionsbeschleuniger, wie z.B. einem quaternären Salz und/oder einem Absorptionsmittel, wie z.B. Bentonit, Kaolin oder Dikalziumphosphat, vermischt wird. Das Pulvergemisch läßt sich granulieren, indem es mit einem Bindemittel, wie z.B. Sirup, Stärkepaste, Acadia-Schleim oder Lösungen aus Zellulose- oder Polymermaterialen benetzt und durch ein Sieb gepreßt wird. Als Alternative zur Granulierung kann man das Pulvergemisch durch eine Tablettiermaschine laufen lassen, wobei ungleichmäßig geformte Klumpen entstehen, die in Granulate aufgebrochen werden. Die Granulate können mittels Zugabe von Stearinsäure, einem Stearatsalz, Talkum oder Mineralöl gefettet werden, um ein Kleben an den Tablettengußformen zu verhindern. Das gefettete Gemisch wird dann zu Tabletten verpreßt. Die erfindungsgemäßen Verbindungen können auch mit einem freifließenden inerten Trägerstoff kombiniert und dann ohne Durchführung der Granulierungs- oder Trockenverpressungsschritte direkt zu Tabletten verpreßt werden. Eine durchsichtige oder undurchsichtige Schutzschicht, bestehend aus einer Versiegelung aus Schellack, einer Schicht aus Zucker oder Polymermaterial und einer Glanzschicht aus Wachs, kann vorhanden sein. Diesen Beschichtungen können Farbstoffe zugesetzt werden, um zwischen unterschiedlichen Dosierungseinheiten unterscheiden zu können.

Orale Flüssigkeiten, wie z.B. Lösung, Sirupe und Elixiere, können in Form von Dosierungseinheiten hergestellt werden, so daß eine gegebene Quantität eine vorgegebene Menge der Verbindung enthält. Sirupe lassen sich herstellen, indem die Verbindung in einer wäßrigen Lösung mit geeignetem Geschmack gelöst wird, während Elixiere unter Verwendung eines nichttoxischen alkoholischen Vehikels hergestellt werden. Suspensionen können durch Dispersion der Verbindung in einem nicht-toxischen Vehikel formuliert werden. Lösungsvermittler und Emulgiermittel, wie z.B. ethoxylierte Isostearylalkohole und Polyoxyethylensorbitolether, Konservierungsmittel, Geschmackszusätze, wie z.B. Pfefferminzöl oder natürliche Süßstoffe oder Saccharin oder andere künstliche Süßstoffe, u.ä. können ebenfalls zugegeben werden.

Die Dosierungseinheitsformulierungen für die orale Verabreichung können gegebenenfalls in Mikrokapseln eingeschlossen werden. Die Formulierung läßt sich auch so herstellen, daß die Freisetzung verlängert oder retardiert wird, wie beispielsweise durch Beschichtung oder Einbettung von partikulärem Material in Polymere, Wachs u.ä.

Die erfindungsgemäßen Verbindungen sowie Salze und Solvate lassen sich auch in Form von Liposomenzuführsystemen, wie z.B. kleinen unilamellaren Vesikeln, großen unilamellaren Vesikeln und multilamellaren Vesikeln, verabreichen. Liposomen können aus verschiedenen Phospholipiden, wie z.B. Cholesterin, Stearylamin oder Phosphatidylcholinen, gebildet werden.

Die erfindungsgemäßen Verbindungen sowie die Salze und Solvate können auch unter Verwendung monoklonaler Antikörper als individuelle Träger, an die die Verbindungsmoleküle gekoppelt werden, zugeführt werden. Die Verbindungen können auch mit löslichen Polymeren als zielgerichtete Arzneistoffträger gekoppelt werden. Solche Polymere können Polyvinylpyrrolidon, Pyran-Copolymer, Polyhydroxypropylmethacrylamidphenol, Polyhydroxyethylaspartamidphenol oder Polyethylenoxidpolylysin, substituiert mit Palmitoylresten, umfassen. Weiterhin können die Verbindungen an eine Klasse von biologisch abbaubaren Polymeren, die zur Erzielung einer kontrollierten Freisetzung eines Arzneistoffs geeignet sind, z.B. Polymilchsäure, Polyepsilon-Caprolacton, Polyhydroxybuttersäure, Polyorthoester, Polyacetale, Polydihydroxypyrane, Polycyanoacrylate und quervernetzte oder amphipatische Blockcopolymere von Hydrogelen, gekoppelt sein.

An die transdermale Verabreichung angepaßte pharmazeutische Formulierungen können als eigenständige Pflaster für längeren, engen Kontakt mit der Epidermis des Empfängers dargereicht werden. So kann beispielsweise der Wirkstoff aus dem Pflaster mittels lontophorese zugeführt werden, wie in Pharmaceutical Research, 3(6), 318 (1986) allgemein beschrieben.

An die topische Verabreichung angepaßte pharmazeutische Verbindungen können als Salben, Cremes, Suspensionen, Lotionen, Pulver, Lösungen, Pasten, Gele, Sprays, Aerosole oder Öle formuliert sein.

Für Behandlungen des Auges oder anderer äußerer Gewebe, z.B. Mund und Haut, werden die Formulierungen vorzugsweise als topische Salbe oder Creme appliziert. Bei Formulierung zu einer Salbe kann der Wirkstoff entweder mit einer paraffinischen oder einer mit Wasser mischbaren Cremebasis eingesetzt werden. Alternativ kann der Wirkstoff zu einer Creme mit einer Öl-in-Wasser-Cremebasis oder einer Wasser-in-Öl-Basis formuliert werden.

Zu den an die topische Applikation am Auge angepaßten pharmazeutischen Formulierungen gehören Augentropfen, wobei der Wirkstoff in einem geeigneten Träger, insbesondere einem wäßrigen Lösungsmittel, gelöst oder suspendiert ist.

An die topische Applikation im Mund angepaßte pharmazeutische Formulierungen umfassen Lutschtabletten, Pastillen und Mundspülmittel.

An die rektale Verabreichung angepaßte pharmazeutische Formulierungen können in Form von Zäpfchen oder Einläufen dargereicht werden.

An die nasale Verabreichung angepaßte pharmazeutische Formulierungen, in denen die Trägersubstanz ein Feststoff ist, enthalten ein grobes Pulver mit einer Teilchengröße beispielsweise im Bereich von 20-500 Mikrometern, das in der Art und Weise, wie Schnupftabak aufgenommen wird, verabreicht wird, d.h. durch Schnellinhalation über die Nasenwege aus einem dicht an die Nase gehaltenen Behälter mit dem Pulver. Geeignete Formulierungen zur Verabreichung als Nasenspray oder Nasentropfen mit einer Flüssigkeit als Trägersubstanz umfassen Wirkstofflösungen in Wasser oder Öl.

An die Verabreichung durch Inhalation angepaßte pharmazeutische Formulierungen umfassen feinpartikuläre Stäube oder Nebel, die mittels verschiedener Arten von unter Druck stehenden Dosierspendern mit Aerosolen, Verneblern oder Insufflatoren erzeugt werden können.

An die vaginale Verabreichung angepaßte pharmazeutische Formulierungen können als Pessare, Tampons, Cremes, Gele, Pasten, Schäume oder Sprayformulierungen dargereicht werden.

Zu den an die parenterale Verabreichung angepaßten pharmazeutischen Formulierungen gehören wäßrige und nichtwäßrige sterile Injektionslösungen, die Antioxidantien, Puffer, Bakteriostatika und Solute, durch die die Formulierung isotonisch mit dem Blut des zu behandelnden Empfängers gemacht wird, enthalten; sowie wäßrige und nichtwäßrige sterile Suspensionen, die Suspensionsmittel und Verdicker enthalten können. Die Formulierungen können in Einzeldosis- oder Mehrfachdosisbehältern, z.B. versiegelten Ampullen und Fläschchen, dargereicht und in gefriergetrocknetem (lyophilisiertem) Zustand gelagert werden, so daß nur die Zugabe der sterilen Trägerflüssigkeit, z.B. Wasser für Injektionszwecke, unmittelbar vor Gebrauch erforderlich ist. Rezepturmäßig hergestellte Injektionslösungen und Suspensionen können aus sterilen Pulvern, Granulaten und Tabletten hergestellt werden.

Es versteht sich, daß die Formulierungen neben den obigen besonders erwähnten Bestandteilen andere im Fachgebiet übliche Mittel mit Bezug auf die jeweilige Art der Formulierung enthalten können; so können beispielsweise für die orale Verabreichung geeignete Formulierungen Geschmacksstoffe enthalten.

Eine therapeutisch wirksame Menge einer Verbindung der vorliegenden Erfindung hängt von einer Reihe von Faktoren ab, einschließlich z.B. dem Alter und Gewicht des Menschen oder Tiers, dem exakten Krankheitszustand, der der Behandlung bedarf, sowie seines Schweregrads, der Beschaffenheit der Formulierung sowie dem Verabreichungsweg, und wird letztendlich von dem behandelnden Arzt bzw. Tierarzt festgelegt. Jedoch liegt eine wirksame Menge einer erfindungsgemäßen Verbindung für die Behandlung im allgemeinen im Bereich von 0,1 bis 100 mg/kg Körpergewicht des Empfängers (Säugers) pro Tag und besonders typisch im Bereich von 1 bis 10 mg/kg Körpergewicht pro Tag. Somit läge für einen 70 kg schweren erwachsenen Säuger die tatsächliche Menge pro Tag für gewöhnlich zwischen 70 und 700 mg, wobei diese Menge als Einzeldosis pro Tag oder üblicher in einer Reihe von Teildosen (wie z.B. zwei, drei, vier, fünf oder sechs) pro Tag gegeben werden kann, so daß die Gesamttagesdosis die gleiche ist. Eine wirksame Menge eines Salzes oder Solvats oder eines physiologisch funktionellen Derivats davon kann als Anteil der wirksamen Menge der erfindungsgemäßen Verbindung *per se* bestimmt werden. Es läßt sich annehmen, daß ähnliche Dosierungen für die Behandlung der anderen, obenerwähnten Krankheitszustände geeignet sind.

Gegenstand der Erfindung sind ferner Arzneimittel enthaltend mindestens eine erfindungsgemäße Verbindung und/oder ihre pharmazeutisch verwendbaren Salze, Solvate und Stereoisomere, einschließlich deren Mischungen in allen Verhältnissen, und mindestens einen weiteren Arzneimittelwirkstoff.

Gegenstand der Erfindung ist auch ein Set (Kit), bestehend aus getrennten Packungen von
(a) einer wirksamen Menge an einer erfindungsgemäßen Verbindung und/oder ihrer pharmazeutisch verwendbaren Salze, Solvate und Stereoisomere, einschließlich deren Mischungen in allen Verhältnissen,
   und
(b) einer wirksamen Menge eines weiteren Arzneimittelwirkstoffs.

Das Set enthält geeignete Behälter, wie Schachteln oder Kartons, individuelle Flaschen, Beutel oder Ampullen. Das Set kann z.B. separate Ampullen enthalten, in denen jeweils eine wirksame Menge an einer erfindungsgemäßen Verbindung und/oder ihrer pharmazeutisch verwendbaren Salze, Solvate und Stereoisomere, einschließlich deren Mischungen in allen Verhältnissen,
und einer wirksamen Menge eines weiteren Arzneimittelwirkstoffs gelöst oder in lyophylisierter Form vorliegt.

### VERWENDUNG

Die vorliegenden Verbindungen eignen sich als pharmazeutische Wirkstoffe für Säugetiere, insbesondere für den Menschen, bei der Behandlung von SGK-bedingten Krankheiten.

Gegenstand der Erfindung ist somit die Verwendung von Verbindungen nach Anspruch 1, sowie ihrer pharmazeutisch verwendbaren Salze, Solvate und Stereoisomere, einschließlich deren Mischungen in allen Verhältnissen, zur Herstellung eines Arzneimittels zur Behandlung von Krankheiten, bei denen die Hemmung, Regulierung und/oder Modulation der Signaltransduktion von Kinasen eine Rolle spielt.
Bevorzugt ist hierbei SGK.

Bevorzugt ist die Verwendung von Verbindungen gemäß Anspruch 1, sowie ihrer pharmazeutisch verwendbaren Salze, Solvate und Stereoisomere, einschließlich deren Mischungen in allen Verhältnissen, zur Herstellung eines Arzneimittels zur Behandlung von Krankheiten, die durch Inhibierung der SGK durch die Verbindungen nach Anspruch 1 beeinflußt werden.

Die vorliegende Erfindung umfasst die Verwendung der erfindungsgemäßen Verbindungen nach Anspruch 1 und/oder ihre physiologisch unbedenklichen Salze und Solvate zur Herstellung eines Arzneimittels zur Behandlung oder Vorbeugung von Diabetes (z.B. Diabetes mellitus, diabetische Nephropathie, diabetische Neuropathie, diabetische Angiopathie und Mikroangiopathie), Fettsucht, metabolisches Syndrom (Dyslipidämie), systemische und pulmonale Hypertonie, Herzkreislauferkrankungen (z.B. kardiale Fibrosen nach Myokardinfarkt, Herzhypertrophie und Herzinsuffizienz, Arteriosklerose) und Nierenerkrankungen (z.B. Glomerulosklerose, Nephrosklerose, Nephritis, Nephropathie, Störung der Elektrolytausscheidung), allgemein bei jeglicher Art von Fibrosen und entzündlichen Prozessen (z.B. Leberzirrhose, Lungenfibrose, fibrosierende Pankreatitis, Rheumatismus und Arthrosen, Morbus Crohn, chronische Bronchitis, Strahlenfibrose, Sklerodermitis, zystische Fibrose, Narbenbildung, Morbus Alzheimer).

Die erfindungsgemäßen Verbindungen können auch das Wachstum von Krebs, Tumorzellen und Tumormetastasen hemmen und sind deshalb für die Tumortherapie geeignet.

Die erfindungsgemäßen Verbindungen finden weiterhin Verwendung zur Behandlung von Koagulopathien, wie z.B. Dysfibrinogenämie, Hypoprokonvertinämie, Hämophilie B, Stuart-Prower-Defekt, ProthrombinKomplex-Mangel, Verbrauchskoagulopathie, Hyperfibrinolyse, lmmunokoagulopathie oder komplexer Koagulopathien, wie auch bei neuronaler Erregbarkeit, z.B. Epilepsie. Die erfindungsgemäßen Verbindungen können auch bei der Behandlung eines Glaukoms oder Katarakt therapeutisch eingesetzt werden.

Die erfindungsgemäßen Verbindungen finden ferner Verwendung bei der Behandlung bakterieller Infektionen sowie in einer antiinfektiösen Therapie. Die erfindungsgemäßen Verbindungen können auch zur Steigerung der Lernfähigkeit und Aufmerksamkeit therapeutisch eingesetzt werden.

Bevorzugt ist die Verwendung von Verbindungen gemäß Anspruch 1, sowie ihrer pharmazeutisch verwendbaren Salze, Solvate und Stereoisomere, einschließlich deren Mischungen in allen Verhältnissen, zur Herstellung eines Arzneimittels zur Behandlung oder Vorbeugung von Diabetes, Fettsucht, metabolischem Syndrom (Dyslipidämie), systemischer und pulmonaler Hypertonie, Herzkreislauferkrankungen und Nierenerkrankungen, allgemein bei jeglicher Art von Fibrosen und entzündlichen Prozessen, Krebs, Tumorzellen, Tumormetastasen, Koagulopathien, neuronaler Erregbarkeit, Glaukom, Katarakt, bakteriellen Infektionen sowie in einer antiinfektiösen Therapie, zur Steigerung der Lernfähigkeit und Aufmerksamkeit, sowie zur Behandlung und Prophylaxe von Zellalterung und Stress.

Bei Diabetes handelt es sich vorzugsweise um Diabetes mellitus, diabetische Nephropathie, diabetische Neuropathie, diabetische Angiopathie und Mikroangiopathie.

Bei Herzkreislauferkrankungen handelt es sich vorzugsweise um kardiale Fibrosen nach Myokardinfarkt, Herzhypertrophie, Herzinsuffizienz und Arteriosklerose.

Bei Nierenerkrankungen handelt es sich vorzugsweise um Glomerulosklerose, Nephrosklerose, Nephritis, Nephropathie und Störung der Elektrolytausscheidung.

Bei Fibrosen und entzündlichen Prozessen handelt es sich vorzugsweise um Leberzirrhose, Lungenfibrose, fibrosierende Pankreatitis, Rheumatismus und Arthrosen, Morbus Crohn, chronische Bronchitis, Strahlenfibrose, Sklerodermitis, zystische Fibrose, Narbenbildung, Morbus Alzheimer.

### ASSAYS

Die in den Beispielen beschriebenen erfindungsgemäßen Verbindungen wurden in den unten beschriebenen Assays geprüft, und es wurde gefunden, dass sie eine kinasehemmende Wirkung aufweisen. Weitere Assays sind aus der Literatur bekannt und könnten vom Fachmann leicht durchgeführt werden (siehe z.B. Dhanabal et al., Cancer Res. 59:189-197; Xin et al., J. Biol. Chem. 274:9116-9121; Sheu et al., Anticancer Res. 18:4435-4441; Ausprunk et al., Dev. Biol. 38:237-248; Gimbrone et al., J. Natl. Cancer Inst. 52:413-427; Nicosia et al., In Vitro 18:538- 549).

Die Hemmung der SGK1 Proteinkinase kann im Filterbindungsverfahren bestimmt werden.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation. Rf-Werte an Kieselgel; Laufmittel: Ethylacetat/Methanol 9:1.
Massenspektrometrie (MS): EI (Elektronenstoß-lonisation) M⁺
   FAB (Fast Atom Bombardment) (M+H)⁺
   ESI (Electrospray lonization) (M+H)⁺ (wenn nichts anderes angegeben)

### HPLC-Methode

Hewlett Packard System der HP 1100 Serie mit den folgenden Merkmalen: lonenquelle: Elektrospray (positive mode); Scan: 100-1000 m/z; Fragmentierspannung: 60 V; Gastemperatur: 300°C, DAD: 220 nm.

Flußrate: 2.4 ml/min. Der verwendete Splitter reduziert nach dem DAD die Flußrate für das MS auf 0.75 ml/min.

### Säule:

Chromolith Speed ROD
RP-18e 50-4,6 mm
Lösungsmittel: LiChrosolv-Qualität der Fa. Merck KGaA
Lösungsmittel A: H₂O (0.01 % TFA)
Lösungsmittel B: Acetonitril (0.008% TFA)

### Gradient:

20% B → 100% B: 0 min. bis 2.8 min.
100% B: 2.8 min. bis 3.3 min.
100% B → 20% B: 3.3 min. bis 4 min.
Gradient für Bedingung "polar":
5% B → 100% B: 0 min. bis 3 min.
100% B: 3 min. bis 3.5 min.
100% B → 5% B: 3.5 min. bis 3.6 min.

### Abkürzungen:

DCM = Dichlormethan
EE = Essigsäureethylester
PE = Petrolether
RT = Raumtemperatur
DAPECI = N-(3-Dimethylaminopropyl)-N'-ethylcarbodiimidhydrochlorid
DMF = Dimethylformamid
HOBT = 1-Hydroxybenzotriazol
NCS = N-Chlorsuccinimid
TFA = Trifluoressigsäure

Um die Erfindung zu erläutern, sind die folgenden Beispiele beigefügt. Es versteht sich jedoch, dass diese Beispiele die Erfindung nicht einschränken.

### Beispiel 1

Die Herstellung von 5-(5-Benzyl-[1,3,4]oxadiazol-2-yl)-1*H-*indazol-3-ylamin ("A1") erfolgt analog nachstehendem Schema (Methode 1)

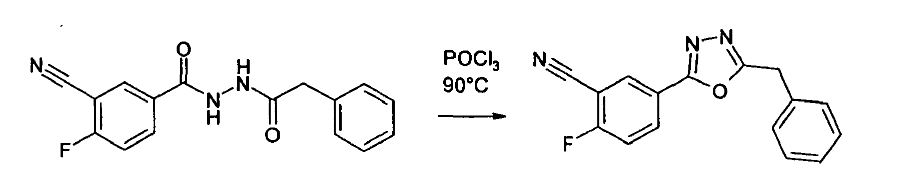

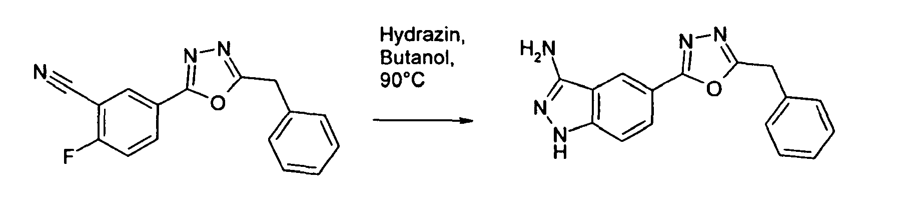

### Schritt 1:

110 mg 3-Cyan-4-fluor-benzoesäure-N'-phenylacetyl-hydrazid werden in 1,5 ml Phosphoroxidtrichlorid 1 Stunde bei 90°C gerührt. Nach dem Abkühlen wird die klare Lösung auf Wasser gegossen und der ausgefallene Feststoff abgesaugt und getrocknet. Man erhält 69 mg 5-(5-Benzyl-[1,3,4]oxadiazol-2-yl)-2-fluor-benzonitril (67%); MS-FAB (M+H⁺) = 280,4; R_{f} (Methode polar): 2,15 Min.

### Schritt 2:

In einem Schraubdeckelgläschen wird eine Mischung von 69 mg 5-(5-Benzyl-[1,3,4]oxadiazol-2-yl)-2-fluoro-benzonitril und 25 mg Hydrazinhydrat in 3 ml 1-Butanol 12 Stunden bei 90°C gerührt. Nach dem Abkühlen wird der ausgefallene Feststoff abgesaugt, mit Wasser gewaschen und lyophilisiert. Man erhält 51 mg 5-(5-Benzyl-[1,3,4]oxadiazol-2-yl)-1H indazol-3-ylamin (71%); MS-FAB (M+H⁺) = 292,3; R_{f} (Methode polar): 1,55 Min.

Die als Ausgangstoffe dienenden Diacylhydrazide sind teilweise kommerziell erhältlich. Sie können alternativ nach den im folgenden angegebenen Methoden 1a und 1 b hergestellt werden.

Die Herstellung von 3-Cyan-4-fluor-benzoesäure-N'-phenylacetyl-hydrazid erfolgt analog nachstehendem Schema **(Methode 1a)**

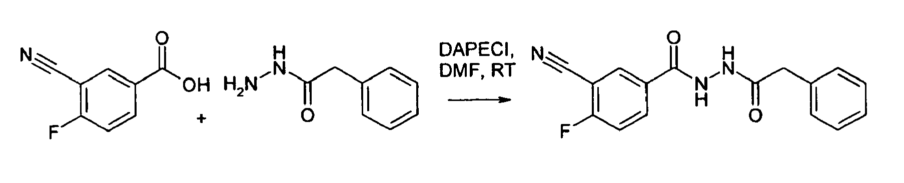

800 mg 3-Cyan-4-fluor-benzoesäure, 728 mg Phenylessigsäurehydrazid und 1054 mg DAPECI werden in 3 ml DMF 2 Tage bei RT gerührt. Die Reaktionsmischung wird auf ca. 20 ml einer 10% Natriumhydrogencarbonatlösung gegossen, der ausgefallene Feststoff abgesaugt und getrocknet. Man erhält 1200 mg 3-Cyan-4-fluor-benzoesäure-N'-phenylacetyl-hydrazid (83%) als farblosen Feststoff; MS-FAB (M+H⁺) = 298,4.

Die Herstellung von 3-Cyan-4-fluor-benzoesäure-N'-[3-(2,5-difluor-phenyl)-propionyl]-hydrazid erfolgt analog nachstehendem Schema **(Methode 1b)**

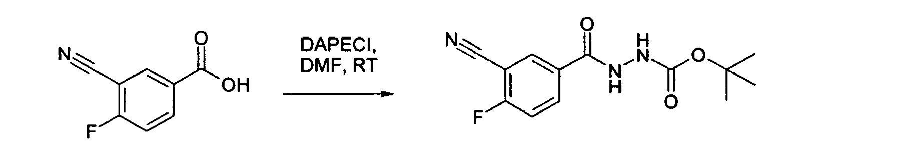

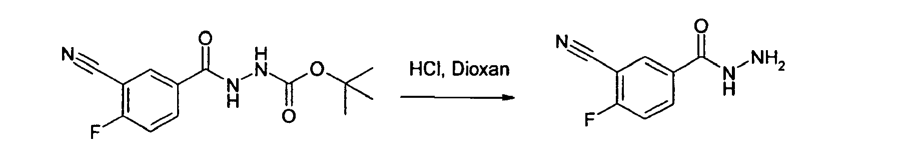

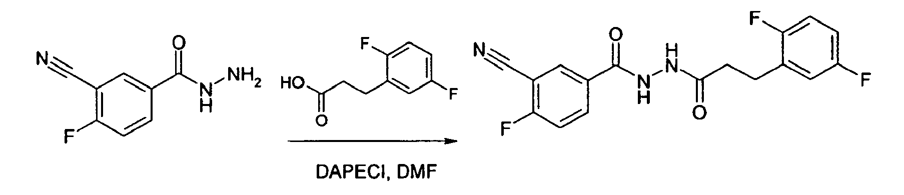

### Schritt 1:

900 mg 3-Cyan-4-fluor-benzoesäure, 727 mg tert.-Butylcarbazat und 1342 mg DAPECI werden in 10 ml DMF 12 Stunden gerührt. Die Reaktionsmischung wird auf ca. 40 ml einer 10% Natriumhydrogencarbonatlösung gegossen, der ausgefallene Feststoff abgesaugt und getrocknet. Man erhält 1140 mg N'-(3-Cyan-4-fluor-benzoyl)-hydrazincarbonsäure-tert.-butyl-ester (75%) als farblosen Feststoff; MS-FAB (M+H⁺-BOC) = 180,3; R_{f} (Methode polar): 1,23 Min.

### Schritt 2:

1140 mg N'-(3-Cyano-4-fluoro-benzoyl)-hydrazincarbonsäure-tert.-butylester werden mit 20 HCl(4N)/Dioxan und 10 ml Methanol versetzt und 1 Stunde bei RT gerührt. Die Reaktionsmischung wird zum Rückstand eingeengt und mit einem Überschuß gesättigter Natriumhydrogencarbonatlösung versetzt. Man extrahiert mehrfach mit DCM, trocknet die vereinigten org. Phasen mit Natriumsulfat und entfernt das Lösungsmittel. Es werden 650 mg 3-Cyan-4-fluor-benzoehydrazid (89%) in Form der freien Base erhalten.

### Schritt 3:

100 mg 3-Cyan-4-fluor-benzoehydrazid, 104 mg 3-(2,5-Difluor-phenyl)-propionsäure und 138 mg DAPECI werden in 8 ml DMF 12 Stunden gerührt. Die Reaktionsmischung wird auf ca. 40 ml einer 10% Natriumhydrogencarbonatlösung gegossen, der ausgefallene Feststoff abgesaugt und getrocknet. Man erhält 136 mg 3-Cyan-4-fluor-benzoesäure-N'-[3-(2,5-difluor-phenyl)-propionyl]-hydrazid (70%) als farblosen Feststoff; MS-FAB (M+H⁺) = 348,4; R_{f} (Methode polar): 1,44 Min.

Das erhaltene 3-Cyan-4-fluor-benzoesäure-N'-[3-(2,5-difluor-phenyl)-propionyl]-hydrazid wird nach Methode 1 in 5-{5-[2-(2,5-Difluor-phenyl)-ethyl]-[1,3,4]oxadiazol-2-yl}-1*H-*indazol-3-ylamin ("A6") überführt: 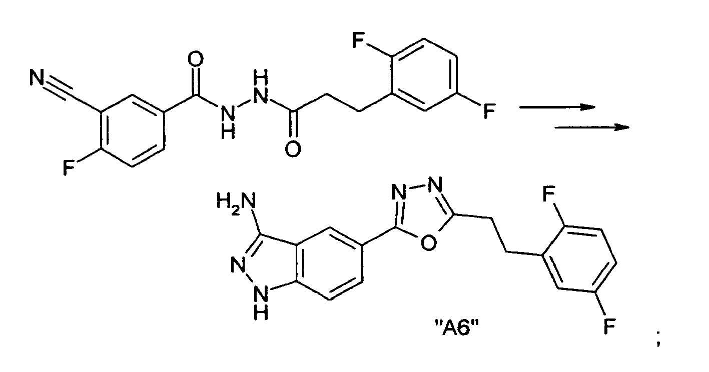 MS-FAB (M+H⁺) = 342,32; R_{f} (Methode polar): 1,37 Min.
Analog Methode 1 werden die nachstehenden Verbindungen erhalten

| Nr. | Strukturformel und/oder Name | M+H⁺ | Rf [min] HPLC-Methode polar |
|---|---|---|---|
| "A2" | 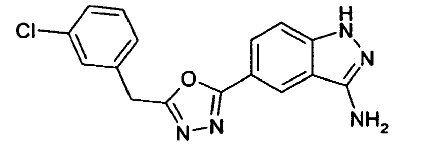 | 326,76 | 1,74 |
| "A3" | 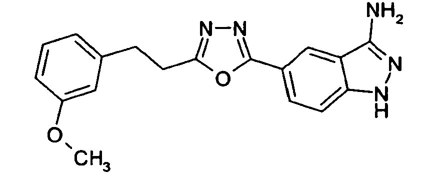 | 336,37 | 1,28 |
| ¹H-NMR (DMSO-d₆): δ [ppm] 11,79 (1H, s, br), 8,45 (1H, s), 7,80 (1H, dd, J = 8,7 und 1,9 Hz), 7,38 (1H, d, J = 8,7 Hz), 7,21 (1H, t, J = 7,9 Hz), 6,82-6,89 (2H, m), 6,73-6,80 (1H, m), 5,66 (2H, s, br), 3,71 (3H, s), 3,31 (4H, s). | | | |
| "A4" | 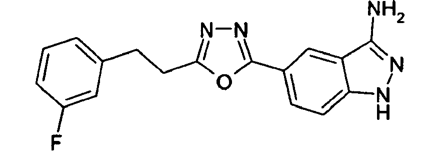 | 324, 33 | 1,35 |
| "A5" | 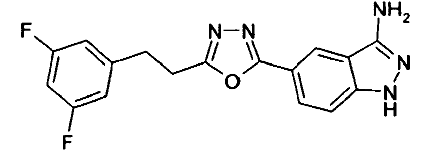 | 342,32 | 1,42 |
| "A7" | 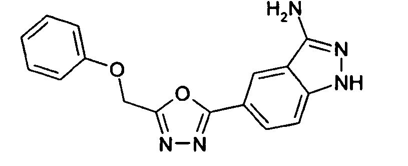 | 308,31 | 1,67 |
| "A8" | 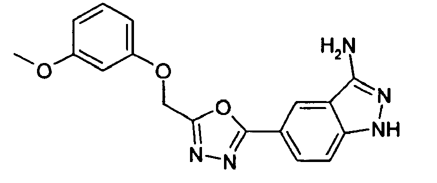 | 338,34 | 1,7 |
| "A9" | 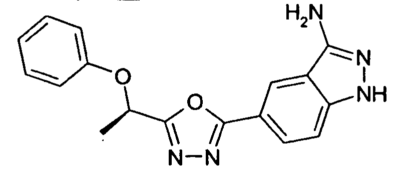 5-[5-((R)-1-Phenoxy-ethyl)-[1,3,4]oxadiazol-2-yl]-1*H-*indazol-3-ylamin | 322,34 | 1,74 |
| "A10" | 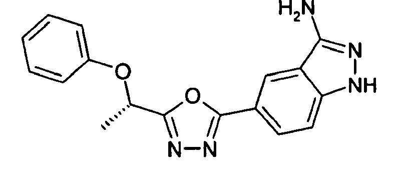 | 322,34 | 1,74 |
| "A11" | 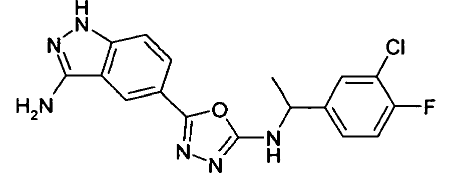 | 373,79 | 1,77 |
| "A12" | 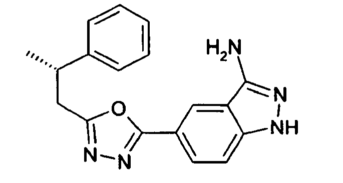 | 320,37 | 1,79 |
| "A13" | 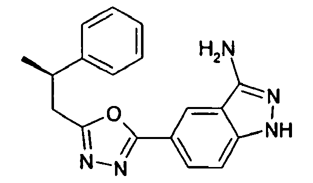 | 320,37 | 1,79 |
| "A14" | 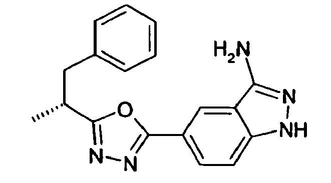 | 320,37 | 1,8 |
| "A15" | 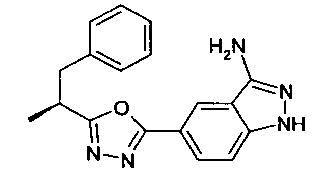 | 320,37 | 1,8 |
| "A16" | 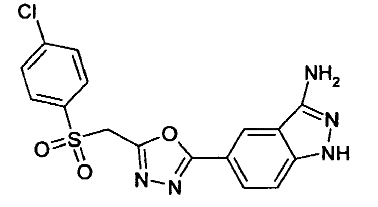 5-[5-(4-Chlor-benzolsulfonylmethyl)-[1,3,4]oxadiazol-2-yl]-1*H-*indazol-3-ylamin | 390,82 | 1,9 |
| "A17" | 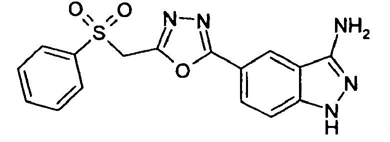 | 356,38 | 1,44 |
| | | | |

### Beispiel 2

Die Herstellung von 5-{5-[(R)-1-(3-Methoxy-phenyl)-ethylamino]-[1,3,4]oxadiazol-2-yl}-1*H-*indazol-3-ylamin ("A22") erfolgt analog nachstehendem Schema **(Methode 2)**

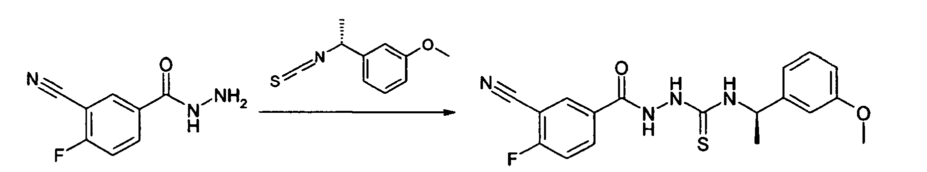

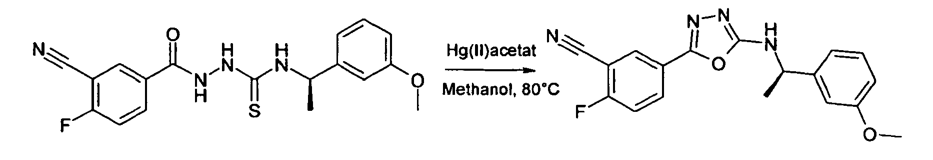

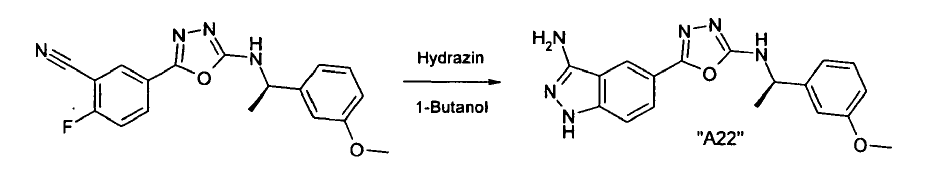

### Schritt 1:

179 mg 3-Cyan-4-fluor-benzoesäurehydrazid und 193 mg 1-((R)-1-Isothiocyanato-ethyl)-3-methoxy-benzol werden in 10 ml Dichlormethan bei RT 18 Stunden gerührt. Nach Zugabe von weiteren 50 mg 1-((R)-1-Isothiocyanato-ethyl)-3-methoxy-benzol wird erneut 5 Stunden gerührt. Die Reaktionslösung wird eingeengt, der Rückstand mit Diethylether und PE verrührt und abgesaugt. Nach dem Trocknen erhält man 350 mg Additionsprodukt [(94%); MS-FAB (M+H⁺) = 373,5; R_{f} (Methode polar): 2,02 Min.], das ohne weitere Reinigung im Folgeschritt eingesetzt wird.

### Schritt 2:

350 mg des Kupplungsprodukts aus Schritt 1 und 373 mg Quecksilber(II)-acetat werden in 10 ml Methanol bei 80°C 2 Stunden gerührt. Der abgekühlte Ansatz wird mit einigen Tropfen einer Natriumsulfidlösung versetzt. Die Suspension wird über Kieselgur filtriert und mit Methanol nachgewaschen. Man erhält 300 mg 2-Fluor-5-{5-[(R)-1-(3-methoxyphenyl)-ethylamino]-[1,3,4]oxadiazol-2-yl}-benzonitril [(94%); MS-FAB (M+H⁺) = 339,5; R_{f} (Methode polar): 2,08 Min.], das ohne weitere Reinigung im Folgeschritt eingesetzt wird.

### Schritt 3:

Eine Mischung von 300 mg 2-Fluor-5-{5-[(R)-1-(3-methoxy-phenyl)-ethyl-amino]-[1,3,4]oxadiazol-2-yl}-benzonitril und 250 mg Hydrazinhydrat wird in 2 ml 1-Butanol 1 Stunde bei 90°C gerührt. Nach dem Abkühlen wird der ausgefallene Feststoff abgesaugt, mit Wasser gewaschen und getrocknet. Reinigung durch Säulenchromatographie ergibt 46 mg 5-{5-[(R)-1-(3-Methoxy-phenyl)-ethylamino]-[1,3,4]oxadiazol-2-yl}-1*H-*indazol-3-ylamin) ("A22") (15%); MS-FAB (M+H⁺) = 351,5; R_{f} (Methode polar): 1,55 Min.

Analog Methode 2 werden die nachstehenden Verbindungen erhalten

| Nr. | Strukturformel und/oder Name | m+H⁺ | Rf [min] HPLC-Methode polar |
|---|---|---|---|
| "A18" | 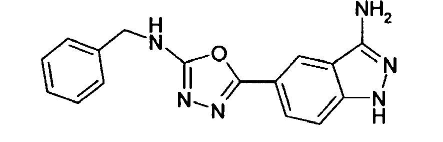 | 307,33 | 0,99 |
| "A19" | 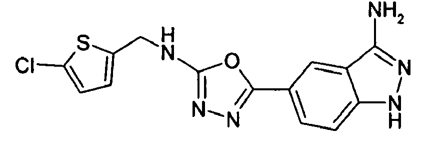 | 347,80 | 1,62 |
| ¹H-NMR (DMSO-d₆): δ [ppm] 11,70 (1H, s, br), 8,25 (1H, t, J = 6,0 Hz), 8,23 (1H, s), 7,69 (1H, dd, J = 9,1 und 1,9 Hz), 7,34 (1H, d, J = 9,1 Hz), 6,98 (2H, s), 5,59 (2H, s, br), 4,55 (2H, d, J = 6,0 Hz). | | | |
| "A20" | 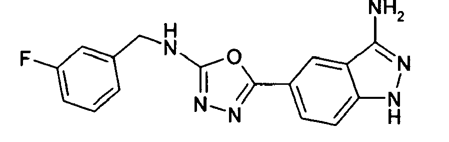 | 325,32 | 1,6 |
| "A21" | 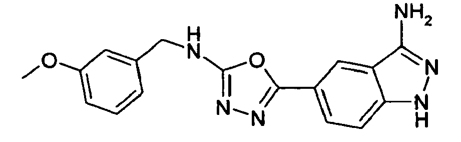 | 337,35 | 1,48 |
| "A23" | 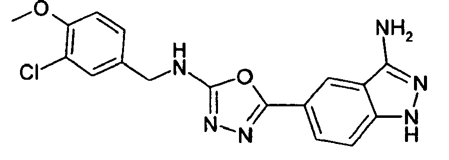 | 371,80 | 1,6 |
| "A24" | 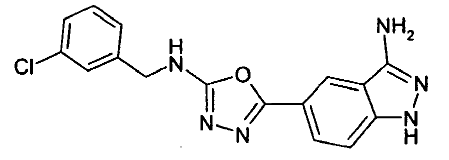 | 341,77 | 1,61 |
| "A25" | 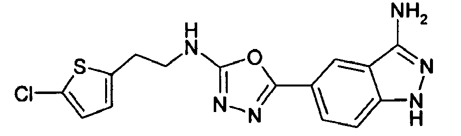 | 361,83 | 1,67 |
| "A26" | 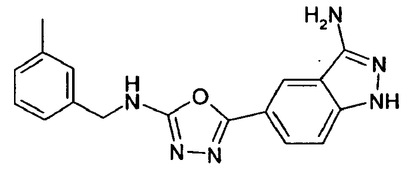 | 321,35 | 1,58 |
| "A27" | 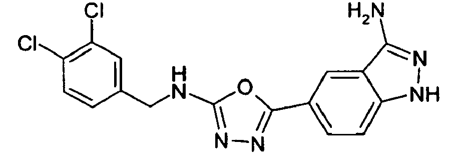 | 376,22 | 1,74 |
| "A28" | 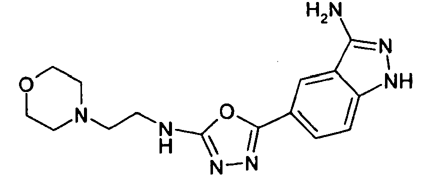 | 330,36 | 0,81 |
| "A29" | 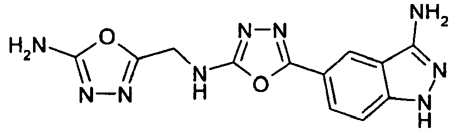 | 314,28 | 0,89 |
| "A30" | 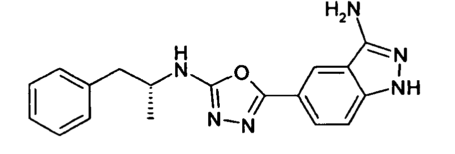 | 335,38 | 1,6 |
| "A31" | 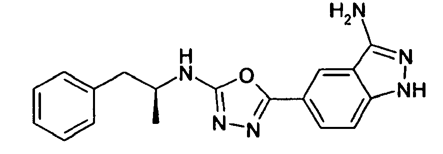 | 335,38 | 1,6 |
| "A32" | 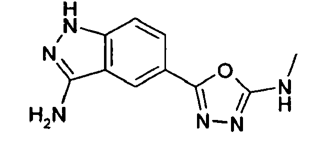 | 231,23 | 0,98 |
| "A33" | 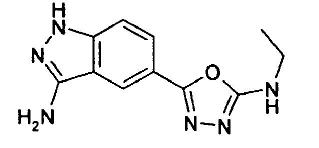 | 245,26 | 1,1 |
| "A34" | 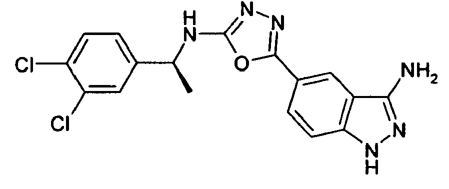 | 390,24 | 1,84 |
| "A35" | 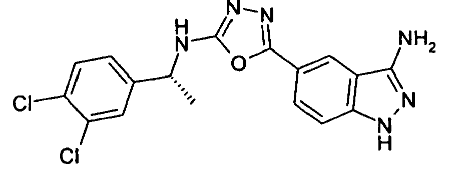 | 390,24 | 1,84 |
| "A36" | 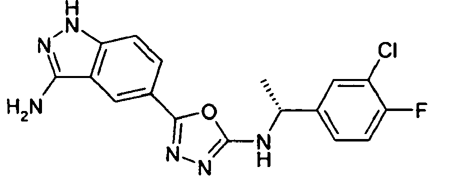 | 373,79 | 1,77 |
| "A37" | 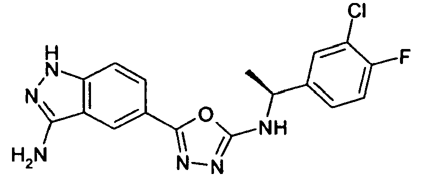 | 373,79 | 1,77 |

### Beispiel 3

Die Herstellung von [5-(4-Fluor-1*H-*indazol-5-yl)-[1,3,4]oxadiazol-2-yl]-(3-methoxy-benzyl)-amin ("A60") erfolgt analog nachstehendem Schema

### (Methode 3)

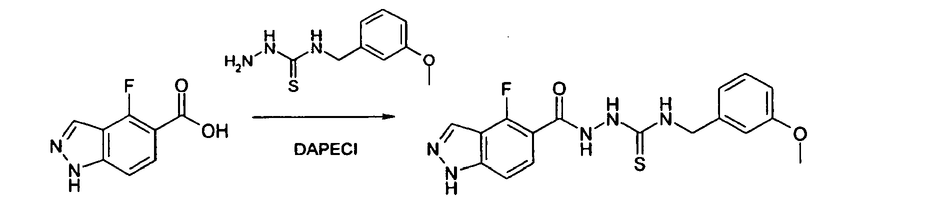

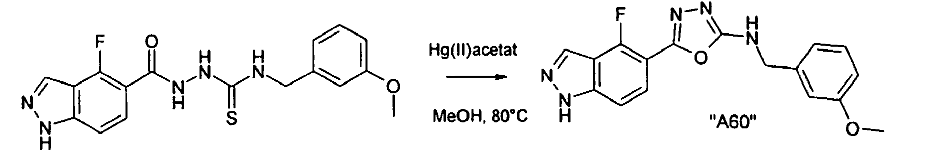

### Schritt 1:

400 mg 4-Fluor-1H-indazol-5-carbonsäure, 470 mg 4-(3-Methoxybenzyl)-thiosemicarbazid und 468 mg DAPECI werden in 5 ml DMF 24 Stunden bei RT gerührt. Der Ansatz wird auf 1 N Salzsäure gegossen und der entstandene Niederschlag abgesaugt und getrocknet. Man erhält 410 mg Produkt [49%, MS-FAB (M+H⁺) = 374,1; R_{f}(Methode polar): 1,69 Min], das ohne weitere Reinigung in Schritt 2 eingesetzt wird.

### Schritt 2:

410 mg des Produkts aus Schritt 1 und 455 mg Quecksilber(II)acetat werden in 6 ml Methanol 1 Stunde bei 80°C gerührt. Der abgekühlte Ansatz wird mit einigen Tropfen einer Natriumsulfidlösung versetzt. Die Suspension wird über Kieselgur filtriert und mit Methanol nachgewaschen. Das Filtrat wird eingeengt und der Rückstand durch Säulenchromatographie an Kieselgel (Eluent: Heptan/EE) gereinigt. Man erhält 300 mg [5-(4-Fluor-1*H-*indazol-5-yl)-[1,3,4]oxadiazol-2-yl]-(3-methoxy-benzyl)-amin ("A60") 81%; MS-FAB (M+H⁺) = 340,3; R_{f} (Methode polar): 1,70 Min.

Die bei dieser Synthesesequenz benötigten Thiosemicarbazide sind aus entsprechenden Isothiocyanaten und Hydrazin leicht zugänglich; z.B.:

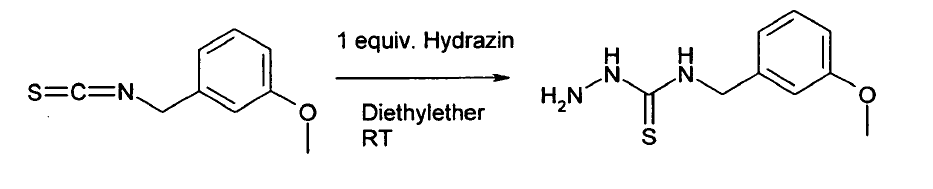

Alternativ können bei Methode 3 auch an N1 geschützte Imidazol-5-carbonsäuren eingesetzt werden (z.B. BOC - geschützte Derivate). Die Schutzgruppe muß dann in einem Folgeschritt durch dem Fachmann bekannte Methoden abgespalten werden.

So erfolgt die Herstellung von [5-(4-Chlor-1*H-*indazol-5-yl)-[1,3,4]oxadiazol-2-yl]-(3-methoxy-benzyl)-amin ("A57"), MS-FAB (M+H⁺) = 356,78; R_{f} (Methode polar): 1,77 Min.
wie im folgenden Schema angegeben:

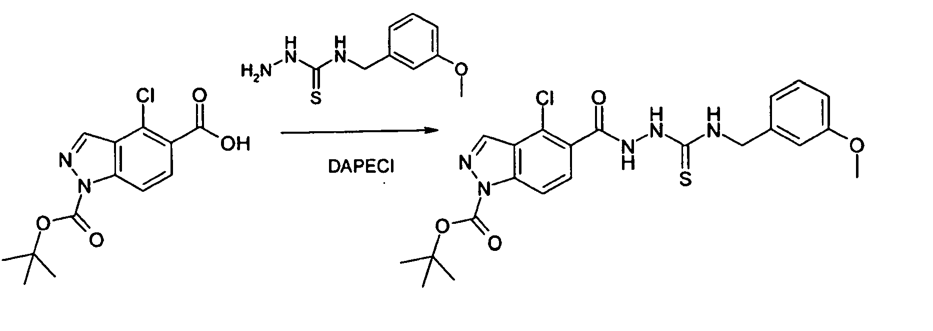

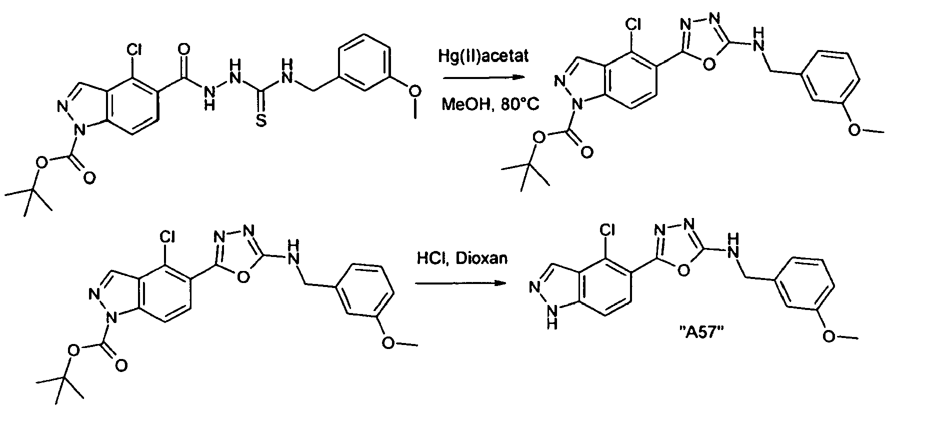

Analog Methode 3 werden die nachstehenden Verbindungen erhalten

| Nr. | Strukturformel und/oder Name | M+H⁺ | Rf [min] HPLC-Methode polar |
|---|---|---|---|
| "A38" | 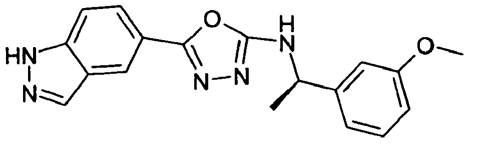 | 336,37 | 1,72 |
| "A39" | 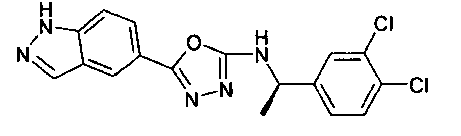 | 375,23 | 2,03 |
| "A40" | 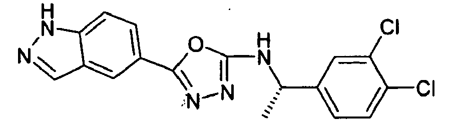 | 375,23 | 2,03 |
| "A41" | 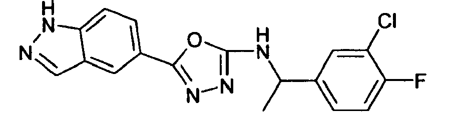 | 358,77 | 1,95 |
| "A42" | 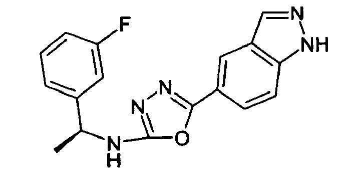 | 324,33 | 2,01 |
| "A43" | 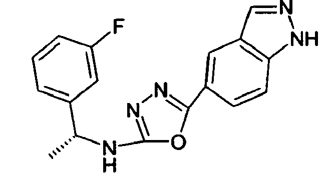 | 324,33 | 2,01 |
| "A44" | 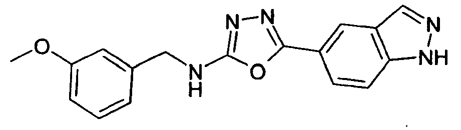 | 322,34 | 1,63 |
| "A45" | 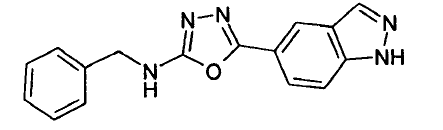 | 292,31 | 1,64 |
| "A46" | 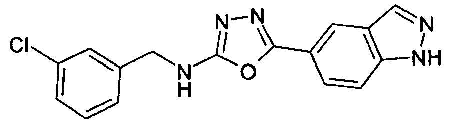 | 326,76 | 1,81 |
| "A47" | 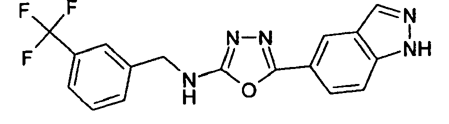 | 360, 31 | 2,14 |
| "A48" | 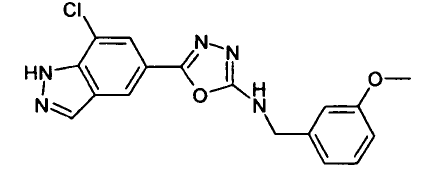 | 356,78 | 1,79 |
| "A49" | 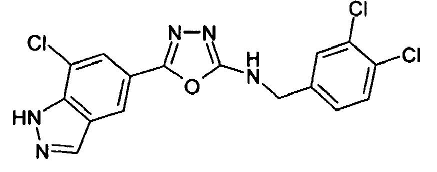 | 395,65 | 2,1 |
| "A50" | 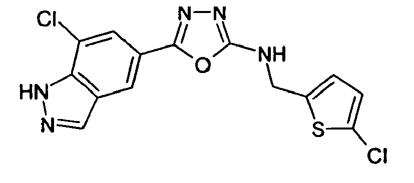 | 367,23 | 1,95 |
| "A51" | 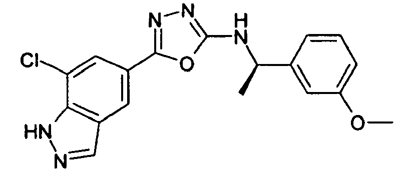 | 370,81 | 1,87 |
| "A52" | 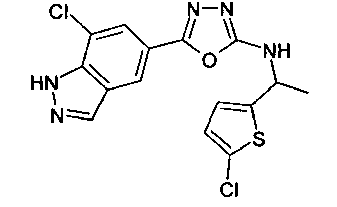 | 381,26 | 2,06 |
| "A53" | 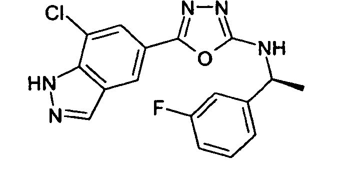 | 358,7 | 1,91 |
| "A54" | 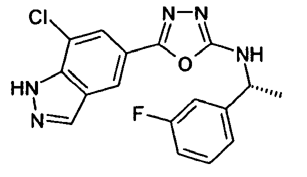 | 358,77 | 1,91 |
| "A55" | 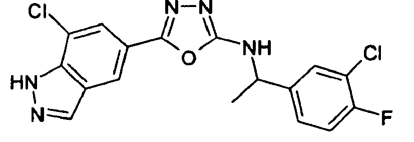 | 393,22 | 2,09 |
| "A56" | 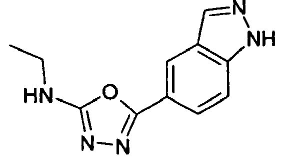 | 230,24 | 1,46 |
| "A58" | 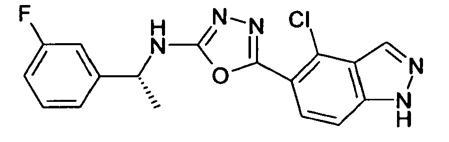 | 358,77 | 1,89 |
| "A59" | 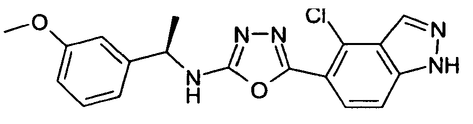 | 370,81 | 1,86 |
| ¹H-NMR (DMSO-d₆, TFA-Austausch): δ [ppm] 8,22 (1H, s, br), 7,75 (1H, d, J = 8,7 Hz), 7,64 (1H, d, J = 8,7 Hz)), 7,24 (1H, t, J = 8,0 Hz), 6,98-7,03 (2H, m), 6,81 (1H, dd, J = 8,3 und 2,2 Hz)), 4,83 (1H, q, J = 7,0 Hz), 3,73 (3H, s), 1,56 (3H, d, J = 7,0 Hz). | | | |
| "A61" | 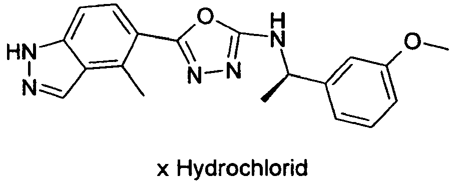 | 386,85 | |
| "A62" | 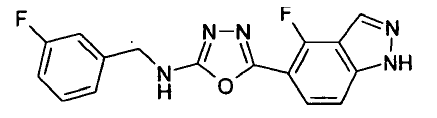 | 328,29 | 1,74 |
| "A63" | 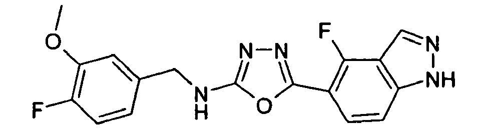 | 358,32 | 1,74 |
| "A64" | 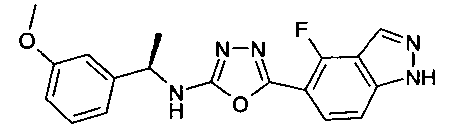 | 354,36 | 1,79 |
| "A65" | 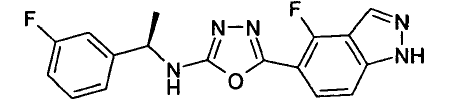 | 342,32 | 1,82 |
| "A66" | 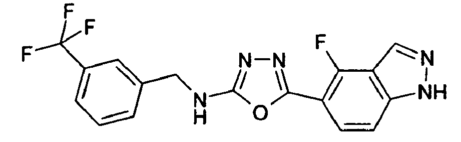 | 378,30 | 1,95 |

### Beispiel 4

Die Herstellung von 5-{5-[2-(3-Fluor-phenyl)-ethyl]-[1,3,4]oxadiazol-2-yl}-1*H-*indazol ("A68") erfolgt analog nachstehendem Schema **(Methode 4)**

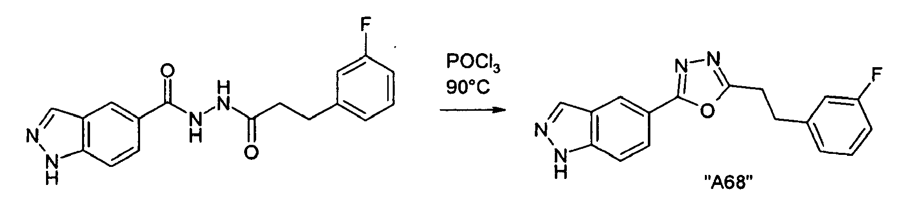

150 mg 1H-Indazol-5-carbonsäure-N'-[3-(3-fluor-phenyl)-propionyl]-hydrazid werden in 3 ml Phosphoroxidtrichlorid 2 Stunden bei 80°C gerührt. Das überschüssige Phosphoroxidtrichlorid wird abdestilliert und der Rückstand mit verdünnter Salzsäure behandelt. Der erhaltene Feststoff wird abfiltriert, getrocknet und durch Chromatographie an Kieselgel RP-18 gereinigt. Man 9 mg "A68" (6%); MS-FAB (M+H⁺) = 309,3; R_{f} (Methode polar): 1,97 Min.

Analog erhält man 5-[5-(4-Chlor-benzolsulfonylmethyl)-[1,3,4]oxadiazol-2-yl]-1*H*-indazol ("A67"), MS-FAB (M+H⁺) = 375,81; R_{f} (Methode polar): 1,81 Min;

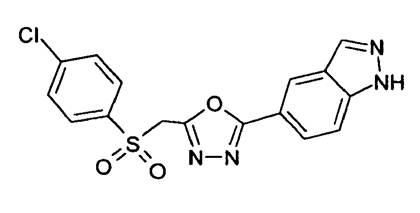

### Beispiel 5

Die Herstellung von [5-(3-Chlor-1*H-*indazol-5-yl)-[1,3,4]oxadiazol-2-yl]-(3-methoxy-benzyl)-amin ("A69") erfolgt analog nachstehendem Schema

### (Methode 5)

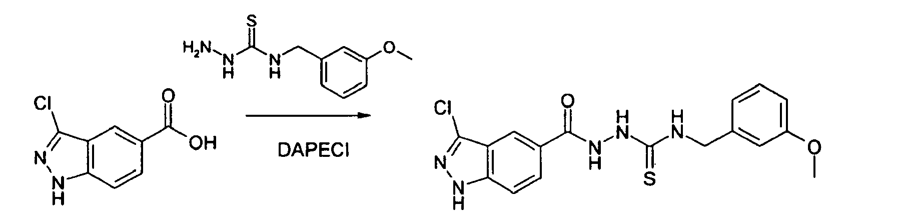

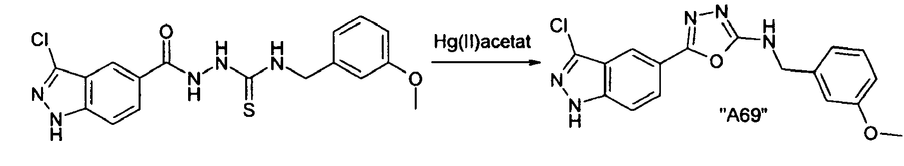

### Schritt 1:

200 mg 3-Chlor-1*H-*indazol-5-carbonsäure, 214 mg 4-(3-Methoxybenzyl)-thiosemicarbazid, 171 mg HOBT und 215 mg DAPECI werden in 3 ml DMF 24 Stunden bei RT gerührt. Der Ansatz wird auf verdünnte Salzsäure gegossen und der entstandene Niederschlag abgesaugt und getrocknet. Man erhält 270 mg Produkt [68%, MS-FAB (M+H⁺) = 390,8; R_{f} (Methode polar): 1,80 Min], das ohne weitere Reinigung in Schritt 2 eingesetzt wird.

### Schritt 2:

270 mg des Produkts aus Schritt 1 und 231 mg Quecksilber(II)acetat werden in 4 ml Methanol über Nacht bei 80°C gerührt. Der abgekühlte Ansatz wird mit einigen Tropfen einer Natriumsulfidlösung versetzt. Die Suspension wird über Kieselgur filtriert und mit Methanol nachgewaschen. Das Filtrat wird eingeengt und der Rückstand durch Säulenchromatographie an Kieselgel (Eluent: Heptan/EE) gereinigt. Man erhält 43 mg [5-(3-Chlor-1*H*-indazol-5-yl)-[1,3,4]oxadiazol-2-yl]-(3-methoxy-benzyl)-amin (18%); MS-FAB (M+H⁺) = 356,8; R_{f} (Methode polar): 1,88 Min.

Analog Methode 5 werden die nachstehenden Verbindungen erhalten

| Nr. | Strukturformel und/oder Name | M+H⁺ | Rf [min] HPLC-Methode polar |
|---|---|---|---|
| "A70" | 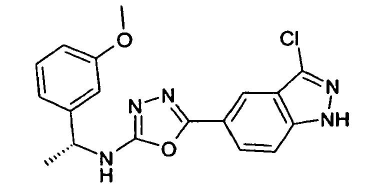 | 370,81 | 1,96 |
| ¹H-NMR (DMSO-d₆): δ [ppm] 13,58 (1H, s), 8,31 (1H, d, J = 8,7 Hz), 7,95 (1H, s), 7,89 (1H, dd, J = 8,5 Hz und 1,5 Hz), 7,71 (1H, d, J = 8,8), 7,25 (1H, t, J = 8,3 Hz), 6,97-7,02 (2H, m), 6,79-6,83 (1H, m), 4,78 (1H, p, J = 7,4), 3,75 (3H, s), 1,49 (3H, d , J = 6,95). | | | |
| "A71" | 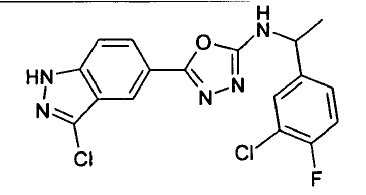 | 393,22 | 2,13 |

### Beispiel 6

Die Herstellung von 5-(4-Chlor-1*H-*indazol-5-yl)-[1,3,4]thiadiazol-2-ylamin ("A72a") erfolgt analog nachstehendem Schema **(Methode 6)**

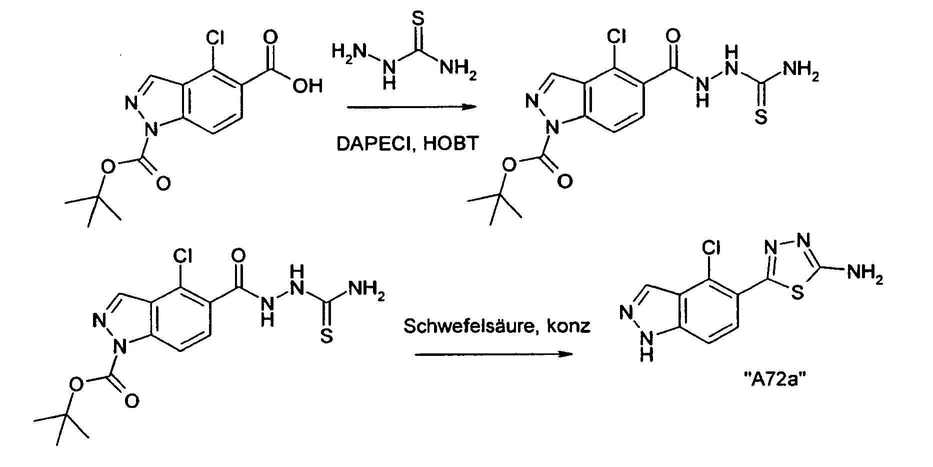

### Schritt 1:

1,00 g 4-Chlor-indazole-1,5-dicarbonsäure-1-tert.-butylester, 307 mg Thiosemicarbazid, 455 mg HOBT und 646 mg DAPECI werden in 5 ml DMF 24 Stunden bei RT gerührt. Der Ansatz wird auf Wasser gegossen und der entstandene Niederschlag abgesaugt und getrocknet. Man erhält 1,01 g Produkt [80%, MS-FAB (M+H⁺) = 370,1; R_{f} (Methode polar): 1,60 Min], das ohne weitere Reinigung in Schritt 2 eingesetzt wird.

### Schritt 2:

200 mg des Kupplungsprodukts aus Schritt 1 werden in 3 ml konz. Schwefelsäure 24 Stunden bei RT gerührt. Der Ansatz wird mit Wasser verdünnt, vorsichtig mit konz. NaOH neutralisiert und dreimal mit Essigester extrahiert. Die organische Phase wird abgetrennt, mit Wasser gewaschen, getrocknet und eingeengt. Reinigung des Rückstands durch präp. HPLC (Chromolith) ergibt 20 mg 5-(4-Chlor-1*H-*indazol-5-yl)-[1,3,4]thiadiazol-2-ylamin (15%); MS-FAB (M+H⁺) = 252,7. R_{f} (Methode polar): 1,30 Min.

Analog Methode 6 erhält man

| Nr. | Strukturformel und/oder Name | M+H⁺ | Rf [min] HPLC-Methode polar |
|---|---|---|---|
| "A72" | 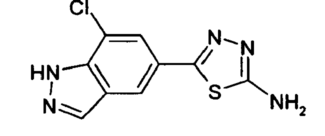 | 252,70 | |

### Beispiel 7

Die Herstellung von 5-(5-Amino-[1,3,4]oxadiazol-2-yl)-1*H-*indazol-3-ylamin ("A73") erfolgt analog nachstehendem Schema **(Methode 7)**

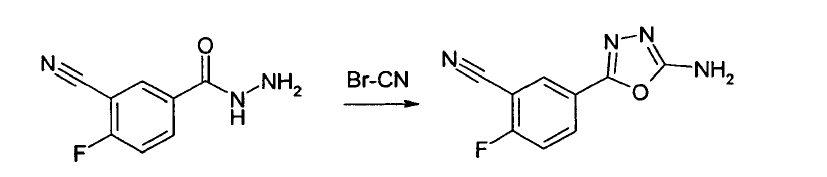

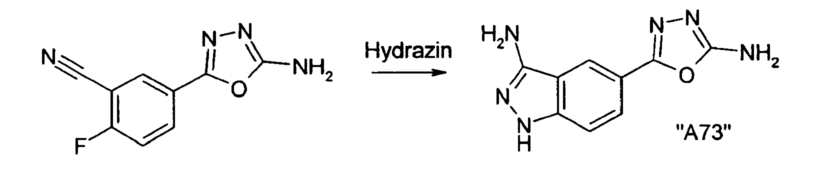

### Schritt 1:

In eine Lösung von 3,0 g Natriumhydrogencarbonat und 3,2 g 3-Cyan-4-fluor-benzoesäurehydrazid in 200 ml Wasser werden portionsweise 1,9 g Bromcyan eingetragen, wobei es zu einer Entwicklung von Kohlendioxid kommt und nach kurzer Zeit ein Feststoff ausfällt. Nach 2 Stunden Rühren bei RT wird abgesaugt, mit Wasser gewaschen und das Produkt getrocknet. Man erhält 1,9 g 5-(5-Amino-[1,3,4]oxadiazol-2-yl)-2-fluor-benzonitril (63%) als farbloses Pulver; MS-FAB (M+H⁺) = 205,2; R_{f} (Methode polar): 1,32 Min.

### Schritt 2:

102 mg 5-(5-Amino-[1,3,4]oxadiazol-2-yl)-2-fluor-benzonitril und 50 mg Hydrazinhydrat werden in 3 ml Butanol bei 90°C 1 Stunde gerührt. Nach Abkühlung auf RT wird der ausgefallene Feststoff abgesaugt und mit Wasser nachgewaschen. Man erhält 28 mg 5-(5-Amino-[1,3,4]oxadiazol-2-yl)-1*H-*indazol-3-ylamin (26%); MS-FAB (M+H⁺) = 217,3; R_{f} (Methode polar): 0,88 Min.

### Beispiel 8

Die Herstellung von 1-[5-(3-Amino-1*H-*indazol-5-yl)-[1,3,4]oxadiazol-2-yl]-3-(3-fluor-benzyl)-harnstoff ("A74") erfolgt analog nachstehendem Schema **(Methode 8)**

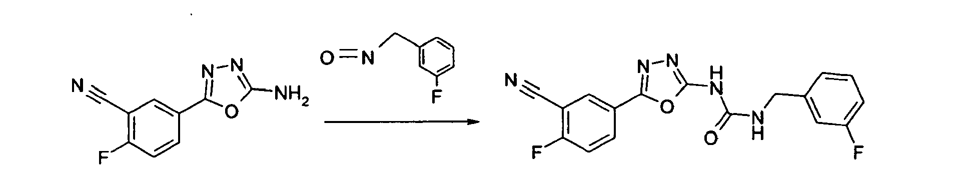

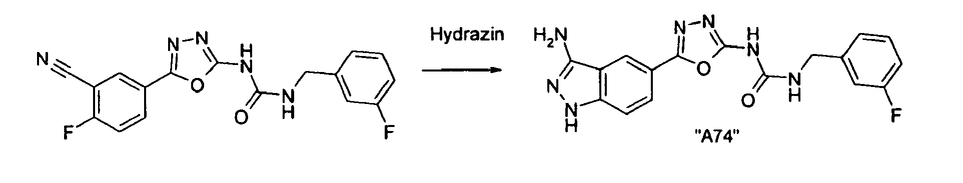

### Schritt 1:

150 mg 5-(5-Amino-[1,3,4]oxadiazol-2-yl)-2-fluor-benzonitril und 113 mg 3-Fuorbenzylisocyanat werden in 2 ml DMF bei RT über Nacht gerührt. Man gibt auf Wasser, saugt den ausgefallenen Feststoff ab und trocknet ihn. Reinigung durch Säulenchromatographie an Kieselgel (Eluent: Heptan/EE) ergibt 50 mg 1-[5-(3-Cyan-4-fluor-phenyl)-[1,3,4]oxadiazol-2-yl)-3-(3-fluorbenzyl)-harnstoff (19%); MS-FAB (M+H⁺) = 356,3; R_{f} (Methode polar): 1,66 Min.

### Schritt 2:

50 mg 1-[5-(3-Cyan-4-fluor-phenyl)-[1,3,4]oxadiazol-2-yl]-3-(3-fluor-benzyl)-harnstoff und 25 mg Hydrazinhydrat werden in 3 ml Butanol bei 90°C 1 Stunde gerührt. Es wird zum Rückstand eingeengt und dieser durch Säulenchromatographie an Kieselgel (Eluent: EE/MeOH) gereinigt. Man erhält 11 mg 1-[5-(3-Amino-1*H-*indazol-5-yl)-[1,3,4]oxadiazol-2-yl]-3-(3-fluor-benzyl)-harnstoff (21 %); MS-FAB (M+H⁺) = 368,3; R_{f} (Methode polar): 1,16 Min.

### Beispiel 9

Die Herstellung von 5-(3-Amino-1*H*-indazol-5-yl)-[1,3,4]oxadiazol-2-carbonsäure-3-fluor-benzylamid ("A75") erfolgt analog nachstehendem Schema **(Methode 9)**

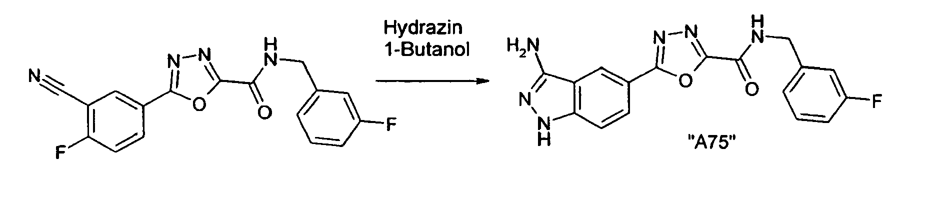

94 mg 5-(3-Cyan-4-fluor-phenyl)-[1,3,4]oxadiazol-2-carbonsäure-3-fluor-benzylamid und 50 mg Hydrazinhydrat werden in 1 ml Butanol bei 90°C 1 Stunde gerührt. Es wird zum Rückstand eingeengt und dieser durch zweimalige Säulenchromatographie zuerst an Kieselgel (Eluent: EE), danach an RP-18 (Eluent: Wasser, Acetonitril) gereinigt. Man erhält 3 mg 5-(3-Amino-1*H-*indazol-5-yl)-[1,3,4]oxadiazol-2-carbonsäure-3-fluor-benzylamid (3%); MS-FAB (M+H⁺) = 353,6; R_{f} (Methode polar): 1,66 Min.

### Beispiel 10

Die Herstellung von 2-[5-(3-Amino-1*H-*indazol-5-yl)-[1,3,4]oxadiazol-2-ylamino]-N-(3-methoxy-phenyl)-acetamid ("A76") erfolgt analog nachstehendem Schema **(Methode 10)**

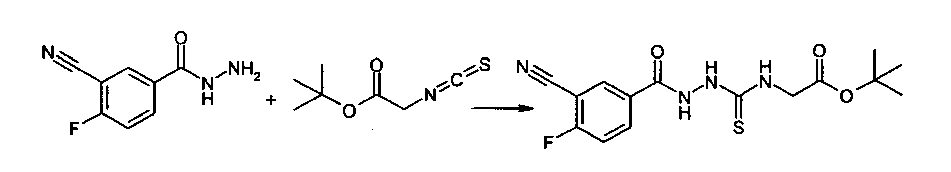

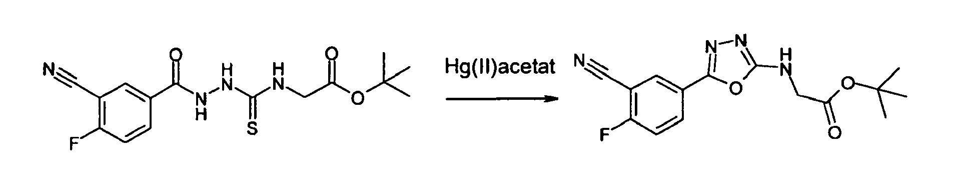

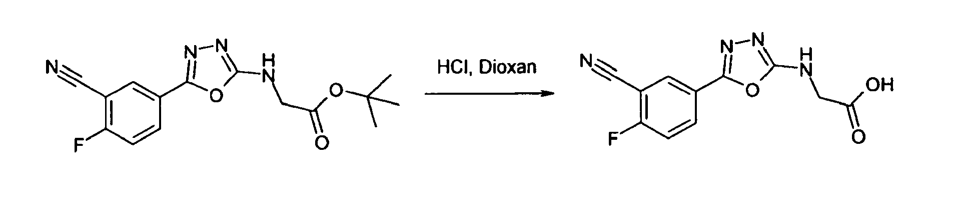

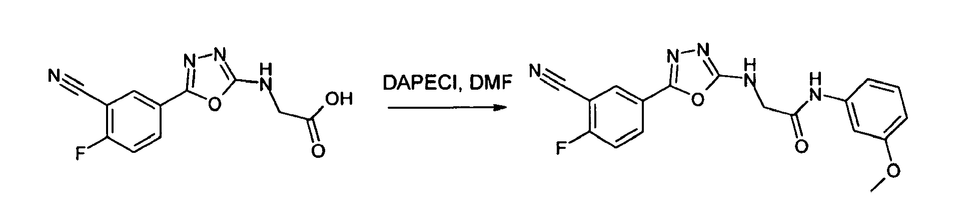

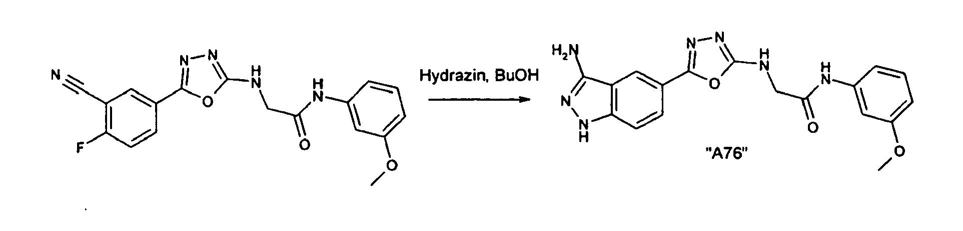

### Schritt 1:

In einem Kolben mit Rückflußkühler werden 620 mg 3-Cyan-4-fluor-benzoesäurehydrazid und 693 mg Isothiocyanato-essigsäure-tert.-butylester in 20 ml Dichlorethan 1 Stunde refluxiert. Nach dem Abkühlen entfernt man das Lösungsmittel, wobei 1,200 g Additionsprodukt (98%) erhalten werden, das ohne weitere Reinigung direkt in der nächsten Stufe eingesetzt wird; MS-FAB (M+H⁺) = 353,5; R_{f} (Methode polar): 1,89 Min.

### Schritt 2:

1,200 g Produkt aus Schritt 1 und 1,338 g Quecksilber(II)acetat werden in 10 ml Methanol bei 80°C 1 Stunde gerührt. Die Reaktionsmischung wird über Kieselgur abfiltriert, mit Methanol nachgewaschen und das Filtrat eingeengt. Man erhält 650 mg [5-(3-Cyan-4-fluor-phenyl)-[1,3,4]oxadiazol-2-ylamino]-essigsäure-tert.-butylester (58%), das ohne weitere Reinigung direkt in der nächsten Stufe eingesetzt wird; MS-FAB (M+H⁺) = 319,4; R_{f} (Methode polar): 1,96 Min.

### Schritt 3:

650 mg [5-(3-Cyan-4-fluor-phenyl)-[1,3,4]oxadiazol-2-ylamino]-essigsäure-tert.-butylester werden in 10 ml HCl/Dioxan (4N) bei 0°C 1 Stunde gerührt. Die Reaktionsmischung wird eingeengt, mit Diethylether/PE suspendiert, der entstehende Feststoff abgesaugt und getrocknet. Man erhält 330 mg [5-(3-Cyan-4-fluor-phenyl)-[1,3,4]oxadiazol-2-ylamino]-essigsäure (62%), das ohne weitere Reinigung direkt in der nächsten Stufe eingesetzt wird; MS-FAB (M+H⁺) = 263,5; R_{f} (Methode polar): 1,33 Min.

### Schritt 4:

100 mg [5-(3-Cyan-4-fluor-phenyl)-[1,3,4]oxadiazol-2-ylamino]-essigsäure, 49 mg m-Anisidin und 88 mg DAPECI werden in 1 ml DMF bei RT über Nacht gerührt. Anschließend wird auf 10 ml 1N HCl gegossen, der ausgefallene Feststoff abgesaugt und getrocknet. Man erhält 46 mg 2-[5-(3-Cyan-4-fluor-phenyl)-[1,3,4]oxadiazol-2-ylamino]-N-(3-methoxy-phenyl)-acetamid (33%), das ohne weitere Reinigung direkt in der nächsten Stufe eingesetzt wird; MS-FAB (M+H⁺) = 368,5; R_{f} (Methode polar): 1,83 Min.

### Schritt 5:

46 mg 2-[5-(3-Cyan-4-fluor-phenyl)-[1,3,4]oxadiazol-2-ylamino]-N-(3-methoxy-phenyl)-acetamid und 40 mg Hydrazinhydrat werden in 1 ml Butanol 2 Stunden bei 90°C gerührt. Die Reaktionsmischung wird eingeengt und der Rückstand durch Säulenchromatographie an RP-18 (Eluent: Wasser, Acetonitril) gereinigt. Man erhält 15 mg 2-[5-(1*H*-Indazol-5-yl)-[1,3,4]oxadiazol-2-ylamino]-N-(3-methoxy-phenyl)-acetamid (32%); MS-FAB (M+H⁺) = 380,5; R_{f} (Methode polar): 1,38 Min.

### Beispiel 11

Die Herstellung von 5-{5-[2-(3-Fluor-phenyl)-ethyl]-[1,3,4]thiadiazol-2-yl}-1H-indazol ("A77") erfolgt analog nachstehendem Schema **(Methode 11)**

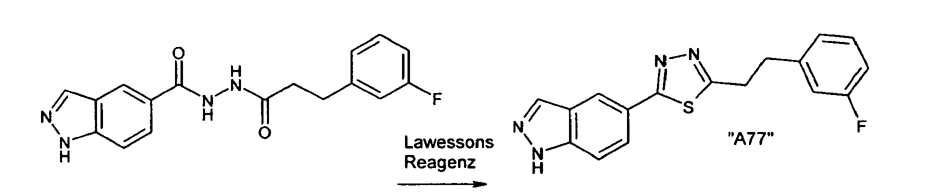

In einem Kolben mit Rückflußkühler werden 188 mg 1H-Indazol-5-carbonsäure-N'-[3-(3-fluor-phenyl)-propionyl]-hydrazid und 283 mg 2,4-Bis-(4-methoxy-phenyl)-[1,3,2,4]dithiadiphosphetan-2,4-disulfid 12 Stunden bei 130°C in Xylol (Isomerengemisch) gerührt. Es wird zum Rückstand eingeengt und dieser durch zweimalige Säulenchromatographie zuerst an Kieselgel (Eluent: EE, Methanol), danach an RP-18 (Eluent: Wasser, Acetonitril) gereinigt. Man erhält 7 mg 5-{5-[2-(3-Fluor-phenyl)-ethyl]-[1,3,4]thiadiazol-2-yl}-1*H-*indazol (4%); MS-FAB (M+H⁺) = 325,5; R_{f} (Methode polar): 2,08 Min.

### Beispiel 12

Die Herstellung von [1-(3-Chlor-4-fluor-phenyl)-ethyl]-[5-(3-iod-1*H*-indazol-5-yl)-[1,3,4]oxadiazol-2-yl]-amin ("A78") erfolgt analog nachstehendem Schema **(Methode 12)**

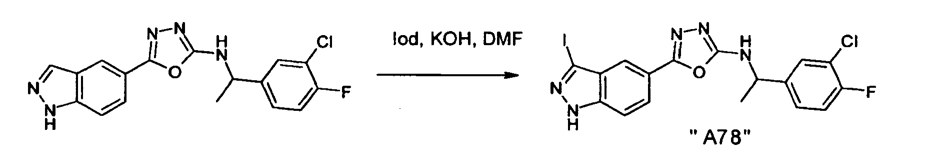

304 mg lod und 134 mg KOH werden in 1ml DMF gelöst. In diese Mischung werden 200 mg [1-(3-Chlor-4-fluor-phenyl)-ethyl]-[5-(1*H-*indazol-5-yl)-[1,3,4]oxadiazol-2-yl]-amin gegeben und 3 Stunden bei RT gerührt. Man gibt die Reaktionsmischung auf Wasser, entfärbt durch Zugabe von etwas Natriumthiosulfat, extrahiert mehrfach mit EE und trocknet die vereinigten organischen Phasen mit Natriumsulfat. Nach Entfernung des Lösungsmittels wird der Rückstand durch Säulenchromatographie an Kieselgel (Eluent: EE, Methanol) gereinigt. Man erhält 60 mg [1-(3-Chlor-4-fluor-phenyl)-ethyl]-[5-(3-iod-1*H*-indazol-5-yl)-[1,3,4]oxadiazol-2-yl]-amin (22%); MS-FAB (M+H⁺) = 484,5; R_{f} (Methode polar): 2,20 Min.

### Beispiel 13

Die Herstellung von 5-(3-Chlor-1*H*-indazol-5-yl)-[1,3,4]oxadiazol-2-yl]-ethylamin ("A79") erfolgt analog nachstehendem Schema **(Methode 13)** 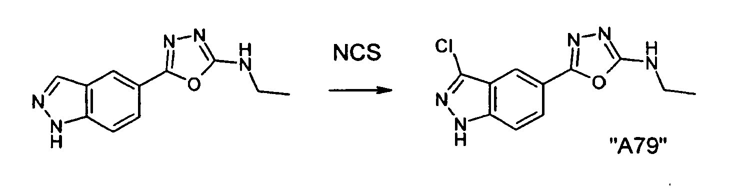 140 mg Ethyl-[5-(1*H-*indazol-5-yl)-[1,3,4]oxadiazol-2-yl]-amin in 5 ml Chloroform werden mit 170 mg NCS über Nacht bei 50°C gerührt. Nach dem Abkühlen wird der Feststoff abfiltriert und durch mehrfache Säulenchromatographie an Kieselgel und RP-18 gereinigt. Man erhält 5 mg (3,1%) "A79" als farbloses Pulver; MS-FAB (M+H⁺) = 264,69; ¹H-NMR (DMSO-d₆, TFA-Austausch): δ [ppm] 8,17 (1H, s), 7,97 (1H, d, J = 8,7 Hz), 7,80 (1H, d, J = 8,7 Hz), 3,49 (2H, q, J = 7,4 Hz), 1,32 (3H, t, J = 7,4 Hz).

Die nachfolgenden Beispiele betreffen pharmazeutische Zubereitungen:

### Beispiel A: Injektionsgläser

Eine Lösung von 100 g eines erfindungsgemäßen Wirkstoffes und 5 g Dinatriumhydrogenphosphat wird in 3 I zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines erfindungsgemäßen Wirkstoffes mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines erfindungsgemäßen Wirkstoffes, 9,38 g NaH₂PO₄ · 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 I auf und sterilisiert durch Bestrahlung. Diese Lösung kann in Form von Augentropfen verwendet werden.

### Beispiel D: Salbe

Man mischt 500 mg eines erfindungsgemäßen Wirkstoffes mit 99,5 g Vaseline unter aseptischen Bedingungen.

### Beispiel E: Tabletten

Ein Gemisch von 1 kg Wirkstoff, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff werden in üblicher Weise in Hartgelatinekapseln gefüllt, so daß jede Kapsel 20 mg des Wirkstoffs enthält.

### Beispiel H: Ampullen

Eine Lösung von 1 kg eines erfindungsgemäßen Wirkstoffes in 60 I zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. Verbindungen der Formel I 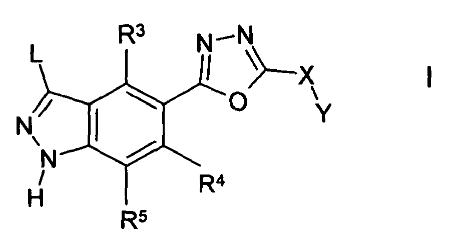 worin
L H, Hal oder NR⁷R⁸,
X CH₂, CH₂CH₂, CH(CH₃)CH₂, CH₂CH(CH₃), NH, O, NHCH₂, NHCH(CH₃), CH(CH₃)NH, CH₂NH, OCH₂, OCH₂CH₂, CH(CH₃)O, CH₂O, CH₂CH₂O, NHCH₂CH₂, NHCH(CH₃)CH₂, CH₂SO₂, NHCONHCH₂ oder NHCH₂CONH,
Y H, A, Ar oder Het,
R³ H, Hal oder A,
R⁴ R⁵ jeweils unabhängig voneinander H, Hal oder A,
R⁷, R⁸ jeweils unabhängig voneinander H oder CH₃,
A Alkyl mit 1-10 C-Atomen, worin 1-7 H-Atome durch F und/oder Cl ersetzt sein können,
Ar unsubstituiertes oder ein-, zwei-, drei- oder vierfach durch A, Hal, OH und/oder OA substituiertes Phenyl,
Het unsubstituiertes oder ein-, zwei- oder dreifach durch A, Hal, OH und/oder OA substituiertes 2- oder 3-Furyl, 2- oder 3-Thienyl, 1-, 2- oder 3-Pyrrolyl, 1-, 2, 4- oder 5- Imidazolyl, 1-, 3-, 4- oder 5-Pyrazolyl, 2-, 4- oder 5- Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, 1,2,3-Triazol-1-, -4- oder -5-yl, 1,2,4- Triazol-1-, -3- oder 5-yl, 1- oder 5-Tetrazolyl, 1,2,3- Oxadiazol-4- oder -5-yl, 1,2,4-Oxadiazol-3- oder -5-yl, 1,3,4-Thiadiazol-2- oder -5-yl, 1,2,4-Thiadiazol-3- oder -5- yl, 1,2,3-Thiadiazol-4- oder -5-yl, 3- oder 4-Pyridazinyl, Pyrazinyl, 1-, 2-, 3-, 4-, 5-, 6- oder 7-Indolyl, 4- oder 5- Isoindolyl, 1-, 2-, 4- oder 5-Benzimidazolyl, 1-, 2-, 3-, 4-, 5-, 6- oder 7-Indazolyl, Pyrrolidinyl, Piperidinyl oder Morpholinyl,
Hal F, Cl, Br oder I,
bedeuten,
sowie ihre pharmazeutisch verwendbaren Salze, Solvate und Stereoisomere, einschließlich deren Mischungen in allen Verhältnissen.

2. Verbindungen nach Anspruch 1 ausgewählt aus der Gruppe
| Nr. | Strukturformel und/oder Name |
|---|---|
| "A1" | 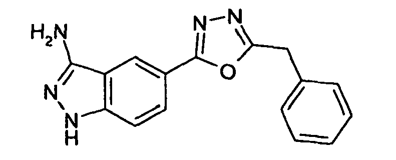 |
| "A2" | 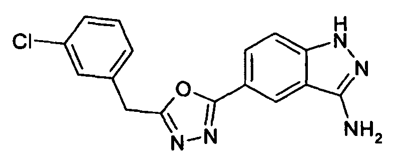 |
| "A3" | 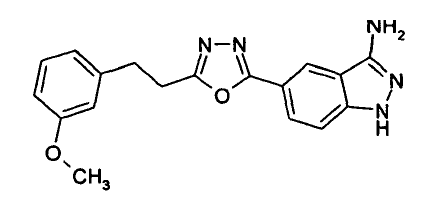 |
| "A4" | 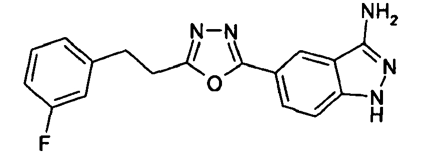 |
| "A5" | 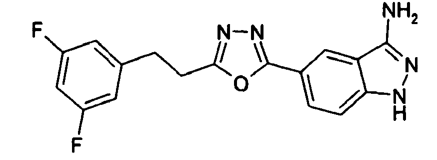 |
| "A6" | 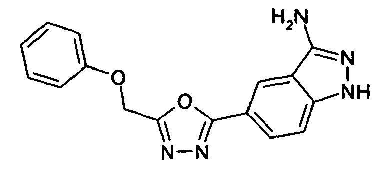 |
| "A7" | 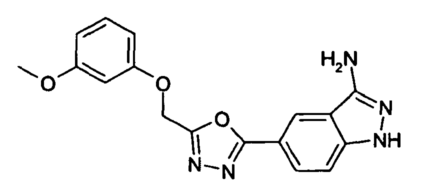 |
| "A8" | 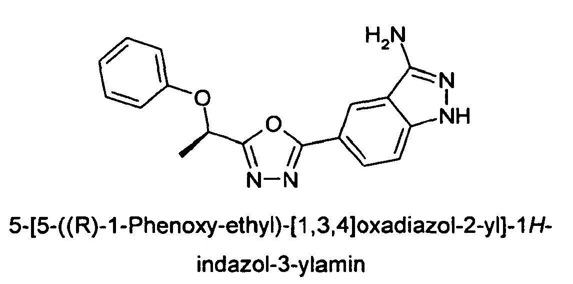 |
| "A9" | 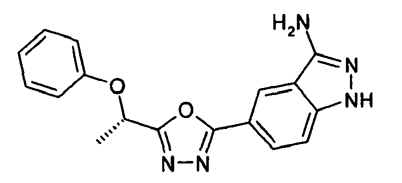 |
| "A10" | 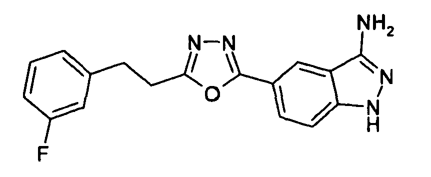 |
| "A11" | 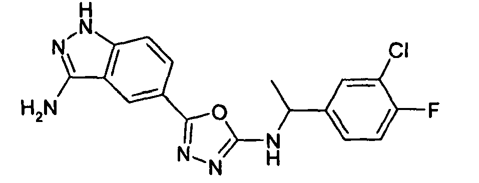 |
| "A12" | 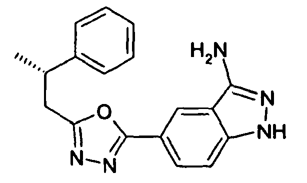 |
| "A13" | 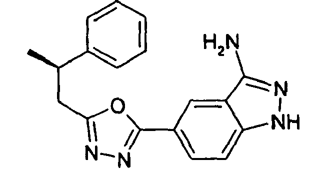 |
| "A14" | 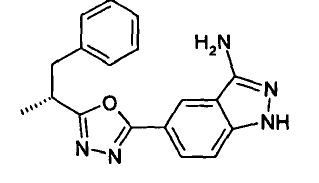 |
| "A15" | 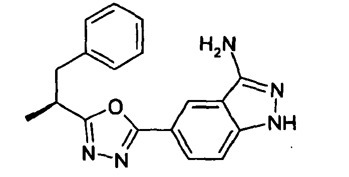 |
| "A16" | 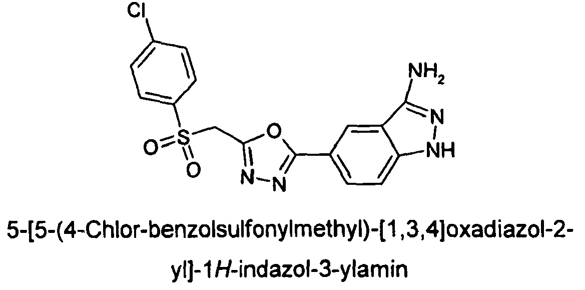 |
| "A17" | 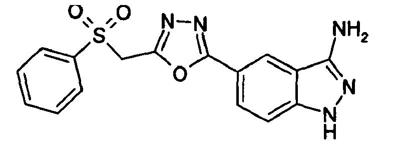 |
| "A18" | 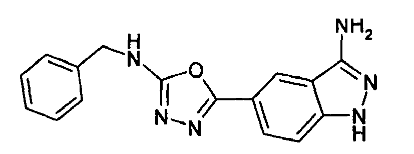 |
| "A19" | 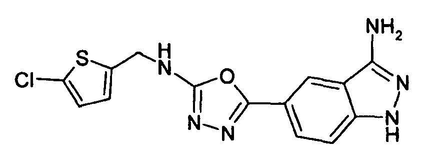 |
| "A20" | 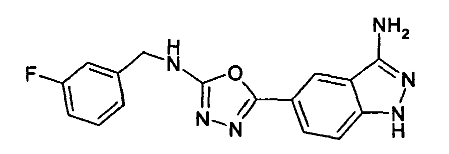 |
| "A21" | 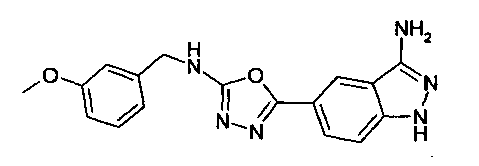 |
| "A22" | 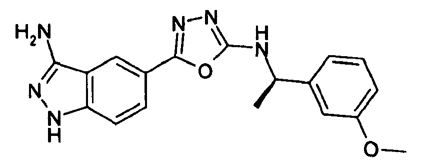 |
| "A23" | 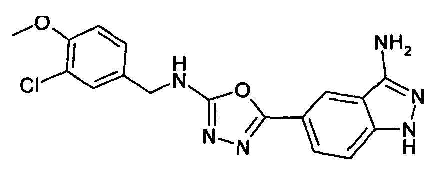 |
| "A24" | 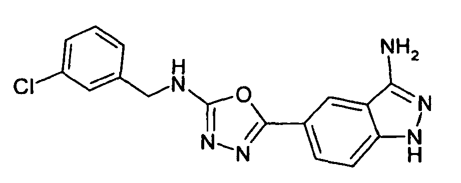 |
| "A25" | 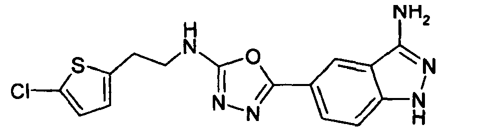 |
| "A26" | 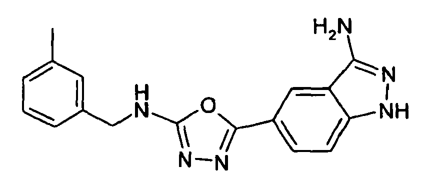 |
| "A27" | 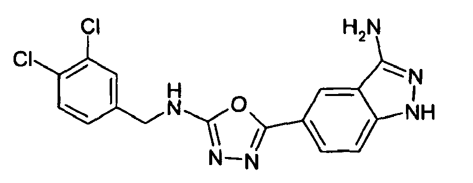 |
| "A28" | 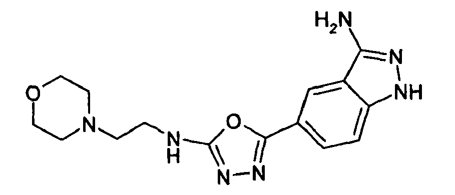 |
| "A29" | 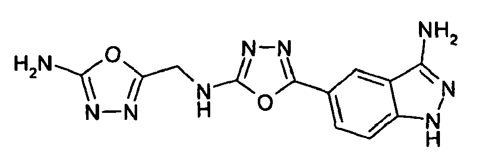 |
| "A30" | 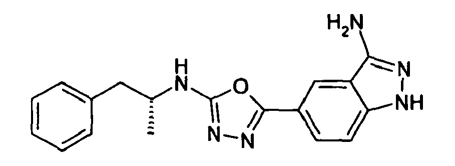 |
| "A31" | 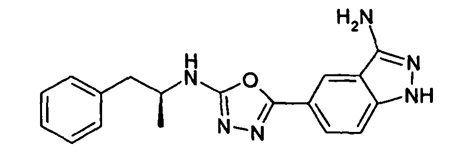 |
| "A32" | 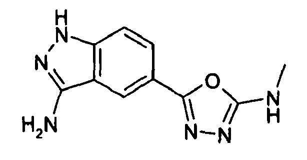 |
| "A33" | 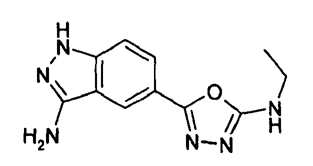 |
| "A34" | 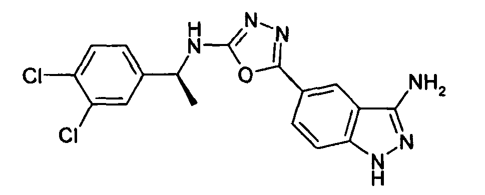 |
| "A35" | 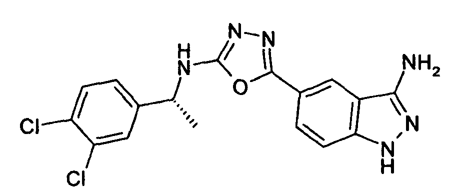 |
| "A36" | 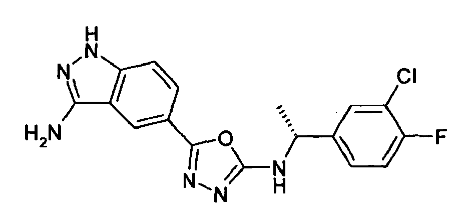 |
| "A37" | 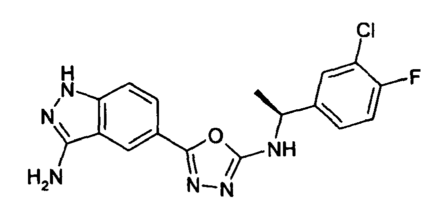 |
| "A38" | 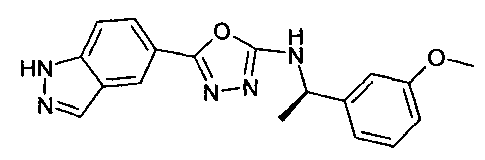 |
| "A39" | 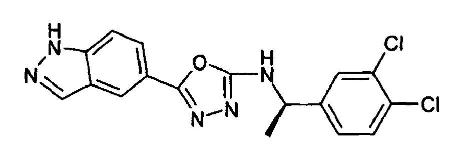 |
| "A40" | 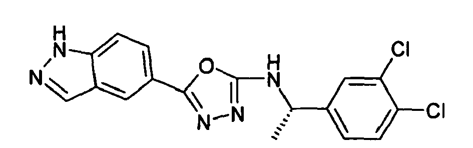 |
| "A41" | 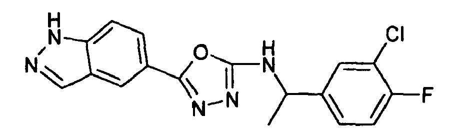 |
| "A42" | 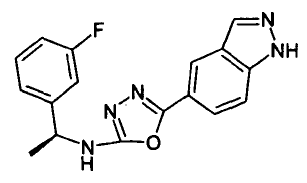 |
| "A43" | 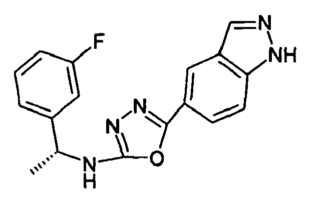 |
| "A44" | 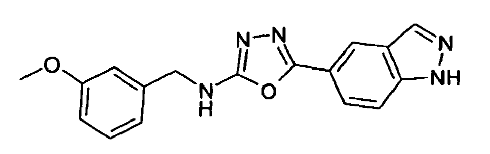 |
| "A45" | 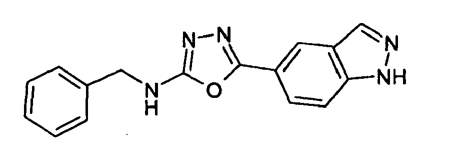 |
| "A46" | 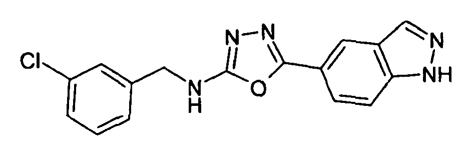 |
| "A47" | 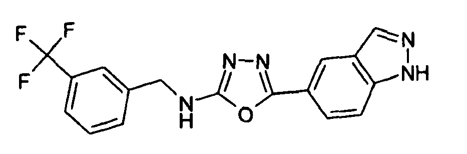 |
| "A48" | 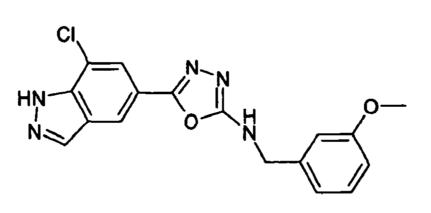 |
| "A49" | 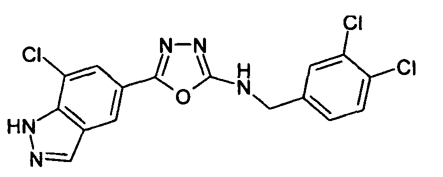 |
| "A50" | 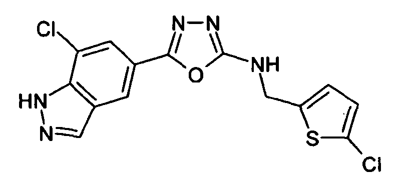 |
| "A51" | 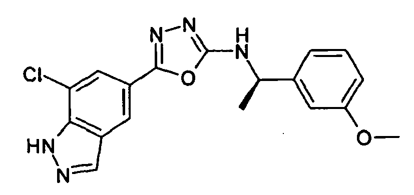 |
| "A52" | 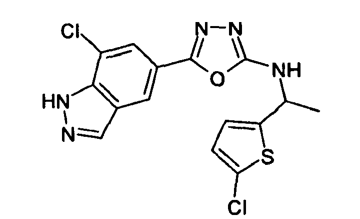 |
| "A53" | 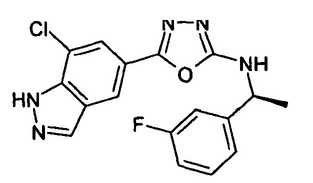 |
| "A54" | 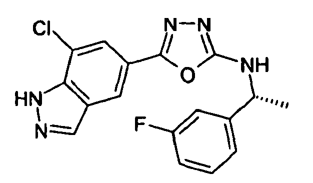 |
| "A55" | 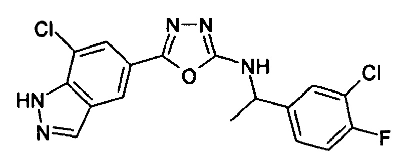 |
| "A56" | 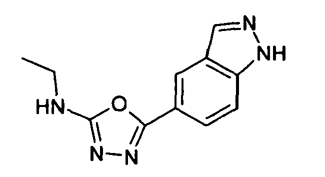 |
| "A57" | 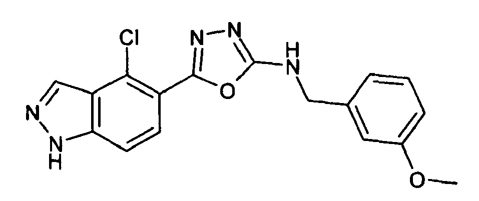 |
| "A58" | 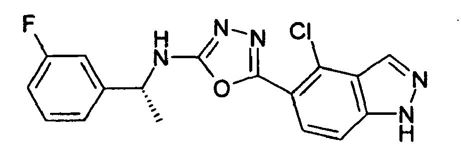 |
| "A59" | 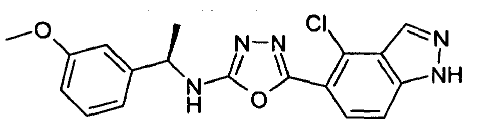 |
| "A60" | 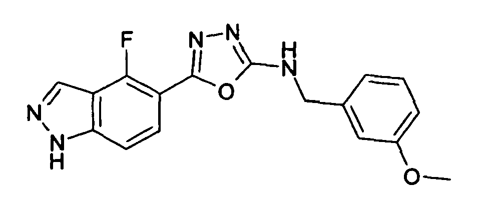 |
| "A61" | 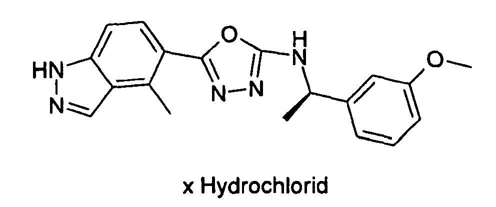 |
| "A62" | 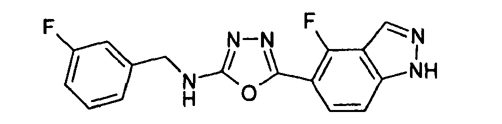 |
| "A63" | 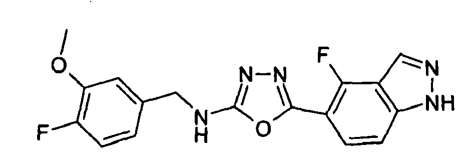 |
| "A64" | 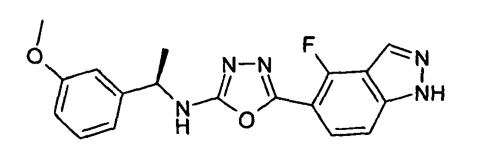 |
| "A65" | 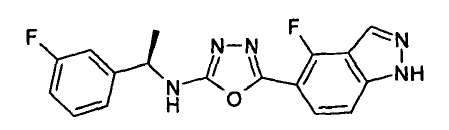 |
| "A66" | 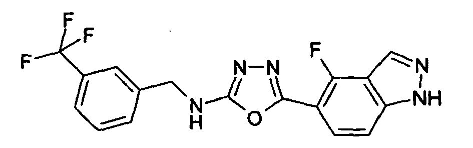 |
| "A67" | 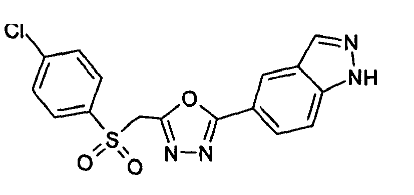 |
| "A68" | 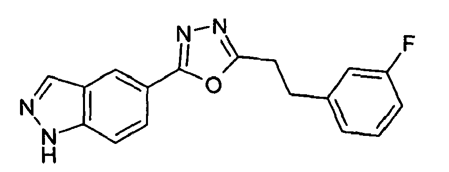 |
| "A69" | 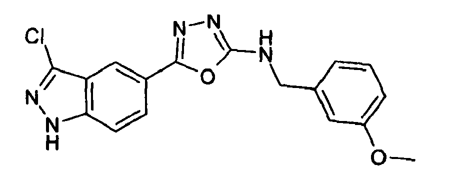 |
| "A70" | 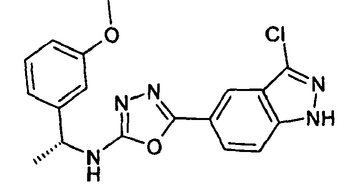 |
| "A71" | 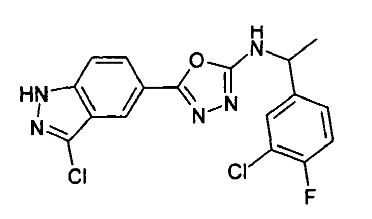 |
| "A73" | 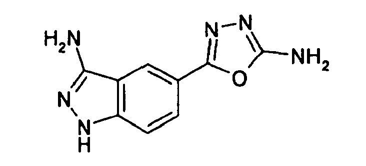 |
| "A74" | 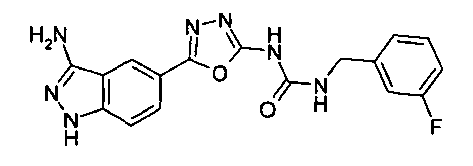 |
| "A75" | 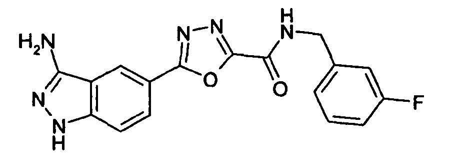 |
| "A76" | 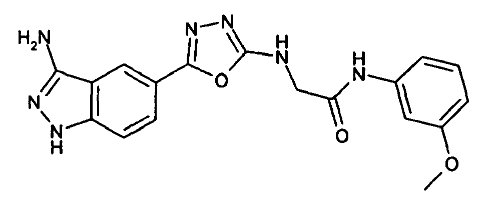 |
| "A78" | 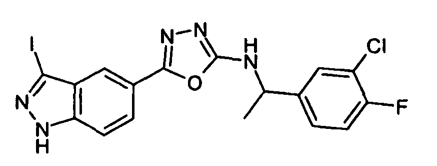 |
| "A79" | 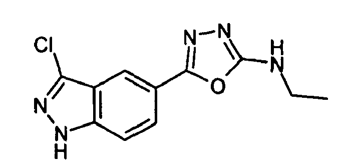 |
sowie ihre pharmazeutisch verwendbaren Salze, Solvate und Stereoisomere, einschließlich deren Mischungen in allen Verhältnissen.

3. Verfahren zur Herstellung von Verbindungen der Formel I nach den Ansprüchen 1-2 sowie ihrer pharmazeutisch verwendbaren, Solvate, Salze und Stereoisomere, **dadurch gekennzeichnet, daß** man
a) sie aus einem ihrer funktionellen Derivate durch Behandeln mit einem solvolysierenden oder hydrogenolysierenden Mittel in Freiheit setzt, indem man
eine konventionelle Aminoschutzgruppe durch Behandeln mit einem solvolysierenden oder hydrogenolysierenden Mittel durch Wasserstoff ersetzt oder
eine durch eine konventionelle Schutzgruppe geschützte Aminogruppe in Freiheit setzt,
oder
b) zur Herstellung von Verbindungen der Formel I, worin
L NH₂ bedeutet,
eine Verbindung der Formel II 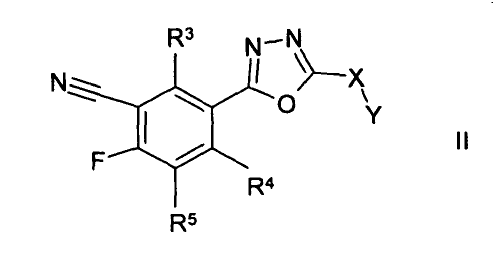 worin
R³, R⁴, R⁵, X und Y die in Anspruch 1 angegebenen Bedeutungen haben,
mit Hydrazin umsetzt,
oder
c) eine Verbindung der Formel III 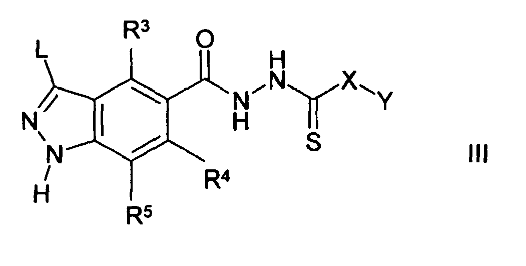 worin
L, R³, R⁴, R⁵, X und Y die in Anspruch 1 angegebenen Bedeutungen haben,
in Gegenwart eines Hg(II) - salzes cyclisiert,
oder
d) eine Verbindung der Formel IV 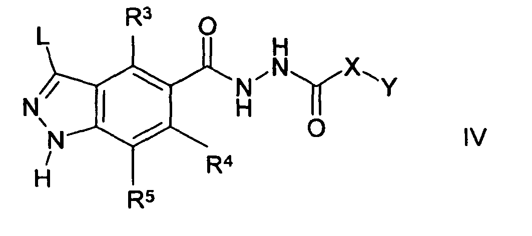
worin
L, R³, R⁴, R⁵, X und Y die in Anspruch 1 angegebenen Bedeutungen haben,
in Gegenwart von POCl₃ cyclisiert,
und/oder eine Base oder Säure der Formel I in eines ihrer Salze umwandelt.

4. Arzneimittel, enthaltend mindestens eine Verbindung nach Anspruch 1-2 und/oder ihre pharmazeutisch verwendbaren Salze, Solvate und Stereoisomere, einschließlich deren Mischungen in allen Verhältnissen, sowie gegebenenfalls Träger- und/oder Hilfsstoffe.

5. Verwendung von Verbindungen gemäß Anspruch 1-2, sowie ihrer pharmazeutisch verwendbaren Salze, Solvate und Stereoisomere, einschließlich deren Mischungen in allen Verhältnissen, zur Herstellung eines Arzneimittels zur Behandlung und/oder Prophylaxe von Krankheiten, bei denen die Hemmung, Regulierung und/oder Modulation der Signaltransduktion von Kinasen eine Rolle spielt.

6. Verwendung nach Anspruch 5, wobei es sich bei der Kinase um SGK handelt.

7. Verwendung nach Anspruch 6 von Verbindungen gemäß Anspruch 1-2, sowie ihrer pharmazeutisch verwendbaren Salze, Solvate und Stereoisomere, einschließlich deren Mischungen in allen Verhältnissen, zur Herstellung eines Arzneimittels zur Behandlung von Krankheiten, die durch Inhibierung der SGK durch die Verbindungen nach Anspruch 1-2 beeinflußt werden.

8. Verwendung nach Anspruch 7 von Verbindungen gemäß Anspruch 1-2, sowie ihrer pharmazeutisch verwendbaren Salze, Solvate und Stereoisomere, einschließlich deren Mischungen in allen Verhältnissen, zur Herstellung eines Arzneimittels zur Behandlung oder Vorbeugung von Diabetes, Fettsucht, metabolischem Syndrom (Dyslipidämie), systemischer und pulmonaler Hypertonie, Herzkreislauferkrankungen und Nierenerkrankungen, allgemein bei jeglicher Art von Fibrosen und entzündlichen Prozessen, Krebs, Tumorzellen, Tumormetastasen, Koagulopathien, neuronaler Erregbarkeit, Glaukom, Katarakt, bakteriellen Infektionen sowie in einer antiinfektiösen Therapie, zur Steigerung der Lernfähigkeit und Aufmerksamkeit, sowie zur Behandlung und Prophylaxe von Zellalterung und Stress und zur Behandlung von Tinitus.

9. Verwendung nach Anspruch 8, wobei es sich bei Diabetes um Diabetes mellitus, diabetische Nephropathie, diabetische Neuropathie, diabetische Angiopathie und Mikroangiopathie handelt.

10. Verwendung nach Anspruch 8, wobei es sich bei Herzkreislauferkrankungen um kardiale Fibrosen nach Myokardinfarkt, Herzhypertrophie, Herzinsuffizienz und Arteriosklerose handelt.

11. Verwendung nach Anspruch 8, wobei es sich bei Nierenerkrankungen um Glomerulosklerose, Nephrosklerose, Nephritis, Nephropathie und Störung der Elektrolytausscheidung handelt.

12. Verwendung nach Anspruch 9, wobei es sich bei Fibrosen und entzündlichen Prozessen um Leberzirrhose, Lungenfibrose, fibrosierende Pankreatitis, Rheumatismus und Arthrosen, Morbus Crohn, chronische Bronchitis, Strahlenfibrose, Sklerodermitis, zystische Fibrose, Narbenbildung und Morbus Alzheimer handelt.

13. Arzneimittel enthaltend mindestens eine Verbindung gemäß Anspruch 1-2 und/oder ihre pharmazeutisch verwendbaren Salze, Solvate und Stereoisomere, einschließlich deren Mischungen in allen Verhältnissen, und mindestens einen weiteren Arzneimittelwirkstoff.

14. Set (Kit), bestehend aus getrennten Packungen von
(a) einer wirksamen Menge an einer Verbindung gemäß Anspruch 1-2 und/oder ihrer pharmazeutisch verwendbaren Salze, Solvate und Stereoisomere, einschließlich deren Mischungen in allen Verhältnissen,
und
(b) einer wirksamen Menge eines weiteren Arzneimittelswirkstoffs.

## Claims

1. Compounds of the formula I 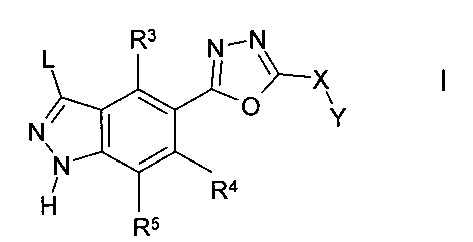 in which
L denotes H, Hal or NR⁷R⁸,
X denotes CH₂, CH₂CH₂, CH(CH₃)CH₂, CH₂CH(CH₃), NH, O, NHCH₂, NHCH(CH₃), CH(CH₃)NH, CH₂NH, OCH₂, OCH₂CH₂, CH(CH₃)O, CH₂O, CH₂CH₂O, NHCH₂CH₂, NHCH(CH₃)CH₂, CH₂SO₂, NHCONHCH₂ or NHCH₂CONH,
Y denotes H, A, Ar or Het,
R³ denotes H, Hal or A,
R⁴, R⁵ each, independently of one another, denote H, Hal or A,
R⁷, R⁸ each, independently of one another, denote H or CH₃,
A denotes alkyl having 1-10 C atoms, in which 1-7 H atoms may be replaced by F and/or Cl,
Ar denotes phenyl which is unsubstituted or mono-, di-, tri- or tetrasubstituted by A, Hal, OH and/or OA,
Het denotes 2- or 3-furyl, 2- or 3-thienyl, 1-, 2- or 3-pyrrolyl, 1-, 2-, 4- or 5-imidazolyl, 1-, 3-, 4- or 5-pyrazolyl, 2-, 4- or 5- oxazolyl, 3-, 4- or 5-isoxazolyl, 2-, 4- or 5-thiazolyl, 3-, 4- or 5-isothiazolyl, 2-, 3- or 4-pyridyl, 2-, 4-, 5- or 6-pyrimidinyl, 1,2,3-triazol-1-, -4- or -5-yl, 1,2,4-triazol-1-, -3- or 5-yl, 1- or 5-tetrazolyl, 1,2,3-oxadiazol-4- or -5-yl, 1,2,4-oxadiazol-3- or -5-yl, 1,3,4-thiadiazol-2- or -5-yl, 1,2,4-thiadiazol-3- or -5- yl, 1,2,3-thiadiazol-4- or -5-yl, 3- or 4-pyridazinyl, pyrazinyl, 1-, 2-, 3-, 4-, 5-, 6- or 7-indolyl, 4- or 5-isoindolyl, 1-, 2-, 4- or 5-benzimidazolyl, 1-, 2-, 3-, 4-, 5-, 6- or 7-indazolyl, pyrrolidinyl, piperidinyl or morpholinyl, each of which is un- substituted or mono-, di- or trisubstituted by A, Hal, OH and/or OA,
Hal denotes F, Cl, Br or I,
and pharmaceutically usable salts, solvates and stereoisomers thereof, including mixtures thereof in all ratios.

2. Compounds according to Claim 1 selected from the group
| No. | Structural formula and/or name |
|---|---|
| "A1" | 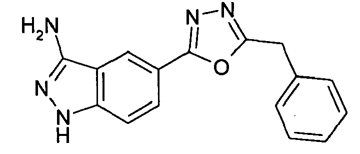 |
| "A2" | 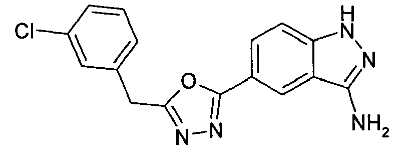 |
| "A3" | 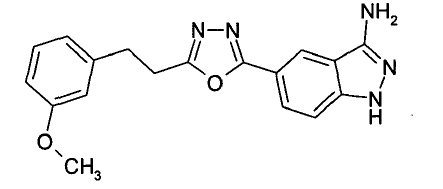 |
| "A4" | 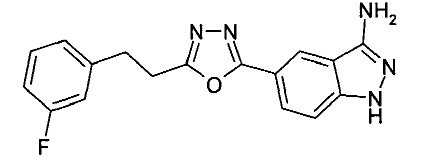 |
| "A5" | 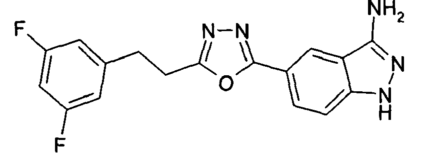 |
| "A6" | 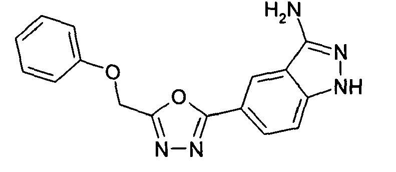 |
| "A7" | 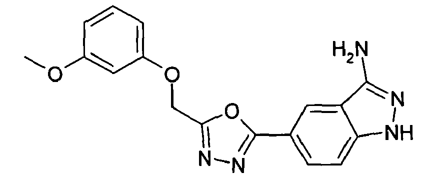 |
| "A8" | 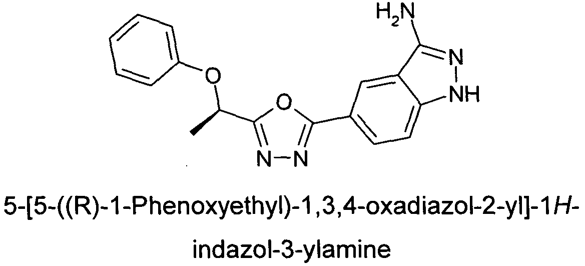 |
| "A9" | 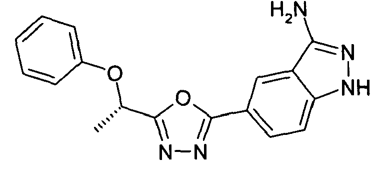 |
| "A10" | 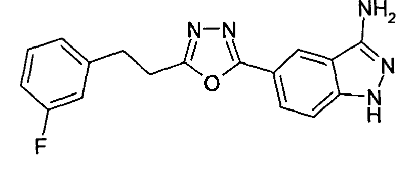 |
| "A11" | 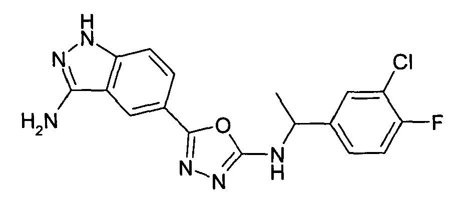 |
| "A12" | 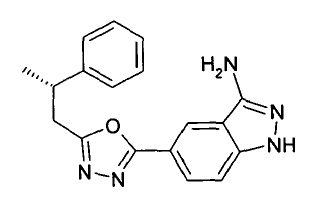 |
| "A13" | 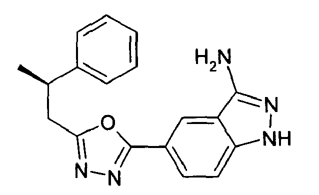 |
| "A14" | 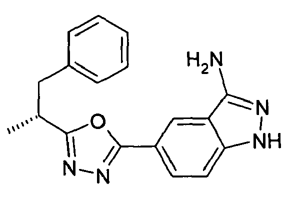 |
| "A15" | 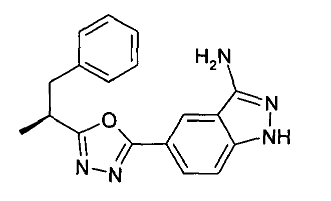 |
| "A16" | 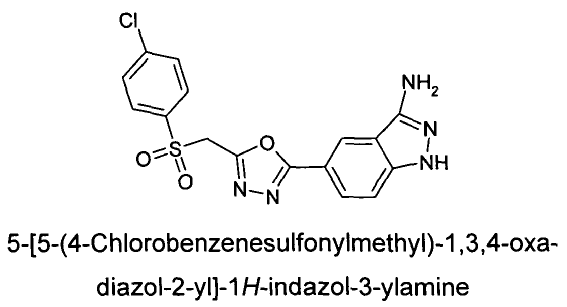 |
| "A17" | 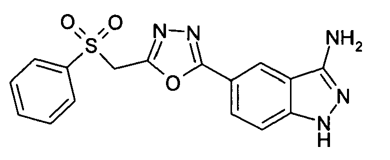 |
| "A18" | 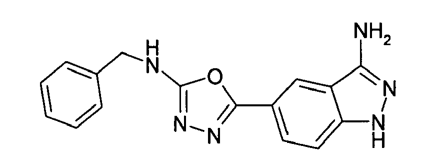 |
| "A19" | 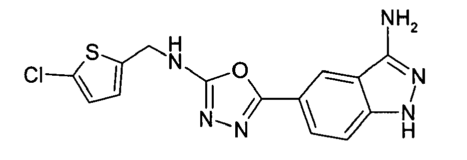 |
| "A20" | 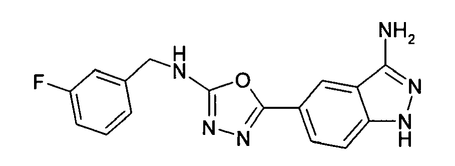 |
| "A21" | 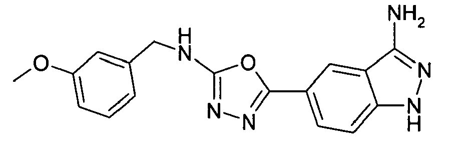 |
| "A22" | 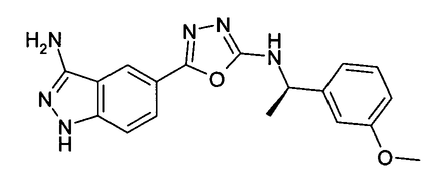 |
| "A23" | 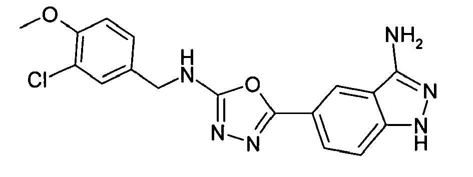 |
| "A24" | 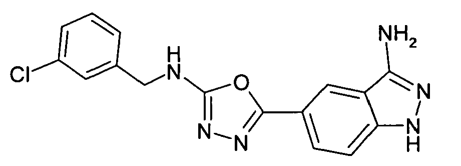 |
| "A25" | 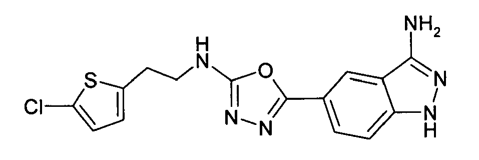 |
| "A26" | 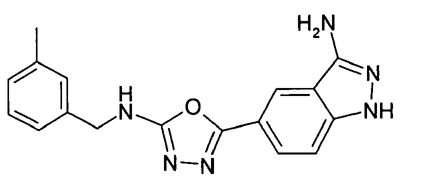 |
| "A27" | 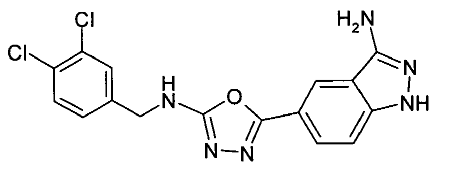 |
| "A28" | 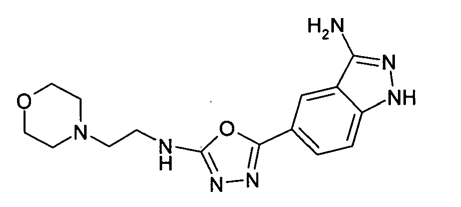 |
| "A29" | 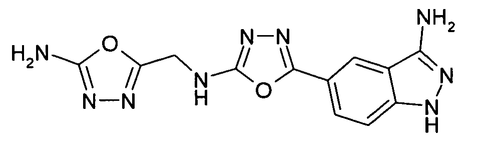 |
| "A30" | 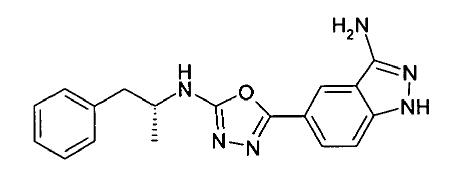 |
| "A31" | 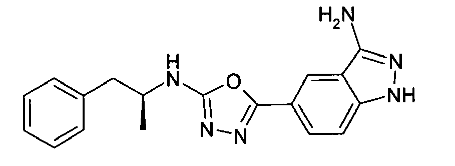 |
| "A32" | 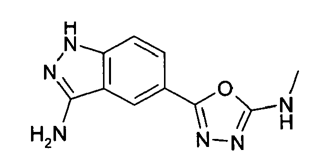 |
| "A33" | 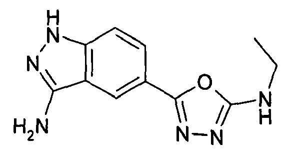 |
| "A34" | 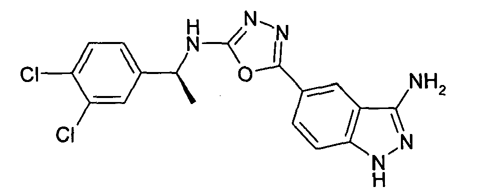 |
| "A35" | 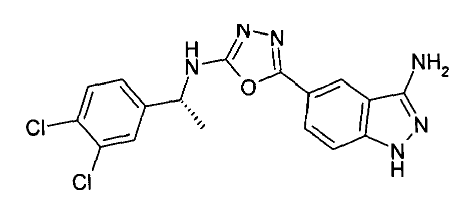 |
| "A36" | 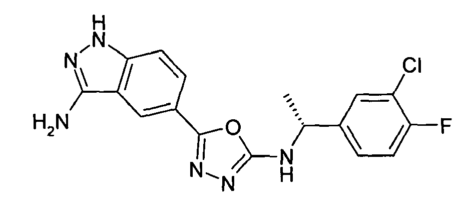 |
| "A37" | 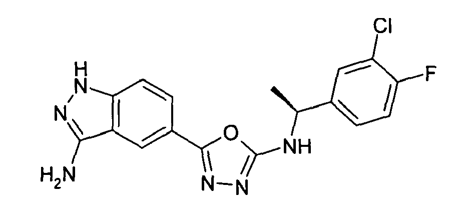 |
| "A38" | 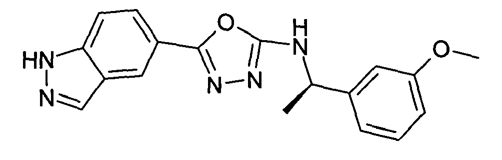 |
| "A39" | 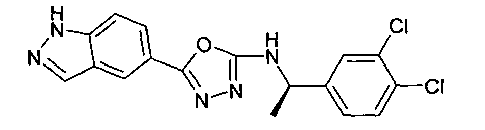 |
| "A40" | 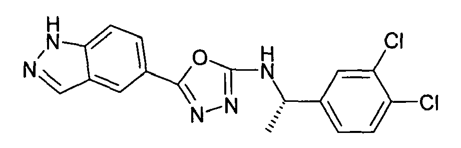 |
| "A41" | 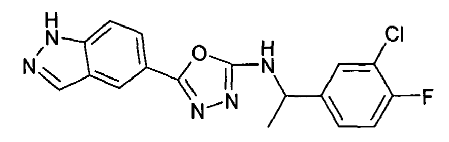 |
| "A42" | 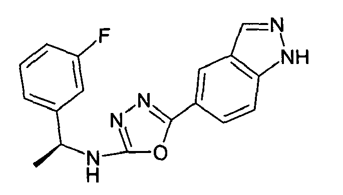 |
| "A43" | 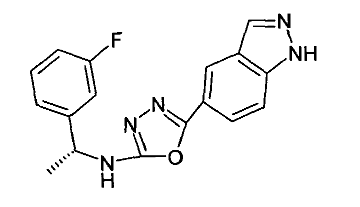 |
| "A44" | 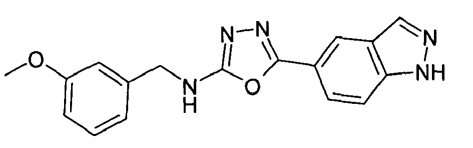 |
| "A45" | 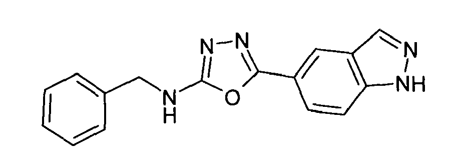 |
| "A46" | 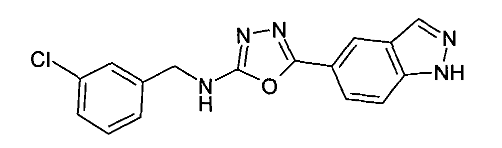 |
| "A47" | 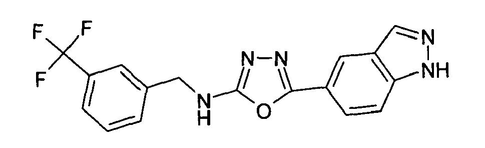 |
| "A48" | 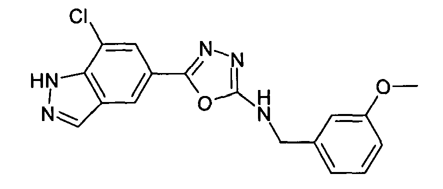 |
| "A49" | 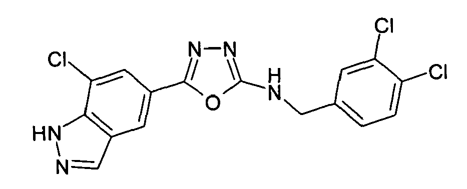 |
| "A50" | 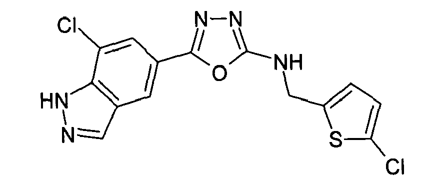 |
| "A51" | 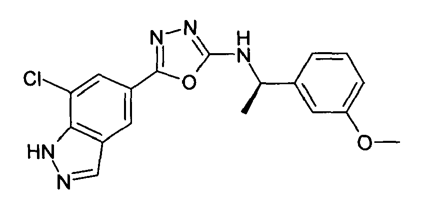 |
| "A52" | 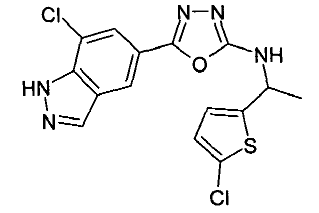 |
| "A53" | 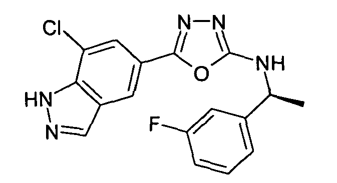 |
| "A54" | 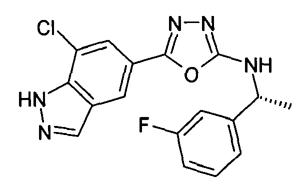 |
| "A55" | 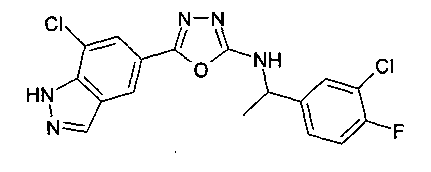 |
| "A56" | 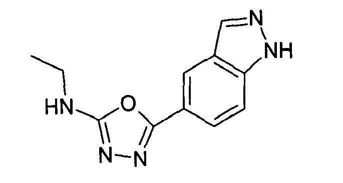 |
| "A57" | 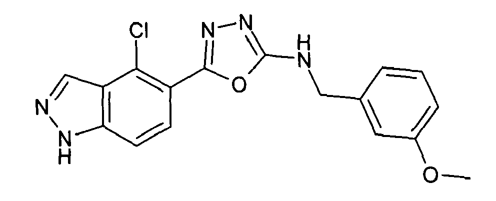 |
| "A58" | 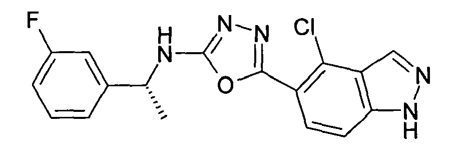 |
| "A59" | 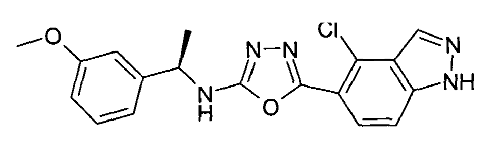 |
| "A60" | 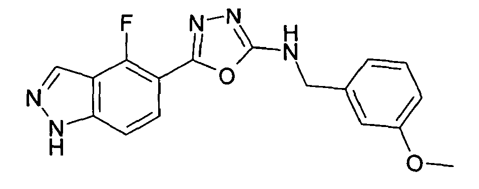 |
| "A61" | 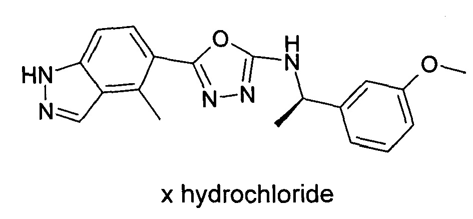 |
| "A62" | 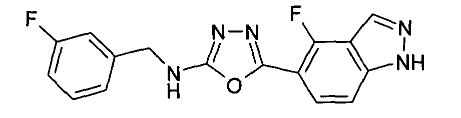 |
| "A63" | 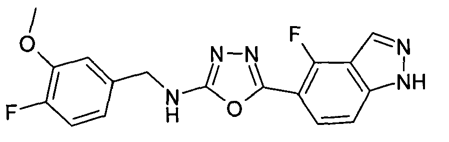 |
| "A64" | 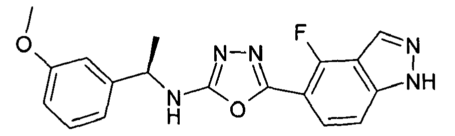 |
| "A65" | 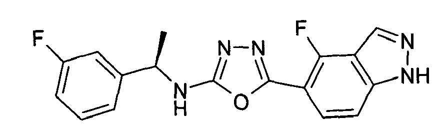 |
| "A66" | 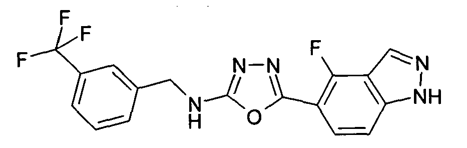 |
| "A67" | 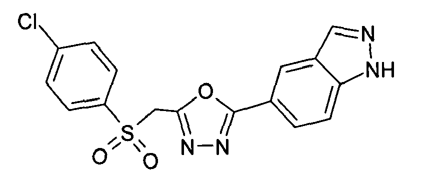 |
| "A68" | 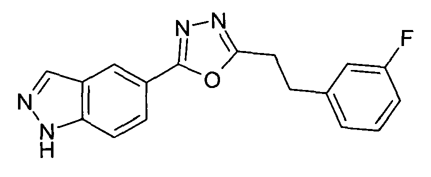 |
| "A69" | 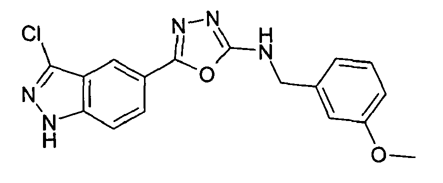 |
| "A70" | 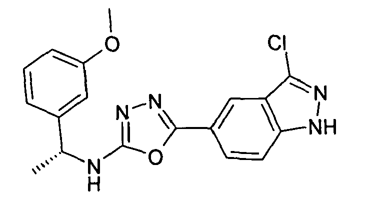 |
| "A71" | 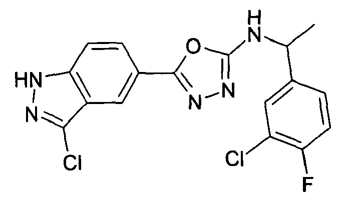 |
| "A73" | 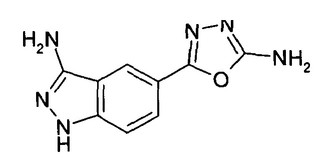 |
| "A74" | 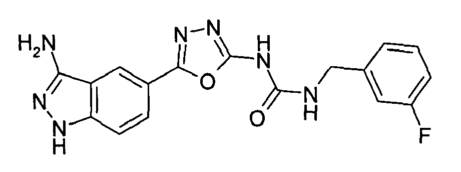 |
| "A75" | 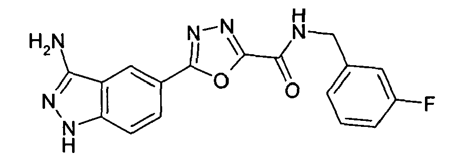 |
| "A76" | 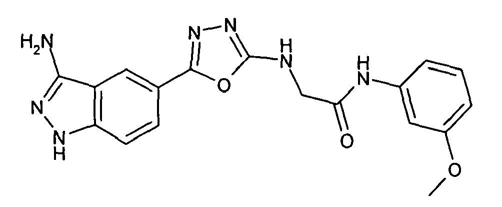 |
| "A78" | 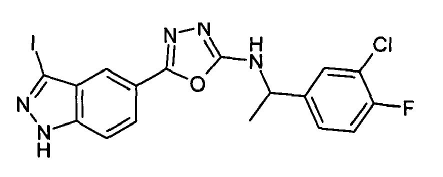 |
| "A79" | 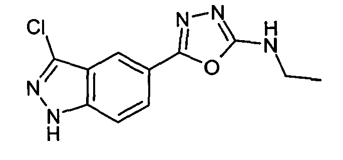 |
and pharmaceutically usable salts, solvates and stereoisomers thereof, including mixtures thereof in all ratios.

3. Process for the preparation of compounds of the formula I according to Claims 1-2 and pharmaceutically usable solvates, salts and stereoisomers thereof, **characterised in that**
a) they are liberated from one of their functional derivatives by treatment with a solvolysing or hydrogenolysing agent by replacing a conventional amino-protecting group with hydrogen by treatment with a solvolysing or hydrogenolysing agent or liberating an amino group protected by a conventional protecting group,
or
b) for the preparation of compounds of the formula I in which
L denotes NH₂,
a compound of the formula II 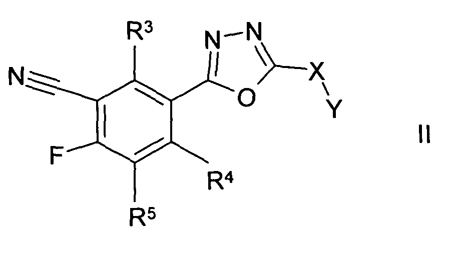 in which
R³, R⁴, R⁵, X and Y have the meanings indicated in Claim 1,
is reacted with hydrazine,
or
c) a compound of the formula III 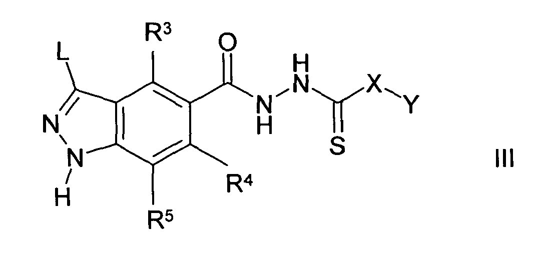 in which
L, R³, R⁴, R⁵, X and Y have the meanings indicated in Claim 1,
is cyclised in the presence of an Hg(II) salt,
or
d) a compound of the formula IV 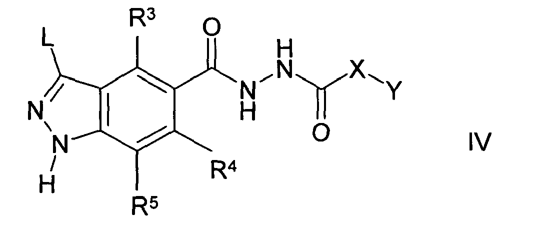
in which
L, R³, R⁴, R⁵, X and Y have the meanings indicated in Claim 1,
is cyclised in the presence of POCl₃,
and/or a base or acid of the formula I is converted into one of its salts.

4. Medicaments comprising at least one compound according to Claims 1-2 and/or pharmaceutically usable salts, solvates and stereoisomers thereof, including mixtures thereof in all ratios, and optionally excipients and/or adjuvants.

5. Use of compounds according to Claims 1-2, and pharmaceutically usable salts, solvates and stereoisomers thereof, including mixtures thereof in all ratios, for the preparation of a medicament for the treatment and/or prophylaxis of diseases in which the inhibition, regulation and/or modulation of kinase signal transduction plays a role.

6. Use according to Claim 5, where the kinase is SGK.

7. Use according to Claim 6 of compounds according to Claims 1-2, and pharmaceutically usable salts, solvates and stereoisomers thereof, including mixtures thereof in all ratios, for the preparation of a medicament for the treatment of diseases which are influenced by inhibition of SGK by the compounds according to Claims 1-2.

8. Use according to Claim 7 of compounds according to Claims 1-2, and pharmaceutically usable salts, solvates and stereoisomers thereof, including mixtures thereof in all ratios, for the preparation of a medicament for the treatment or prevention of diabetes, obesity, metabolic syndrome (dyslipidaemia), systemic and pulmonary hypertonia, cardiovascular diseases and kidney diseases, generally in fibroses and inflammatory processes of any type, cancer, tumour cells, tumour metastases, coagulopathies, neuronal excitability, glaucoma, cataract, bacterial infections and in antiinfection therapy, for increasing learning ability and attention, and for the treatment and prophylaxis of cell ageing and stress and for the treatment of tinnitus.

9. Use according to Claim 8, where diabetes is diabetes mellitus, diabetic nephropathy, diabetic neuropathy, diabetic angiopathy and microangiopathy.

10. Use according to Claim 8, where cardiovascular diseases are cardiac fibroses after myocardial infarction, cardiac hypertrophy, cardiac insufficiency and arteriosclerosis.

11. Use according to Claim 8, where kidney diseases are glomerulosclerosis, nephrosclerosis, nephritis, nephropathy and electrolyte excretion disorder.

12. Use according to Claim 9, where fibroses and inflammatory processes are liver cirrhosis, pulmonary fibrosis, fibrosing pancreatitis, rheumatism and arthroses, Crohn's disease, chronic bronchitis, radiation fibrosis, sclerodermatitis, cystic fibrosis, scarring and Alzheimer's disease.

13. Medicaments comprising at least one compound according to Claims 1-2 and/or pharmaceutically usable salts, solvates and stereoisomers thereof, including mixtures thereof in all ratios, and at least one further medicament active compound.

14. Set (kit) consisting of separate packs of
(a) an effective amount of a compound according to Claims 1-2 and/or pharmaceutically usable salts, solvates and stereoisomers thereof, including mixtures thereof in all ratios,
and
(b) an effective amount of a further medicament active compound.

## Revendications

1. Composés de formule I 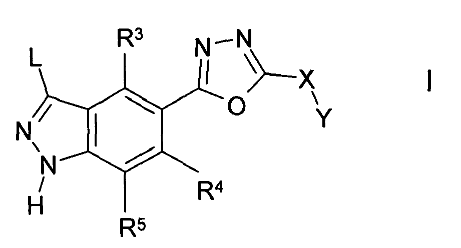 dans laquelle
L désigne H, Hal ou NR⁷R⁸,
X désigne CH₂, CH₂CH₂, CH(CH₃)CH₂, CH₂CH(CH₃), NH, O, NHCH₂, NHCH(CH₃), CH(CH₃)NH, CH₂NH, OCH₂, OCH₂CH₂, CH(CH₃)O, CH₂O, CH₂CH₂O, NHCH₂CH₂, NHCH(CH₃)CH₂, CH₂SO₂, NHCONHCH₂ ou NHCH₂CONH,
Y désigne H, A, Ar ou Hét,
R³ désigne H, Hal ou A,
R⁴, R⁵ désignent chacun, indépendamment l'un de l'autre, H, Hal ou A,
R⁷, R⁸ désignent chacun, indépendamment l'un de l'autre, H ou CH₃,
A désigne alkyle ayant 1-10 atomes de C, où 1-7 atomes de H peuvent être remplacés par F et/ou Cl,
Ar désigne phényle qui est non substitué ou mono-, di-, tri- ou tétrasubstitué par A, Hal, OH et/ou OA,
Hét désigne 2- ou 3-furyle, 2- ou 3-thiényle, 1-, 2- ou 3-pyrro- lyle, 1-, 2-, 4- ou 5-imidazolyle, 1-, 3-, 4- ou 5-pyrazolyle, 2-, 4- ou 5-oxazolyle, 3-, 4- ou 5-isoxazolyle, 2-, 4- ou 5- thiazolyle, 3-, 4- ou 5-isothiazolyle, 2-, 3- ou 4-pyridyle, 2-, 4-, 5- ou 6-pyrimidinyle, 1,2,3-triazol-1-, -4- ou -5-yle, 1,2,4- triazol-1-, -3- ou 5-yle, 1- ou 5-tétrazolyle, 1,2,3-oxadiazol- 4- ou -5-yle, 1,2,4-oxadiazol-3- ou -5-yle, 1,3,4-thiadiazol- 2- ou -5-yle, 1,2,4-thiadiazol-3- ou -5-yle, 1,2,3-thiadiazol- 4- ou -5-yle, 3- ou 4-pyridazinyle, pyrazinyle, 1-, 2-, 3-, 4-, 5-, 6- ou 7-indolyle, 4- ou 5-isoindolyle, 1-, 2-, 4- ou 5- benzimidazolyle, 1-, 2-, 3-, 4-, 5-, 6- ou 7-indazolyle, pyr- rolidinyle, pipéridinyle ou morpholinyle, chacun d'entre eux étant non substitué ou mono-, di- ou trisubstitué par A, Hal, OH et/ou OA,
Hal désigne F, Cl, Br ou I,
et les sels, solvats et stéréoisomères pharmaceutiquement utilisables de ceux-ci, y compris des mélanges de ceux-ci en toutes proportions.

2. Composés selon la revendication 1, choisis dans le groupe constitué par
| n° | Formule structurelle et/ou nom |
|---|---|
| "A1" | 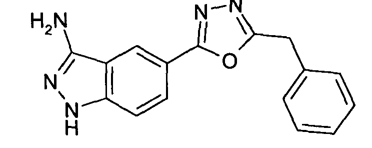 |
| "A2" | 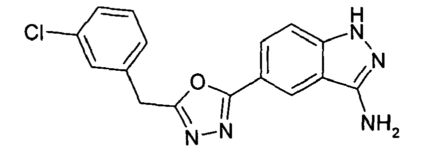 |
| "A3" | 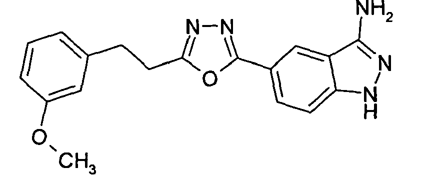 |
| "A4" | 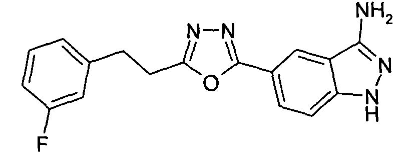 |
| "A5" | 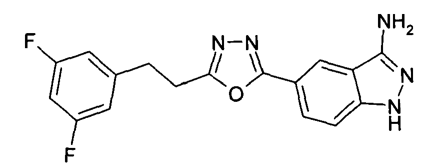 |
| "A6" | 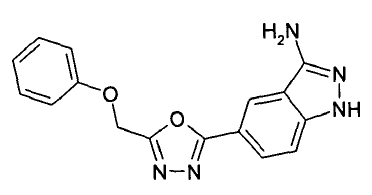 |
| "A7" | 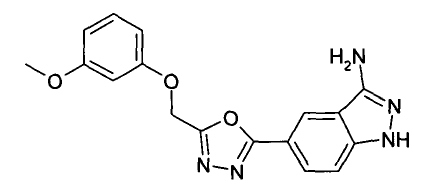 |
| "A8" | 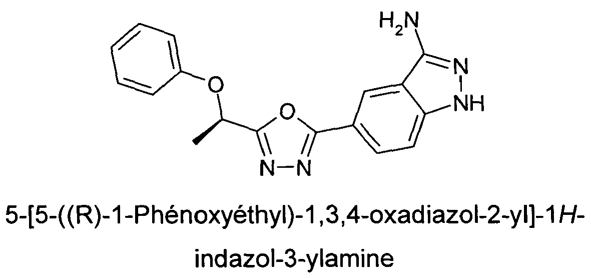 |
| "A9" | 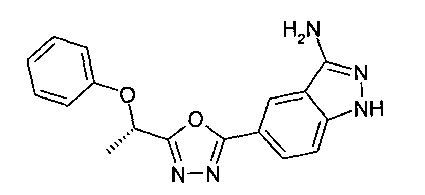 |
| "A10" | 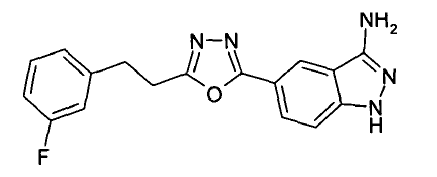 |
| "A11" | 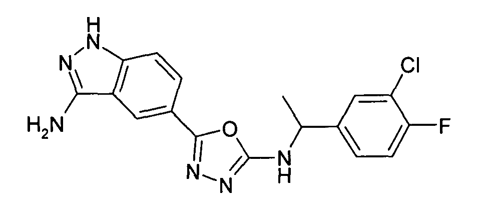 |
| "A12" | 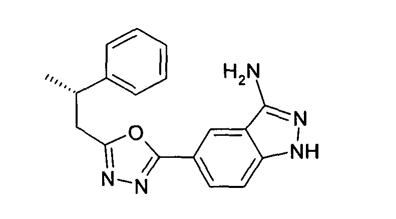 |
| "A13" | 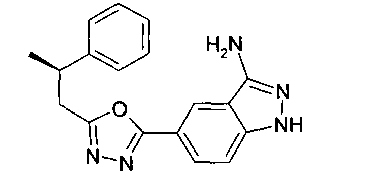 |
| "A14" | 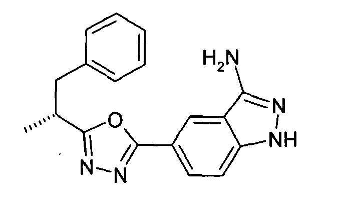 |
| "A15" | 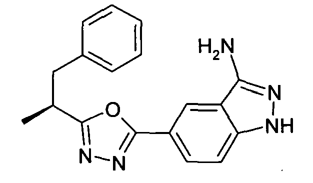 |
| "A16" | 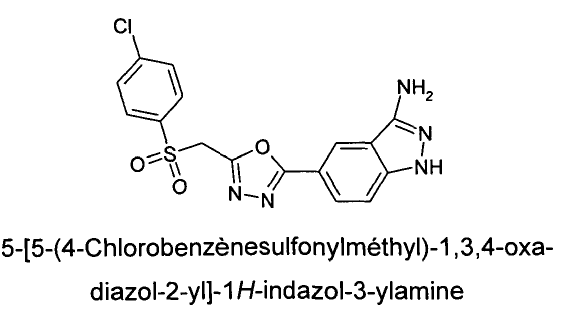 |
| "A17" | 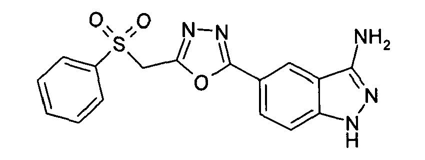 |
| "A18" | 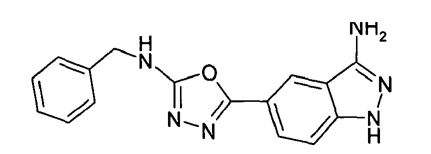 |
| "A19" | 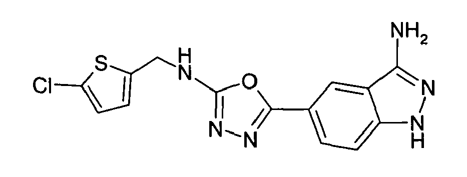 |
| "A20" | 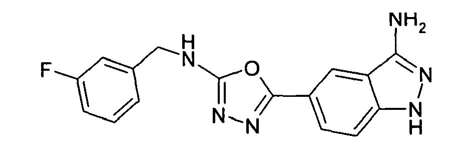 |
| "A21" | 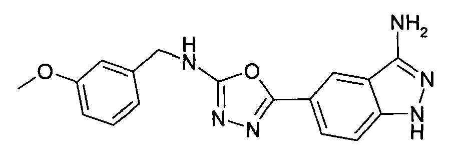 |
| "A22" | 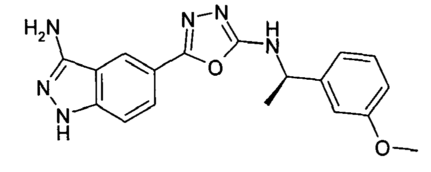 |
| "A23" | 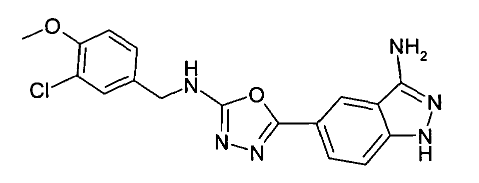 |
| "A24" | 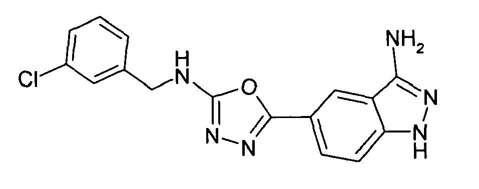 |
| "A25" | 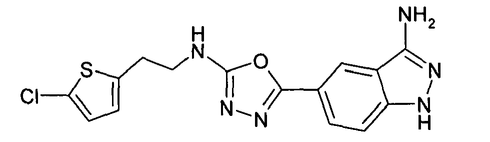 |
| "A26" | 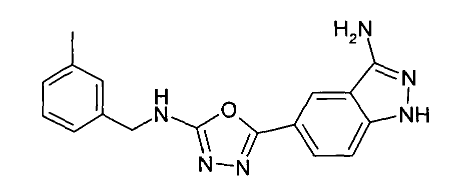 |
| "A27" | 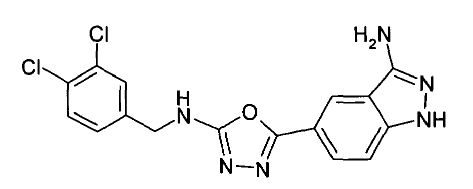 |
| "A28" | 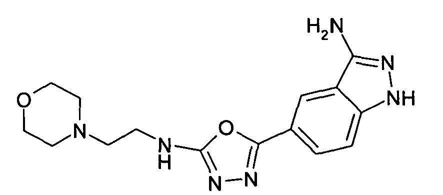 |
| "A29" | 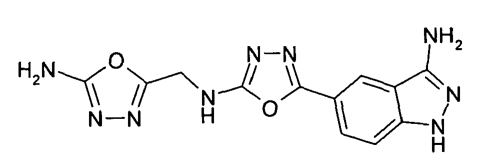 |
| "A30" | 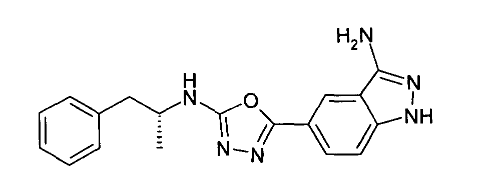 |
| "A31" | 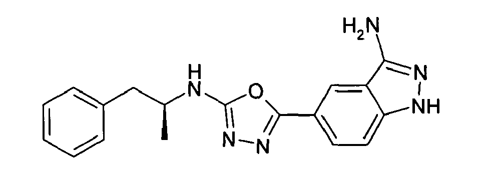 |
| "A32" | 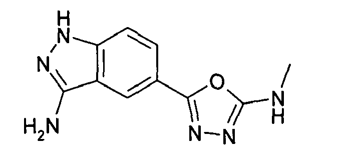 |
| "A33" | 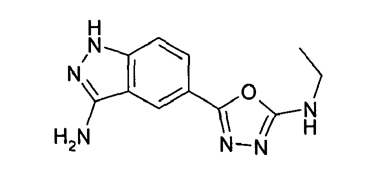 |
| "A34" | 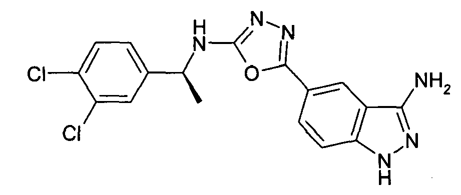 |
| "A35" | 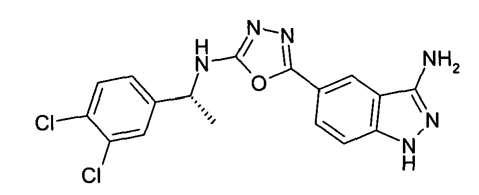 |
| "A36" | 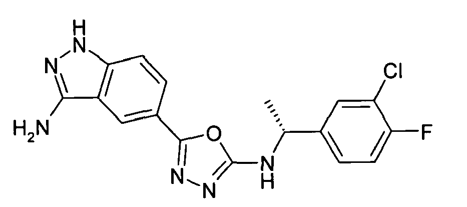 |
| "A37" | 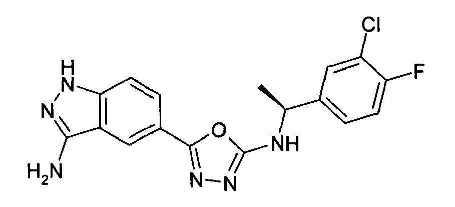 |
| "A38" | 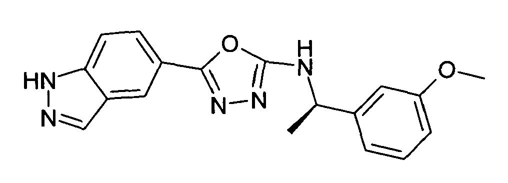 |
| "A39" | 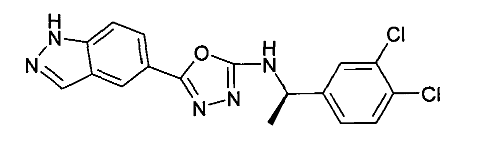 |
| "A40" | 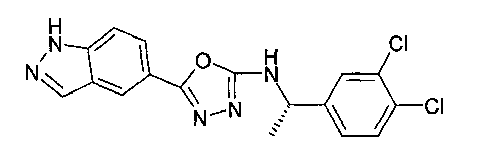 |
| "A41" | 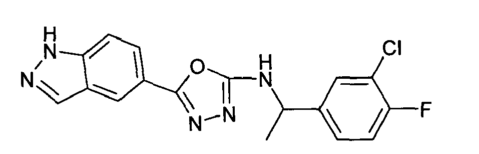 |
| "A42" | 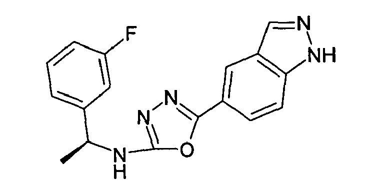 |
| "A43" | 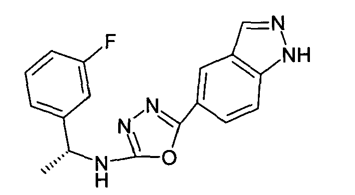 |
| "A44" | 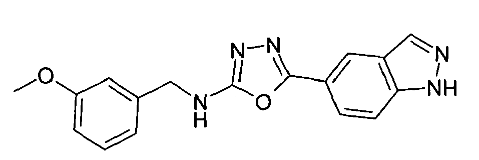 |
| "A45" | 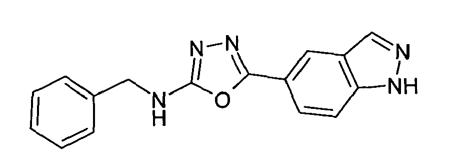 |
| "A46" | 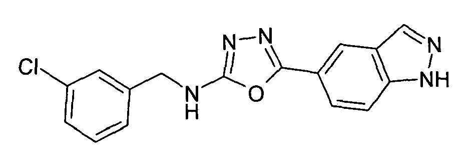 |
| "A47" | 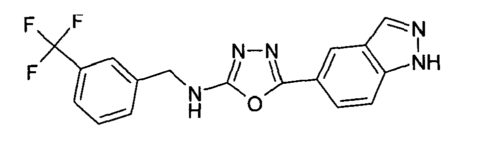 |
| "A48" | 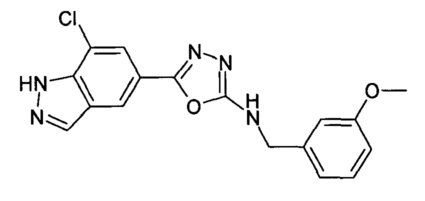 |
| "A49" | 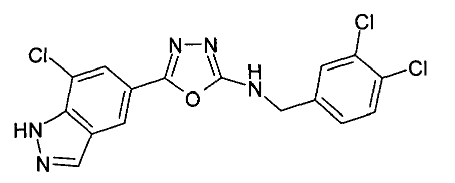 |
| "A50" | 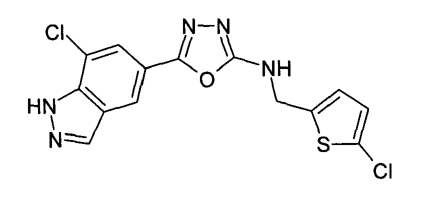 |
| "A51" | 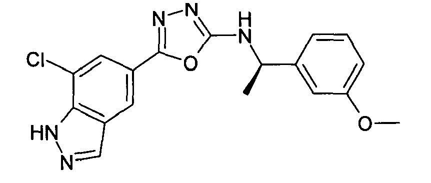 |
| "A52" | 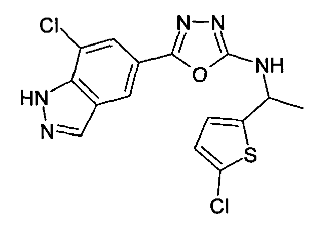 |
| "A53" | 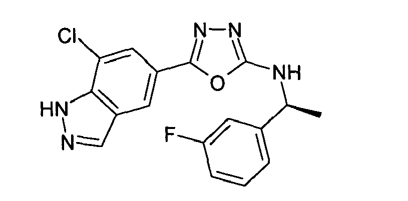 |
| "A54" | 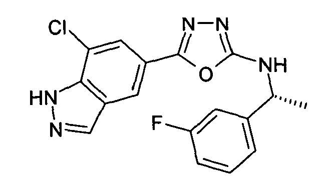 |
| "A55" | 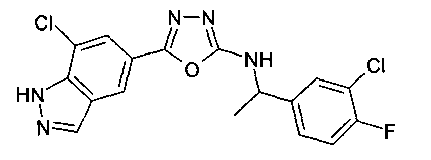 |
| "A56" | 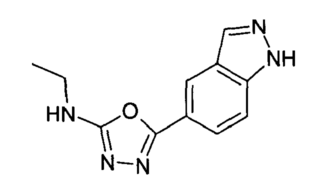 |
| "A57" | 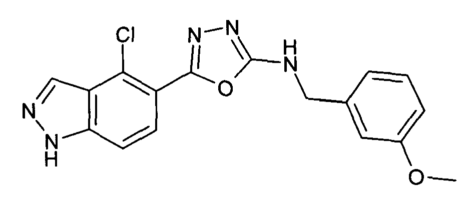 |
| "A58" | 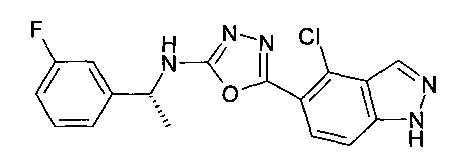 |
| "A59" | 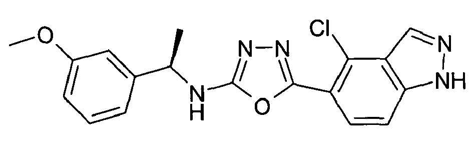 |
| "A60" | 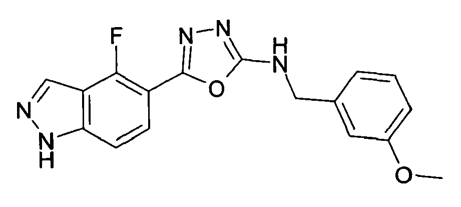 |
| "A61" | 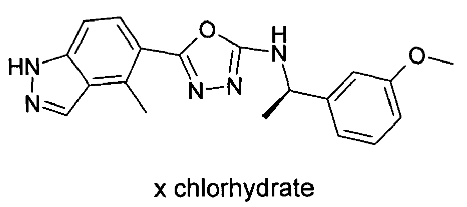 |
| "A62" | 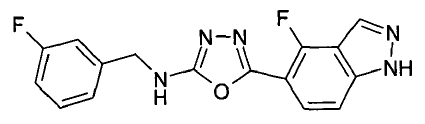 |
| "A63" | 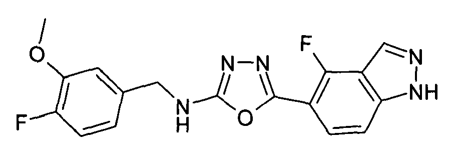 |
| "A64" | 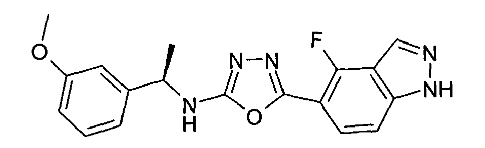 |
| "A65" | 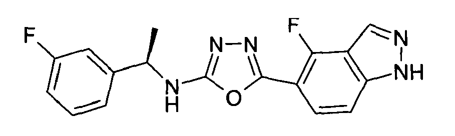 |
| "A66" | 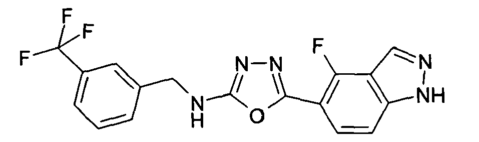 |
| "A67" | 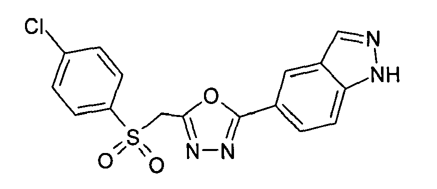 |
| "A68" | 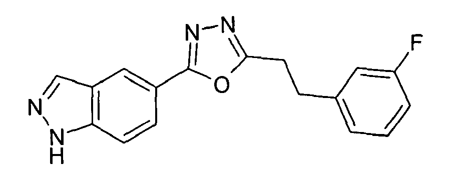 |
| "A69" | 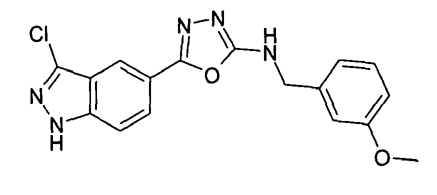 |
| "A70" | 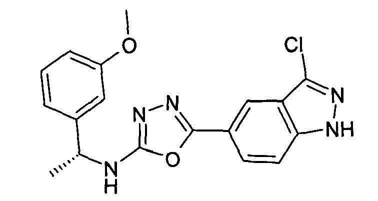 |
| "A71" | 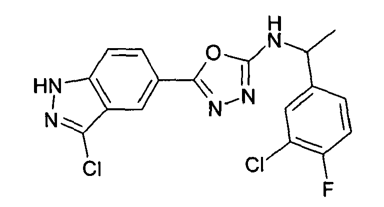 |
| "A73" | 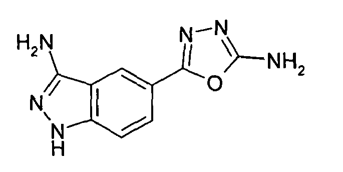 |
| "A74" | 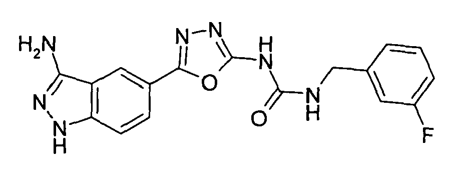 |
| "A75" | 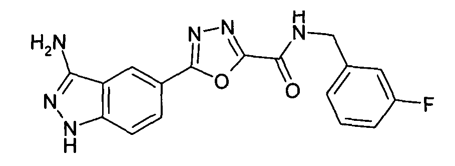 |
| "A76" | 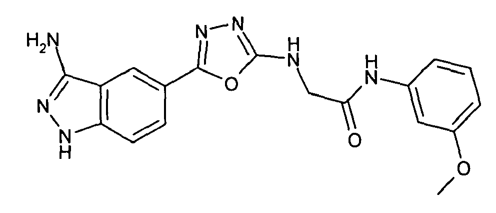 |
| "A78" | 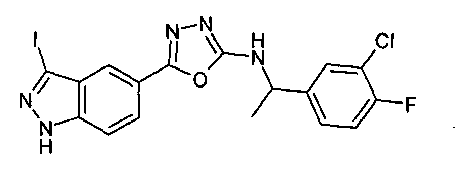 |
| "A79" | 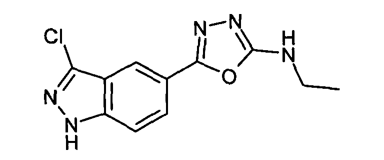 |
et les sels, solvats et stéréoisomères pharmaceutiquement utilisables de ceux-ci, y compris des mélanges de ceux-ci en toutes proportions.

3. Procédé de préparation de composés de formule I selon les revendications 1-2 et de solvats, sels et stéréoisomères pharmaceutiquement utilisables de ceux-ci, **caractérisé en ce que**
a) ils sont libérés à partir de l'un de leurs dérivés fonctionnels par un traitement par un agent de solvolyse ou d'hydrogénolyse en remplaçant un groupement protecteur d'amino classique par de l'hydrogène par un traitement par un agent de solvolyse ou d'hydrogénolyse ou
en libérant un groupement amino protégé par un groupement protecteur classique,
ou
b) pour la préparation de composés de formule I dans laquelle
L désigne NH₂,
un composé de formule II 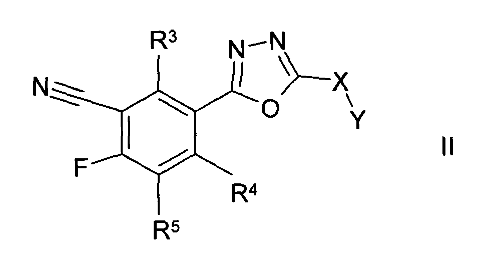 dans laquelle
R³, R⁴, R⁵, X et Y revêtent les significations indiquées selon la revendication 1,
est réagi avec de l'hydrazine,
ou
c) un composé de formule III 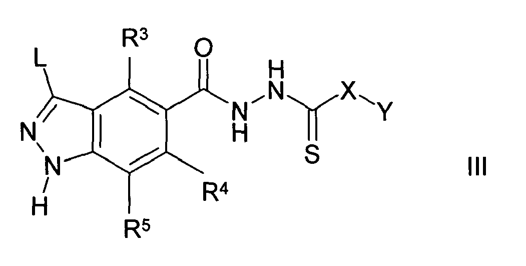 dans laquelle
L, R³, R⁴, R⁵, X et Y revêtent les significations indiquées selon la revendication 1,
est cyclisé en présence d'un sel de Hg(II),
ou
d) un composé de formule IV 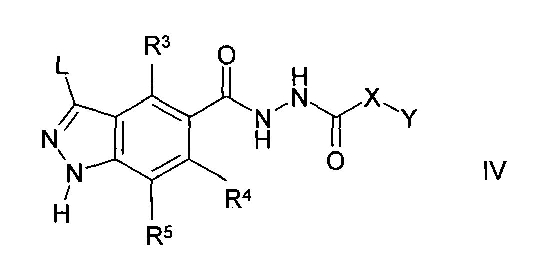
dans laquelle
L, R³, R⁴, R⁵, X et Y revêtent les significations indiquées selon la revendication 1,
est cyclisé en présence de POCl₃,
et/ou une base ou un acide de formule I est converti(e) en l'un de ses sels.

4. Médicaments comprenant au moins un composé selon les revendications 1-2 et/ou des sels, solvats et stéréoisomères pharmaceutiquement utilisables de celui-ci, y compris des mélanges de ceux-ci en toutes proportions, et éventuellement des excipients et/ou des adjuvants.

5. Utilisation de composés selon les revendications 1-2, et de sels, solvats et stéréoisomères pharmaceutiquement utilisables de ceux-ci, y compris des mélanges de ceux-ci en toutes proportions, pour la préparation d'un médicament destiné au traitement et/ou à la prophylaxie de maladies dans lesquelles l'inhibition, la régulation et/ou la modulation de la transduction des signaux de kinases joue un rôle.

6. Utilisation selon la revendication 5, dans laquelle la kinase est la SGK.

7. Utilisation selon la revendication 6 de composés selon les revendications 1-2, et de sels, solvats et stéréoisomères pharmaceutiquement utilisables de ceux-ci, y compris des mélanges de ceux-ci en toutes proportions, pour la préparation d'un médicament destiné au traitement de maladies qui sont influencées par l'inhibition des SGK par les composés selon les revendications 1-2.

8. Utilisation selon la revendication 7 de composés selon les revendications 1-2, et de sels, solvats et stéréoisomères pharmaceutiquement utilisables de ceux-ci, y compris des mélanges de ceux-ci en toutes proportions, pour la préparation d'un médicament destiné au traitement ou à la prévention du diabète, de l'obésité, du syndrome métabolique (dyslipidémie), de l'hypertension systémique et pulmonaire, des maladies cardiovasculaires et des maladies rénales, généralement dans tous les types de fibroses et de processus inflammatoires, du cancer, des cellules tumorales, des métastases tumorales, des coagulopathies, de l'excitabilité neuronale, du glaucome, des cataractes, des infections bactériennes et de la thérapie anti-infectieuse, pour augmenter les capacités d'apprentissage et l'attention, et pour le traitement et la prophylaxie du stress et du vieillissement cellulaires, et pour le traitement de l'acouphène.

9. Utilisation selon la revendication 8, dans laquelle le diabète est le diabète sucré, la néphropathie diabétique, la neuropathie diabétique, l'angiopathie diabétique et la microangiopathie.

10. Utilisation selon la revendication 8, dans laquelle les maladies cardiovasculaires sont les fibroses cardiaques après un infarctus du myocarde, l'hypertrophie cardiaque, l'insuffisance cardiaque et l'artériosclérose.

11. Utilisation selon la revendication 8, dans laquelle les maladies rénales sont la glomérulosclérose, la néphrosclérose, la néphrite, la néphropathie et l'altération de l'excrétion des électrolytes.

12. Utilisation selon la revendication 9, dans laquelle les fibroses et les processus inflammatoires sont la cirrhose hépatique, la fibrose pulmonaire, la pancréatite fibrosante, les rhumatismes et les arthroses, la maladie de Crohn, la bronchite chronique, la fibrose induite par une radiothérapie, la sclérodermie, la mucoviscidose, la cicatrisation et la maladie d'Alzheimer.

13. Médicaments comprenant au moins un composé selon les revendications 1-2 et/ou des sels, solvats et stéréoisomères pharmaceutiquement utilisables de celui-ci, y compris des mélanges de ceux-ci en toutes proportions, et au moins un autre composé actif médicamenteux.

14. Ensemble (kit) constitué de conditionnements séparés
(a) d'une quantité efficace d'un composé selon les revendications 1-2 et/ou de sels, solvats et stéréoisomères pharmaceutiquement utilisables de celui-ci, y compris des mélanges de ceux-ci en toutes proportions,
et
(b) d'une quantité efficace d'un autre composé actif médicamenteux.
